# EUROPÄISCHE PATENTANMELDUNG

(11) **EP 1 854 891 A1**
(43) Veröffentlichungstag der Anmeldung: **14.11.2007**
(21) Anmeldenummer: 07111936.6
(22) Anmeldetag: 25.11.2003
(51) Int. Cl.: C12N 15/85, C12N 9/12, C12N 5/00

(54) **Neue Neomycin-Phosphotransferase-Gene und Verfahren zur Selektion von hochproduzierenden rekombinanten Zellen**

(30) Priorität: 29.11.2002 DE 10256081; 08.07.2003 DE 10330686
(62) Teilanmeldung aus: 03812157.0
(71) Anmelder: Boehringer Ingelheim Pharma GmbH & Co. KG, 55216 Ingelheim am Rhein (DE)
(72) Erfinder: Enenkel, Barbara, 88447 Warthausen (DE); Sautter, Kerstin, 88400 Biberach (DE); Fieder, Jürgen, 89619 Unterstadion (DE); Otto, Ralf, 88422 Oggelshausen (DE); Bergemann, Klaus, 88400 Biberach (DE)
(74) Vertreter: Hammann, Heinz

(57) **Zusammenfassung**

Die Erfindung betrifft neue modifizierte Neomycin-Phosphotransferase-Gene, sowie deren Verwendung in einem Selektionsverfahren für hochproduzierende rekombinante Zellen. Ferner betrifft die vorliegende Erfindung Expressionsvektoren, die ein modifiziertes Neomycin-Phosphotransferase-Gen sowie ein Gen von Interesse in funktioneller Verknüpfung mit einem heterologen Promotor enthalten und ein Verfahren zur Herstellung von heterologen Genprodukten unter Verwendung dieser Expressionsvektoren.

SEQUENZPROTOKOLL

<110> BOEHRINGER INGELHEIM PHARMA GMBH & CO KG

<120> NEUE NEOMYCIN-PHOSPHOTRANSFERASE-GENE UND VERFAHREN
      ZUR SELEKTION VON HOCHPRODUZIERENDEN REKOMBINANTEN ZELLEN

<130> Case 1-1411

<140>
<141>

<160> 55

<170> PatentIn Ver. 3.1

<210> 1
<211> 795
<212> DNA
<213> Escherichia coli

<400> 1
atgattgaac aagatggatt gcacgcaggt tctccggccg cttgggtgga gaggctattc 60
ggctatgact gggcacaaca gacaatcggc tgctctgatg ccgccgtgtt ccggctgtca 120
gcgcaggggc gcccggttct ttttgtcaag accgacctgt ccggtgccct gaatgaactg 180
caagacgagg cagcgcggct atcgtggctg gccacgacgg gcgttccttg cgcagctgtg 240
ctcgacgttg tcactgaagc gggaagggac tggctgctat tgggcgaagt gccggggcag 300
gatctcctgt catctcacct tgctcctgcc gagaaagtat ccatcatggc tgatgcaatg 360
cggcggctgc atacgcttga tccggctacc tgcccattcg accaccaagc gaaacatcgc 420
atcgagcgag cacgtactcg gatggaagcc ggtcttgtcg atcaggatga tctggacgaa 480
gagcatcagg ggctcgcgcc agccgaactg ttcgccaggc tcaaggcgag catgcccgac 540
ggcgaggatc tcgtcgtgac ccatggcgat gcctgcttgc cgaatatcat ggtggaaaat 600
ggccgctttt ctggattcat cgactgtggc cggctgggtg tggcggaccg ctatcaggac 660
atagcgttgg ctacccgtga tattgctgaa gagcttggcg gcgaatgggc tgaccgcttc 720
ctcgtgcttt acggtatcgc cgctcccgat tcgcagcgca tcgccttcta tcgccttctt 780
gacgagttct tctga 795


<210> 2
<211> 264
<212> PRT
<213> Escherichia coli

<400> 2
Met Ile Glu Gln Asp Gly Leu His Ala Gly Ser Pro Ala Ala Trp Val
  1               5                  10                  15

Glu Arg Leu Phe Gly Tyr Asp Trp Ala Gln Gln Thr Ile Gly Cys Ser
             20                  25                  30

Asp Ala Ala Val Phe Arg Leu Ser Ala Gln Gly Arg Pro Val Leu Phe
         35                  40                  45

Val Lys Thr Asp Leu Ser Gly Ala Leu Asn Glu Leu Gln Asp Glu Ala

    50                  55                  60

Ala Arg Leu Ser Trp Leu Ala Thr Thr Gly Val Pro Cys Ala Ala Val
65                  70                  75                  80

Leu Asp Val Val Thr Glu Ala Gly Arg Asp Trp Leu Leu Leu Gly Glu
                85                  90                  95

Val Pro Gly Gln Asp Leu Leu Ser Ser His Leu Ala Pro Ala Glu Lys
            100                 105                 110

Val Ser Ile Met Ala Asp Ala Met Arg Arg Leu His Thr Leu Asp Pro
        115                 120                 125

Ala Thr Cys Pro Phe Asp His Gln Ala Lys His Arg Ile Glu Arg Ala
    130                 135                 140

Arg Thr Arg Met Glu Ala Gly Leu Val Asp Gln Asp Asp Leu Asp Glu
145                 150                 155                 160

Glu His Gln Gly Leu Ala Pro Ala Glu Leu Phe Ala Arg Leu Lys Ala
                165                 170                 175

Ser Met Pro Asp Gly Glu Asp Leu Val Val Thr His Gly Asp Ala Cys
            180                 185                 190

Leu Pro Asn Ile Met Val Glu Asn Gly Arg Phe Ser Gly Phe Ile Asp
        195                 200                 205

Cys Gly Arg Leu Gly Val Ala Asp Arg Tyr Gln Asp Ile Ala Leu Ala
    210                 215                 220

Thr Arg Asp Ile Ala Glu Glu Leu Gly Gly Glu Trp Ala Asp Arg Phe
225                 230                 235                 240

Leu Val Leu Tyr Gly Ile Ala Ala Pro Asp Ser Gln Arg Ile Ala Phe
                245                 250                 255

Tyr Arg Leu Leu Asp Glu Phe Phe
            260

<210> 3
<211> 795
<212> DNA
<213> Künstliche Sequenz

<220>
<223> Beschreibung der künstlichen Sequenz:
Neomycin-Mutante E182G

<400> 3
atgattgaac aagatggatt gcacgcaggt tctccggccg cttgggtgga gaggctattc 60
ggctatgact gggcacaaca gacaatcggc tgctctgatg ccgccgtgtt ccggctgtca 120
gcgcaggggc gcccggttct ttttgtcaag accgacctgt ccggtgccct gaatgaactg 180
caagacgagg cagcgcggct atcgtggctg gccacgacgg gcgttccttg cgcagctgtg 240
ctcgacgttg tcactgaagc gggaagggac tggctgctat tgggcgaagt gccggggcag 300

gatctcctgt catctcacct tgctcctgcc gagaaagtat ccatcatggc tgatgcaatg 360
cggcggctgc atacgcttga tccggctacc tgcccattcg accaccaagc gaaacatcgc 420
atcgagcgag cacgtactcg gatggaagcc ggtcttgtcg atcaggatga tctggacgaa 480
gagcatcagg ggctcgcgcc agccgaactg ttcgccaggc tcaaggcgag catgcccgac 540
ggcggggatc tcgtcgtgac ccatggcgat gcctgcttgc cgaatatcat ggtggaaaat 600
ggccgctttt ctggattcat cgactgtggc cggctgggtg tggcggaccg ctatcaggac 660
atagcgttgg ctacccgtga tattgctgaa gagcttggcg gcgaatgggc tgaccgcttc 720
ctcgtgcttt acggtatcgc cgctcccgat tcgcagcgca tcgccttcta tcgccttctt 780
gacgagttct tctga 795

<210> 4
<211> 264
<212> PRT
<213> Künstliche Sequenz

<220>
<223> Beschreibung der künstlichen Sequenz:
      Neomycin-Mutante E182G

<400> 4
Met Ile Glu Gln Asp Gly Leu His Ala Gly Ser Pro Ala Ala Trp Val
1               5                   10                  15

Glu Arg Leu Phe Gly Tyr Asp Trp Ala Gln Gln Thr Ile Gly Cys Ser
            20                  25                  30

Asp Ala Ala Val Phe Arg Leu Ser Ala Gln Gly Arg Pro Val Leu Phe
        35                  40                  45

Val Lys Thr Asp Leu Ser Gly Ala Leu Asn Glu Leu Gln Asp Glu Ala
    50                  55                  60

Ala Arg Leu Ser Trp Leu Ala Thr Thr Gly Val Pro Cys Ala Ala Val
65                  70                  75                  80

Leu Asp Val Val Thr Glu Ala Gly Arg Asp Trp Leu Leu Leu Gly Glu
                85                  90                  95

Val Pro Gly Gln Asp Leu Leu Ser Ser His Leu Ala Pro Ala Glu Lys
            100                 105                 110

Val Ser Ile Met Ala Asp Ala Met Arg Arg Leu His Thr Leu Asp Pro
        115                 120                 125

Ala Thr Cys Pro Phe Asp His Gln Ala Lys His Arg Ile Glu Arg Ala
    130                 135                 140

Arg Thr Arg Met Glu Ala Gly Leu Val Asp Gln Asp Asp Leu Asp Glu
145                 150                 155                 160

Glu His Gln Gly Leu Ala Pro Ala Glu Leu Phe Ala Arg Leu Lys Ala
                165                 170                 175

Ser Met Pro Asp Gly Gly Asp Leu Val Val Thr His Gly Asp Ala Cys
            180                 185                 190

Leu Pro Asn Ile Met Val Glu Asn Gly Arg Phe Ser Gly Phe Ile Asp
        195                 200                 205

Cys Gly Arg Leu Gly Val Ala Asp Arg Tyr Gln Asp Ile Ala Leu Ala
    210                 215                 220

Thr Arg Asp Ile Ala Glu Glu Leu Gly Gly Glu Trp Ala Asp Arg Phe
225                 230                 235                 240

Leu Val Leu Tyr Gly Ile Ala Ala Pro Asp Ser Gln Arg Ile Ala Phe
                245                 250                 255

Tyr Arg Leu Leu Asp Glu Phe Phe
            260


<210> 5
<211> 795
<212> DNA
<213> Künstliche Sequenz

<220>
<223> Beschreibung der künstlichen Sequenz:
      Neomycin-Mutante W91A

<400> 5
atgattgaac aagatggatt gcacgcaggt tctccggccg cttgggtgga gaggctattc 60
ggctatgact gggcacaaca gacaatcggc tgctctgatg ccgccgtgtt ccggctgtca 120
gcgcaggggc gcccggttct ttttgtcaag accgacctgt ccggtgccct gaatgaactg 180
caagacgagg cagcgcggct atcgtggctg gccacgacgg gcgttccttg cgcagctgtg 240
ctcgacgttg tcactgaagc gggaagggac gcgctgctat tgggcgaagt gccggggcag 300
gatctcctgt catctcacct tgctcctgcc gagaaagtat ccatcatggc tgatgcaatg 360
cggcggctgc atacgcttga tccggctacc tgcccattcg accaccaagc gaaacatcgc 420
atcgagcgag cacgtactcg gatggaagcc ggtcttgtcg atcaggatga tctggacgaa 480
gagcatcagg ggctcgcgcc agccgaactg ttcgccaggc tcaaggcgag catgcccgac 540
ggcgaggatc tcgtcgtgac ccatggcgat gcctgcttgc cgaatatcat ggtggaaaat 600
ggccgctttt ctggattcat cgactgtggc cggctgggtg tggcggaccg ctatcaggac 660
atagcgttgg ctacccgtga tattgctgaa gagcttggcg gcgaatgggc tgaccgcttc 720
ctcgtgcttt acggtatcgc cgctcccgat tcgcagcgca tcgccttcta tcgccttctt 780
gacgagttct tctga                                                  795


<210> 6
<211> 264
<212> PRT
<213> Künstliche Sequenz

<220>
<223> Beschreibung der künstlichen Sequenz:
      Neomycin-Mutante W91A

<400> 6
Met Ile Glu Gln Asp Gly Leu His Ala Gly Ser Pro Ala Ala Trp Val
  1               5                  10                  15

Glu Arg Leu Phe Gly Tyr Asp Trp Ala Gln Gln Thr Ile Gly Cys Ser
             20                  25                  30

Asp Ala Ala Val Phe Arg Leu Ser Ala Gln Gly Arg Pro Val Leu Phe
        35                  40                  45

Val Lys Thr Asp Leu Ser Gly Ala Leu Asn Glu Leu Gln Asp Glu Ala
    50                  55                  60

Ala Arg Leu Ser Trp Leu Ala Thr Thr Gly Val Pro Cys Ala Ala Val
65                  70                  75                  80

Leu Asp Val Val Thr Glu Ala Gly Arg Asp Ala Leu Leu Leu Gly Glu
                85                  90                  95

Val Pro Gly Gln Asp Leu Leu Ser Ser His Leu Ala Pro Ala Glu Lys
            100                 105                 110

Val Ser Ile Met Ala Asp Ala Met Arg Arg Leu His Thr Leu Asp Pro
        115                 120                 125

Ala Thr Cys Pro Phe Asp His Gln Ala Lys His Arg Ile Glu Arg Ala
    130                 135                 140

Arg Thr Arg Met Glu Ala Gly Leu Val Asp Gln Asp Asp Leu Asp Glu
145                 150                 155                 160

Glu His Gln Gly Leu Ala Pro Ala Glu Leu Phe Ala Arg Leu Lys Ala
                165                 170                 175

Ser Met Pro Asp Gly Glu Asp Leu Val Val Thr His Gly Asp Ala Cys
            180                 185                 190

Leu Pro Asn Ile Met Val Glu Asn Gly Arg Phe Ser Gly Phe Ile Asp
        195                 200                 205

Cys Gly Arg Leu Gly Val Ala Asp Arg Tyr Gln Asp Ile Ala Leu Ala
    210                 215                 220

Thr Arg Asp Ile Ala Glu Glu Leu Gly Gly Glu Trp Ala Asp Arg Phe
225                 230                 235                 240

Leu Val Leu Tyr Gly Ile Ala Ala Pro Asp Ser Gln Arg Ile Ala Phe
                245                 250                 255

Tyr Arg Leu Leu Asp Glu Phe Phe
            260

<210> 7
<211> 795
<212> DNA
<213> Künstliche Sequenz

<220>
<223> Beschreibung der künstlichen Sequenz:
      Neomycin-Mutante V198G

<400> 7

atgattgaac aagatggatt gcacgcaggt tctccggccg cttgggtgga gaggctattc 60
ggctatgact gggcacaaca gacaatcggc tgctctgatg ccgccgtgtt ccggctgtca 120
gcgcaggggc gcccggttct ttttgtcaag accgacctgt ccggtgccct gaatgaactg 180
caagacgagg cagcgcggct atcgtggctg gccacgacgg gcgttccttg cgcagctgtg 240
ctcgacgttg tcactgaagc gggaagggac tggctgctat tgggcgaagt gccggggcag 300
gatctcctgt catctcacct tgctcctgcc gagaaagtat ccatcatggc tgatgcaatg 360
cggcggctgc atacgcttga tccggctacc tgcccattcg accaccaagc gaaacatcgc 420
atcgagcgag cacgtactcg gatggaagcc ggtcttgtcg atcaggatga tctggacgaa 480
gagcatcagg ggctcgcgcc agccgaactg ttcgccaggc tcaaggcgag catgcccgac 540
ggcgaggatc tcgtcgtgac ccatggcgat gcctgcttgc cgaatatcat gggggaaaat 600
ggccgctttt ctggattcat cgactgtggc cggctgggtg tggcggaccg ctatcaggac 660
atagcgttgg ctacccgtga tattgctgaa gagcttggcg gcgaatgggc tgaccgcttc 720
ctcgtgcttt acggtatcgc cgctcccgat tcgcagcgca tcgccttcta tcgccttctt 780
gacgagttct tctga 795

<210> 8
<211> 264
<212> PRT
<213> Künstliche Sequenz

<220>
<223> Beschreibung der künstlichen Sequenz:
      Neomycin-Mutante V198G

<400> 8
Met Ile Glu Gln Asp Gly Leu His Ala Gly Ser Pro Ala Ala Trp Val
  1               5                  10                  15

Glu Arg Leu Phe Gly Tyr Asp Trp Ala Gln Gln Thr Ile Gly Cys Ser
             20                  25                  30

Asp Ala Ala Val Phe Arg Leu Ser Ala Gln Gly Arg Pro Val Leu Phe
         35                  40                  45

Val Lys Thr Asp Leu Ser Gly Ala Leu Asn Glu Leu Gln Asp Glu Ala
     50                  55                  60

Ala Arg Leu Ser Trp Leu Ala Thr Thr Gly Val Pro Cys Ala Ala Val
 65                  70                  75                  80

Leu Asp Val Val Thr Glu Ala Gly Arg Asp Trp Leu Leu Leu Gly Glu
                 85                  90                  95

Val Pro Gly Gln Asp Leu Leu Ser Ser His Leu Ala Pro Ala Glu Lys
            100                 105                 110

Val Ser Ile Met Ala Asp Ala Met Arg Arg Leu His Thr Leu Asp Pro
        115                 120                 125

Ala Thr Cys Pro Phe Asp His Gln Ala Lys His Arg Ile Glu Arg Ala
    130                 135                 140

Arg Thr Arg Met Glu Ala Gly Leu Val Asp Gln Asp Asp Leu Asp Glu
145                 150                 155                 160

Glu His Gln Gly Leu Ala Pro Ala Glu Leu Phe Ala Arg Leu Lys Ala

                165                 170                 175

Ser Met Pro Asp Gly Glu Asp Leu Val Val Thr His Gly Asp Ala Cys
            180                 185                 190

Leu Pro Asn Ile Met Gly Glu Asn Gly Arg Phe Ser Gly Phe Ile Asp
        195                 200                 205

Cys Gly Arg Leu Gly Val Ala Asp Arg Tyr Gln Asp Ile Ala Leu Ala
    210                 215                 220

Thr Arg Asp Ile Ala Glu Glu Leu Gly Gly Glu Trp Ala Asp Arg Phe
225                 230                 235                 240

Leu Val Leu Tyr Gly Ile Ala Ala Pro Asp Ser Gln Arg Ile Ala Phe
                245                 250                 255

Tyr Arg Leu Leu Asp Glu Phe Phe
            260

<210> 9
<211> 795
<212> DNA
<213> Künstliche Sequenz

<220>
<223> Beschreibung der künstlichen Sequenz:
Neomycin-Mutante D227A

<400> 9
atgattgaac aagatggatt gcacgcaggt tctccggccg cttgggtgga gaggctattc 60
ggctatgact gggcacaaca gacaatcggc tgctctgatg ccgccgtgtt ccggctgtca 120
gcgcaggggc gcccggttct ttttgtcaag accgacctgt ccggtgccct gaatgaactg 180
caagacgagg cagcgcggct atcgtggctg gccacgacgg gcgttccttg cgcagctgtg 240
ctcgacgttg tcactgaagc gggaagggac tggctgctat tgggcgaagt gccggggcag 300
gatctcctgt catctcacct tgctcctgcc gagaaagtat ccatcatggc tgatgcaatg 360
cggcggctgc atacgcttga tccggctacc tgcccattcg accaccaagc gaaacatcgc 420
atcgagcgag cacgtactcg gatggaagcc ggtcttgtcg atcaggatga tctggacgaa 480
gagcatcagg ggctcgcgcc agccgaactg ttcgccaggc tcaaggcgag catgcccgac 540
ggcgaggatc tcgtcgtgac ccatggcgat gcctgcttgc cgaatatcat ggtggaaaat 600
ggccgctttt ctggattcat cgactgtggc cggctgggtg tggcggaccg ctatcaggac 660
atagcgttgg ctacccgtgc tattgctgaa gagcttggcg gcgaatgggc tgaccgcttc 720
ctcgtgcttt acggtatcgc cgctcccgat tcgcagcgca tcgccttcta tcgccttctt 780
gacgagttct tctga 795

<210> 10
<211> 264
<212> PRT
<213> Künstliche Sequenz

<220>
<223> Beschreibung der künstlichen Sequenz:
Neomycin-Mutante D227A

<400> 10

Met Ile Glu Gln Asp Gly Leu His Ala Gly Ser Pro Ala Ala Trp Val
1               5                   10                  15

Glu Arg Leu Phe Gly Tyr Asp Trp Ala Gln Gln Thr Ile Gly Cys Ser
            20                  25                  30

Asp Ala Ala Val Phe Arg Leu Ser Ala Gln Gly Arg Pro Val Leu Phe
        35                  40                  45

Val Lys Thr Asp Leu Ser Gly Ala Leu Asn Glu Leu Gln Asp Glu Ala
    50                  55                  60

Ala Arg Leu Ser Trp Leu Ala Thr Thr Gly Val Pro Cys Ala Ala Val
65                  70                  75                  80

Leu Asp Val Val Thr Glu Ala Gly Arg Asp Trp Leu Leu Leu Gly Glu
                85                  90                  95

Val Pro Gly Gln Asp Leu Leu Ser Ser His Leu Ala Pro Ala Glu Lys
            100                 105                 110

Val Ser Ile Met Ala Asp Ala Met Arg Arg Leu His Thr Leu Asp Pro
        115                 120                 125

Ala Thr Cys Pro Phe Asp His Gln Ala Lys His Arg Ile Glu Arg Ala
    130                 135                 140

Arg Thr Arg Met Glu Ala Gly Leu Val Asp Gln Asp Asp Leu Asp Glu
145                 150                 155                 160

Glu His Gln Gly Leu Ala Pro Ala Glu Leu Phe Ala Arg Leu Lys Ala
                165                 170                 175

Ser Met Pro Asp Gly Glu Asp Leu Val Val Thr His Gly Asp Ala Cys
            180                 185                 190

Leu Pro Asn Ile Met Val Glu Asn Gly Arg Phe Ser Gly Phe Ile Asp
        195                 200                 205

Cys Gly Arg Leu Gly Val Ala Asp Arg Tyr Gln Asp Ile Ala Leu Ala
    210                 215                 220

Thr Arg Ala Ile Ala Glu Glu Leu Gly Gly Glu Trp Ala Asp Arg Phe
225                 230                 235                 240

Leu Val Leu Tyr Gly Ile Ala Ala Pro Asp Ser Gln Arg Ile Ala Phe
                245                 250                 255

Tyr Arg Leu Leu Asp Glu Phe Phe
            260

<210> 11
<211> 795
<212> DNA
<213> Künstliche Sequenz

<220>
<223> Beschreibung der künstlichen Sequenz:
      Neomycin-Mutante D227V

<400> 11
atgattgaac aagatggatt gcacgcaggt tctccggccg cttgggtgga gaggctattc 60
ggctatgact gggcacaaca gacaatcggc tgctctgatg ccgccgtgtt ccggctgtca 120
gcgcaggggc gcccggttct ttttgtcaag accgacctgt ccggtgccct gaatgaactg 180
caagacgagg cagcgcggct atcgtggctg gccacgacgg gcgttccttg cgcagctgtg 240
ctcgacgttg tcactgaagc gggaagggac tggctgctat tgggcgaagt gccggggcag 300
gatctcctgt catctcacct tgctcctgcc gagaaagtat ccatcatggc tgatgcaatg 360
cggcggctgc atacgcttga tccggctacc tgcccattcg accaccaagc gaaacatcgc 420
atcgagcgag cacgtactcg gatggaagcc ggtcttgtcg atcaggatga tctggacgaa 480
gagcatcagg ggctcgcgcc agccgaactg ttcgccaggc tcaaggcgag catgcccgac 540
ggcgaggatc tcgtcgtgac ccatggcgat gcctgcttgc cgaatatcat ggtggaaaat 600
ggccgctttt ctggattcat cgactgtggc cggctgggtg tggcggaccg ctatcaggac 660
atagcgttgg ctacccgtgt tattgctgaa gagcttggcg gcgaatgggc tgaccgcttc 720
ctcgtgcttt acggtatcgc cgctcccgat tcgcagcgca tcgccttcta tcgccttctt 780
gacgagttct tctga                                                  795


<210> 12
<211> 264
<212> PRT
<213> Künstliche Sequenz

<220>
<223> Beschreibung der künstlichen Sequenz:
      Neomycin-Mutante D227V

<400> 12
Met Ile Glu Gln Asp Gly Leu His Ala Gly Ser Pro Ala Ala Trp Val
  1               5                  10                  15

Glu Arg Leu Phe Gly Tyr Asp Trp Ala Gln Gln Thr Ile Gly Cys Ser
             20                  25                  30

Asp Ala Ala Val Phe Arg Leu Ser Ala Gln Gly Arg Pro Val Leu Phe
         35                  40                  45

Val Lys Thr Asp Leu Ser Gly Ala Leu Asn Glu Leu Gln Asp Glu Ala
     50                  55                  60

Ala Arg Leu Ser Trp Leu Ala Thr Thr Gly Val Pro Cys Ala Ala Val
 65                  70                  75                  80

Leu Asp Val Val Thr Glu Ala Gly Arg Asp Trp Leu Leu Leu Gly Glu
                 85                  90                  95

Val Pro Gly Gln Asp Leu Leu Ser Ser His Leu Ala Pro Ala Glu Lys
            100                 105                 110

Val Ser Ile Met Ala Asp Ala Met Arg Arg Leu His Thr Leu Asp Pro
        115                 120                 125

Ala Thr Cys Pro Phe Asp His Gln Ala Lys His Arg Ile Glu Arg Ala
    130                 135                 140

Arg Thr Arg Met Glu Ala Gly Leu Val Asp Gln Asp Asp Leu Asp Glu
145                 150                 155                 160

Glu His Gln Gly Leu Ala Pro Ala Glu Leu Phe Ala Arg Leu Lys Ala
                165                 170                 175

Ser Met Pro Asp Gly Glu Asp Leu Val Val Thr His Gly Asp Ala Cys
            180                 185                 190

Leu Pro Asn Ile Met Val Glu Asn Gly Arg Phe Ser Gly Phe Ile Asp
        195                 200                 205

Cys Gly Arg Leu Gly Val Ala Asp Arg Tyr Gln Asp Ile Ala Leu Ala
    210                 215                 220

Thr Arg Val Ile Ala Glu Glu Leu Gly Gly Glu Trp Ala Asp Arg Phe
225                 230                 235                 240

Leu Val Leu Tyr Gly Ile Ala Ala Pro Asp Ser Gln Arg Ile Ala Phe
                245                 250                 255

Tyr Arg Leu Leu Asp Glu Phe Phe
            260


<210> 13
<211> 795
<212> DNA
<213> Künstliche Sequenz

<220>
<223> Beschreibung der künstlichen Sequenz:
      Neomycin-Mutante D261G

<400> 13
atgattgaac aagatggatt gcacgcaggt tctccggccg cttgggtgga gaggctattc 60
ggctatgact gggcacaaca gacaatcggc tgctctgatg ccgccgtgtt ccggctgtca 120
gcgcaggggc gcccggttct ttttgtcaag accgacctgt ccggtgccct gaatgaactg 180
caagacgagg cagcgcggct atcgtggctg gccacgacgg gcgttccttg cgcagctgtg 240
ctcgacgttg tcactgaagc gggaagggac tggctgctat tgggcgaagt gccggggcag 300
gatctcctgt catctcacct tgctcctgcc gagaaagtat ccatcatggc tgatgcaatg 360
cggcggctgc atacgcttga tccggctacc tgcccattcg accaccaagc gaaacatcgc 420
atcgagcgag cacgtactcg gatggaagcc ggtcttgtcg atcaggatga tctggacgaa 480
gagcatcagg ggctcgcgcc agccgaactg ttcgccaggc tcaaggcgag catgcccgac 540
ggcgaggatc tcgtcgtgac ccatggcgat gcctgcttgc cgaatatcat ggtggaaaat 600
ggccgctttt ctggattcat cgactgtggc cggctgggtg tggcggaccg ctatcaggac 660
atagcgttgg ctacccgtga tattgctgaa gagcttggcg gcgaatgggc tgaccgcttc 720
ctcgtgcttt acggtatcgc cgctcccgat tcgcagcgca tcgccttcta tcgccttctt 780
ggcgagttct tctga                                                  795


<210> 14
<211> 264
<212> PRT
<213> Künstliche Sequenz

<220>
<223> Beschreibung der künstlichen Sequenz:
      Neomycin-Mutante D261G

<400> 14
Met Ile Glu Gln Asp Gly Leu His Ala Gly Ser Pro Ala Ala Trp Val
  1               5                  10                  15

Glu Arg Leu Phe Gly Tyr Asp Trp Ala Gln Gln Thr Ile Gly Cys Ser
             20                  25                  30

Asp Ala Ala Val Phe Arg Leu Ser Ala Gln Gly Arg Pro Val Leu Phe
         35                  40                  45

Val Lys Thr Asp Leu Ser Gly Ala Leu Asn Glu Leu Gln Asp Glu Ala
     50                  55                  60

Ala Arg Leu Ser Trp Leu Ala Thr Thr Gly Val Pro Cys Ala Ala Val
 65                  70                  75                  80

Leu Asp Val Val Thr Glu Ala Gly Arg Asp Trp Leu Leu Leu Gly Glu
                 85                  90                  95

Val Pro Gly Gln Asp Leu Leu Ser Ser His Leu Ala Pro Ala Glu Lys
            100                 105                 110

Val Ser Ile Met Ala Asp Ala Met Arg Arg Leu His Thr Leu Asp Pro
        115                 120                 125

Ala Thr Cys Pro Phe Asp His Gln Ala Lys His Arg Ile Glu Arg Ala
    130                 135                 140

Arg Thr Arg Met Glu Ala Gly Leu Val Asp Gln Asp Asp Leu Asp Glu
145                 150                 155                 160

Glu His Gln Gly Leu Ala Pro Ala Glu Leu Phe Ala Arg Leu Lys Ala
                165                 170                 175

Ser Met Pro Asp Gly Glu Asp Leu Val Val Thr His Gly Asp Ala Cys
            180                 185                 190

Leu Pro Asn Ile Met Val Glu Asn Gly Arg Phe Ser Gly Phe Ile Asp
        195                 200                 205

Cys Gly Arg Leu Gly Val Ala Asp Arg Tyr Gln Asp Ile Ala Leu Ala
    210                 215                 220

Thr Arg Asp Ile Ala Glu Glu Leu Gly Gly Glu Trp Ala Asp Arg Phe
225                 230                 235                 240

Leu Val Leu Tyr Gly Ile Ala Ala Pro Asp Ser Gln Arg Ile Ala Phe
                245                 250                 255

Tyr Arg Leu Leu Gly Glu Phe Phe
            260

<210> 15
<211> 795
<212> DNA
<213> Künstliche Sequenz

<220>
<223> Beschreibung der künstlichen Sequenz:
      Neomycin-Mutante D261N

<400> 15
atgattgaac aagatggatt gcacgcaggt tctccggccg cttgggtgga gaggctattc 60
ggctatgact gggcacaaca gacaatcggc tgctctgatg ccgccgtgtt ccggctgtca 120
gcgcaggggc gcccggttct ttttgtcaag accgacctgt ccggtgccct gaatgaactg 180
caagacgagg cagcgcggct atcgtggctg gccacgacgg gcgttccttg cgcagctgtg 240
ctcgacgttg tcactgaagc gggaagggac tggctgctat tgggcgaagt gccggggcag 300
gatctcctgt catctcacct tgctcctgcc gagaaagtat ccatcatggc tgatgcaatg 360
cggcggctgc atacgcttga tccggctacc tgcccattcg accaccaagc gaaacatcgc 420
atcgagcgag cacgtactcg gatggaagcc ggtcttgtcg atcaggatga tctggacgaa 480
gagcatcagg ggctcgcgcc agccgaactg ttcgccaggc tcaaggcgag catgcccgac 540
ggcgaggatc tcgtcgtgac ccatggcgat gcctgcttgc cgaatatcat ggtggaaaat 600
ggccgctttt ctggattcat cgactgtggc cggctgggtg tggcggaccg ctatcaggac 660
atagcgttgg ctacccgtga tattgctgaa gagcttggcg gcgaatgggc tgaccgcttc 720
ctcgtgcttt acggtatcgc cgctcccgat tcgcagcgca tcgccttcta tcgccttctt 780
aacgagttct tctga                                                   795


<210> 16
<211> 264
<212> PRT
<213> Künstliche Sequenz

<220>
<223> Beschreibung der künstlichen Sequenz:
      Neomycin-Mutante D261N

<400> 16
Met Ile Glu Gln Asp Gly Leu His Ala Gly Ser Pro Ala Ala Trp Val
  1               5                  10                  15

Glu Arg Leu Phe Gly Tyr Asp Trp Ala Gln Gln Thr Ile Gly Cys Ser
             20                  25                  30

Asp Ala Ala Val Phe Arg Leu Ser Ala Gln Gly Arg Pro Val Leu Phe
         35                  40                  45

Val Lys Thr Asp Leu Ser Gly Ala Leu Asn Glu Leu Gln Asp Glu Ala
     50                  55                  60

Ala Arg Leu Ser Trp Leu Ala Thr Thr Gly Val Pro Cys Ala Ala Val
 65                  70                  75                  80

Leu Asp Val Val Thr Glu Ala Gly Arg Asp Trp Leu Leu Leu Gly Glu
                 85                  90                  95

Val Pro Gly Gln Asp Leu Leu Ser Ser His Leu Ala Pro Ala Glu Lys
            100                 105                 110

Val Ser Ile Met Ala Asp Ala Met Arg Arg Leu His Thr Leu Asp Pro
        115                 120                 125

Ala Thr Cys Pro Phe Asp His Gln Ala Lys His Arg Ile Glu Arg Ala
    130                 135                 140

Arg Thr Arg Met Glu Ala Gly Leu Val Asp Gln Asp Asp Leu Asp Glu
145                 150                 155                 160

Glu His Gln Gly Leu Ala Pro Ala Glu Leu Phe Ala Arg Leu Lys Ala
                165                 170                 175

Ser Met Pro Asp Gly Glu Asp Leu Val Val Thr His Gly Asp Ala Cys
            180                 185                 190

Leu Pro Asn Ile Met Val Glu Asn Gly Arg Phe Ser Gly Phe Ile Asp
        195                 200                 205

Cys Gly Arg Leu Gly Val Ala Asp Arg Tyr Gln Asp Ile Ala Leu Ala
    210                 215                 220

Thr Arg Asp Ile Ala Glu Glu Leu Gly Gly Glu Trp Ala Asp Arg Phe
225                 230                 235                 240

Leu Val Leu Tyr Gly Ile Ala Ala Pro Asp Ser Gln Arg Ile Ala Phe
                245                 250                 255

Tyr Arg Leu Leu Asn Glu Phe Phe
            260

<210> 17
<211> 795
<212> DNA
<213> Künstliche Sequenz

<220>
<223> Beschreibung der künstlichen Sequenz:
      Neomycin-Mutante F240I

<400> 17
atgattgaac aagatggatt gcacgcaggt tctccggccg cttgggtgga gaggctattc 60
ggctatgact gggcacaaca gacaatcggc tgctctgatg ccgccgtgtt ccggctgtca 120
gcgcaggggc gcccggttct ttttgtcaag accgacctgt ccggtgccct gaatgaactg 180
caagacgagg cagcgcggct atcgtggctg gccacgacgg gcgttccttg cgcagctgtg 240
ctcgacgttg tcactgaagc gggaagggac tggctgctat tgggcgaagt gccggggcag 300
gatctcctgt catctcacct tgctcctgcc gagaaagtat ccatcatggc tgatgcaatg 360
cggcggctgc atacgcttga tccggctacc tgcccattcg accaccaagc gaaacatcgc 420
atcgagcgag cacgtactcg gatggaagcc ggtcttgtcg atcaggatga tctggacgaa 480
gagcatcagg ggctcgcgcc agccgaactg ttcgccaggc tcaaggcgag catgcccgac 540
ggcgaggatc tcgtcgtgac ccatggcgat gcctgcttgc cgaatatcat ggtggaaaat 600
ggccgctttt ctggattcat cgactgtggc cggctgggtg tggcggaccg ctatcaggac 660
atagcgttgg ctacccgtga tattgctgaa gagcttggcg gcgaatgggc tgaccgcatc 720
ctcgtgcttt acggtatcgc cgctcccgat tcgcagcgca tcgccttcta tcgccttctt 780
gacgagttct tctga                                                  795

<210> 18
<211> 264
<212> PRT
<213> Künstliche Sequenz

<220>
<223> Beschreibung der künstlichen Sequenz:
      Neomycin-Mutante F240I

<400> 18
Met Ile Glu Gln Asp Gly Leu His Ala Gly Ser Pro Ala Ala Trp Val
  1               5                  10                  15

Glu Arg Leu Phe Gly Tyr Asp Trp Ala Gln Gln Thr Ile Gly Cys Ser
             20                  25                  30

Asp Ala Ala Val Phe Arg Leu Ser Ala Gln Gly Arg Pro Val Leu Phe
         35                  40                  45

Val Lys Thr Asp Leu Ser Gly Ala Leu Asn Glu Leu Gln Asp Glu Ala
     50                  55                  60

Ala Arg Leu Ser Trp Leu Ala Thr Thr Gly Val Pro Cys Ala Ala Val
 65                  70                  75                  80

Leu Asp Val Val Thr Glu Ala Gly Arg Asp Trp Leu Leu Leu Gly Glu
                 85                  90                  95

Val Pro Gly Gln Asp Leu Leu Ser Ser His Leu Ala Pro Ala Glu Lys
            100                 105                 110

Val Ser Ile Met Ala Asp Ala Met Arg Arg Leu His Thr Leu Asp Pro
        115                 120                 125

Ala Thr Cys Pro Phe Asp His Gln Ala Lys His Arg Ile Glu Arg Ala
    130                 135                 140

Arg Thr Arg Met Glu Ala Gly Leu Val Asp Gln Asp Asp Leu Asp Glu
145                 150                 155                 160

Glu His Gln Gly Leu Ala Pro Ala Glu Leu Phe Ala Arg Leu Lys Ala
                165                 170                 175

Ser Met Pro Asp Gly Glu Asp Leu Val Val Thr His Gly Asp Ala Cys
            180                 185                 190

Leu Pro Asn Ile Met Val Glu Asn Gly Arg Phe Ser Gly Phe Ile Asp
        195                 200                 205

Cys Gly Arg Leu Gly Val Ala Asp Arg Tyr Gln Asp Ile Ala Leu Ala
    210                 215                 220

Thr Arg Asp Ile Ala Glu Glu Leu Gly Gly Glu Trp Ala Asp Arg Ile
225                 230                 235                 240

Leu Val Leu Tyr Gly Ile Ala Ala Pro Asp Ser Gln Arg Ile Ala Phe
                245                 250                 255

Tyr Arg Leu Leu Asp Glu Phe Phe
            260

<210> 19
<211> 795
<212> DNA
<213> Künstliche Sequenz

<220>
<223> Beschreibung der künstlichen Sequenz:
      Neomycin-Mutante E182D

<400> 19
atgattgaac aagatggatt gcacgcaggt tctccggccg cttgggtgga gaggctattc 60
ggctatgact gggcacaaca gacaatcggc tgctctgatg ccgccgtgtt ccggctgtca 120
gcgcaggggc gcccggttct ttttgtcaag accgacctgt ccggtgccct gaatgaactg 180
caagacgagg cagcgcggct atcgtggctg gccacgacgg gcgttccttg cgcagctgtg 240
ctcgacgttg tcactgaagc gggaagggac tggctgctat tgggcgaagt gccggggcag 300
gatctcctgt catctcacct tgctcctgcc gagaaagtat ccatcatggc tgatgcaatg 360
cggcggctgc atacgcttga tccggctacc tgcccattcg accaccaagc gaaacatcgc 420
atcgagcgag cacgtactcg gatggaagcc ggtcttgtcg atcaggatga tctggacgaa 480
gagcatcagg ggctcgcgcc agccgaactg ttcgccaggc tcaaggcgag catgcccgac 540
ggcgatgatc tcgtcgtgac ccatggcgat gcctgcttgc cgaatatcat ggtggaaaat 600
ggccgctttt ctggattcat cgactgtggc cggctgggtg tggcggaccg ctatcaggac 660
atagcgttgg ctacccgtga tattgctgaa gagcttggcg gcgaatgggc tgaccgcttc 720
ctcgtgcttt acggtatcgc cgctcccgat tcgcagcgca tcgccttcta tcgccttctt 780
gacgagttct tctga                                                  795

<210> 20
<211> 264
<212> PRT
<213> Künstliche Sequenz

<220>
<223> Beschreibung der künstlichen Sequenz:
      Neomycin-Mutante E182D

<400> 20
Met Ile Glu Gln Asp Gly Leu His Ala Gly Ser Pro Ala Ala Trp Val
  1               5                  10                  15

Glu Arg Leu Phe Gly Tyr Asp Trp Ala Gln Gln Thr Ile Gly Cys Ser
             20                  25                  30

Asp Ala Ala Val Phe Arg Leu Ser Ala Gln Gly Arg Pro Val Leu Phe
         35                  40                  45

Val Lys Thr Asp Leu Ser Gly Ala Leu Asn Glu Leu Gln Asp Glu Ala
     50                  55                  60

Ala Arg Leu Ser Trp Leu Ala Thr Thr Gly Val Pro Cys Ala Ala Val
 65                  70                  75                  80

Leu Asp Val Val Thr Glu Ala Gly Arg Asp Trp Leu Leu Leu Gly Glu

85                  90                  95

Val Pro Gly Gln Asp Leu Leu Ser Ser His Leu Ala Pro Ala Glu Lys
            100                 105                 110

Val Ser Ile Met Ala Asp Ala Met Arg Arg Leu His Thr Leu Asp Pro
        115                 120                 125

Ala Thr Cys Pro Phe Asp His Gln Ala Lys His Arg Ile Glu Arg Ala
    130                 135                 140

Arg Thr Arg Met Glu Ala Gly Leu Val Asp Gln Asp Asp Leu Asp Glu
145                 150                 155                 160

Glu His Gln Gly Leu Ala Pro Ala Glu Leu Phe Ala Arg Leu Lys Ala
                165                 170                 175

Ser Met Pro Asp Gly Asp Asp Leu Val Val Thr His Gly Asp Ala Cys
            180                 185                 190

Leu Pro Asn Ile Met Val Glu Asn Gly Arg Phe Ser Gly Phe Ile Asp
        195                 200                 205

Cys Gly Arg Leu Gly Val Ala Asp Arg Tyr Gln Asp Ile Ala Leu Ala
    210                 215                 220

Thr Arg Asp Ile Ala Glu Glu Leu Gly Gly Glu Trp Ala Asp Arg Phe
225                 230                 235                 240

Leu Val Leu Tyr Gly Ile Ala Ala Pro Asp Ser Gln Arg Ile Ala Phe
                245                 250                 255

Tyr Arg Leu Leu Asp Glu Phe Phe
            260

<210> 21
<211> 795
<212> DNA
<213> Künstliche Sequenz

<220>
<223> Beschreibung der künstlichen Sequenz:
      Neomycin-Mutante D227G

<400> 21
atgattgaac aagatggatt gcacgcaggt tctccggccg cttgggtgga gaggctattc 60
ggctatgact gggcacaaca gacaatcggc tgctctgatg ccgccgtgtt ccggctgtca 120
gcgcaggggc gcccggttct ttttgtcaag accgacctgt ccggtgccct gaatgaactg 180
caagacgagg cagcgcggct atcgtggctg gccacgacgg gcgttccttg cgcagctgtg 240
ctcgacgttg tcactgaagc gggaagggac tggctgctat tgggcgaagt gccggggcag 300
gatctcctgt catctcacct tgctcctgcc gagaaagtat ccatcatggc tgatgcaatg 360
cggcggctgc atacgcttga tccggctacc tgcccattcg accaccaagc gaaacatcgc 420
atcgagcgag cacgtactcg gatggaagcc ggtcttgtcg atcaggatga tctggacgaa 480
gagcatcagg ggctcgcgcc agccgaactg ttcgccaggc tcaaggcgag catgcccgac 540
ggcgaggatc tcgtcgtgac ccatggcgat gcctgcttgc cgaatatcat ggtggaaaat 600
ggccgctttt ctggattcat cgactgtggc cggctgggtg tggcggaccg ctatcaggac 660

atagcgttgg ctacccgtgg tattgctgaa gagcttggcg gcgaatgggc tgaccgcttc 720
ctcgtgcttt acggtatcgc cgctcccgat tcgcagcgca tcgccttcta tcgccttctt 780
gacgagttct tctga 795

<210> 22
<211> 264
<212> PRT
<213> Künstliche Sequenz

<220>
<223> Beschreibung der künstlichen Sequenz:
      Neomycin-Mutante D227G

<400> 22
Met Ile Glu Gln Asp Gly Leu His Ala Gly Ser Pro Ala Ala Trp Val
  1               5                  10                  15

Glu Arg Leu Phe Gly Tyr Asp Trp Ala Gln Gln Thr Ile Gly Cys Ser
             20                  25                  30

Asp Ala Ala Val Phe Arg Leu Ser Ala Gln Gly Arg Pro Val Leu Phe
         35                  40                  45

Val Lys Thr Asp Leu Ser Gly Ala Leu Asn Glu Leu Gln Asp Glu Ala
     50                  55                  60

Ala Arg Leu Ser Trp Leu Ala Thr Thr Gly Val Pro Cys Ala Ala Val
 65                  70                  75                  80

Leu Asp Val Val Thr Glu Ala Gly Arg Asp Trp Leu Leu Leu Gly Glu
                 85                  90                  95

Val Pro Gly Gln Asp Leu Leu Ser Ser His Leu Ala Pro Ala Glu Lys
            100                 105                 110

Val Ser Ile Met Ala Asp Ala Met Arg Arg Leu His Thr Leu Asp Pro
        115                 120                 125

Ala Thr Cys Pro Phe Asp His Gln Ala Lys His Arg Ile Glu Arg Ala
    130                 135                 140

Arg Thr Arg Met Glu Ala Gly Leu Val Asp Gln Asp Asp Leu Asp Glu
145                 150                 155                 160

Glu His Gln Gly Leu Ala Pro Ala Glu Leu Phe Ala Arg Leu Lys Ala
                165                 170                 175

Ser Met Pro Asp Gly Glu Asp Leu Val Val Thr His Gly Asp Ala Cys
            180                 185                 190

Leu Pro Asn Ile Met Val Glu Asn Gly Arg Phe Ser Gly Phe Ile Asp
        195                 200                 205

Cys Gly Arg Leu Gly Val Ala Asp Arg Tyr Gln Asp Ile Ala Leu Ala
    210                 215                 220

Thr Arg Gly Ile Ala Glu Glu Leu Gly Gly Glu Trp Ala Asp Arg Phe
225                 230                 235                 240

Leu Val Leu Tyr Gly Ile Ala Ala Pro Asp Ser Gln Arg Ile Ala Phe
                245                 250                 255

Tyr Arg Leu Leu Asp Glu Phe Phe
            260

<210> 23
<211> 795
<212> DNA
<213> Künstliche Sequenz

<220>
<223> Beschreibung der künstlichen Sequenz:
      Neomycin-Mutante D190G

<400> 23
atgattgaac aagatggatt gcacgcaggt tctccggccg cttgggtgga gaggctattc 60
ggctatgact gggcacaaca gacaatcggc tgctctgatg ccgccgtgtt ccggctgtca 120
gcgcaggggc gcccggttct ttttgtcaag accgacctgt ccggtgccct gaatgaactg 180
caagacgagg cagcgcggct atcgtggctg gccacgacgg gcgttccttg cgcagctgtg 240
ctcgacgttg tcactgaagc gggaagggac tggctgctat tgggcgaagt gccggggcag 300
gatctcctgt catctcacct tgctcctgcc gagaaagtat ccatcatggc tgatgcaatg 360
cggcggctgc atacgcttga tccggctacc tgcccattcg accaccaagc gaaacatcgc 420
atcgagcgag cacgtactcg gatggaagcc ggtcttgtcg atcaggatga tctggacgaa 480
gagcatcagg ggctcgcgcc agccgaactg ttcgccaggc tcaaggcgag catgcccgac 540
ggcgaggatc tcgtcgtgac ccatggcggt gcctgcttgc cgaatatcat ggtggaaaat 600
ggccgctttt ctggattcat cgactgtggc cggctgggtg tggcggaccg ctatcaggac 660
atagcgttgg ctacccgtga tattgctgaa gagcttggcg gcgaatgggc tgaccgcttc 720
ctcgtgcttt acggtatcgc cgctcccgat tcgcagcgca tcgccttcta tcgccttctt 780
gacgagttct tctga                                                  795

<210> 24
<211> 264
<212> PRT
<213> Künstliche Sequenz

<220>
<223> Beschreibung der künstlichen Sequenz:
      Neomycin-Mutante D190G

<400> 24
Met Ile Glu Gln Asp Gly Leu His Ala Gly Ser Pro Ala Ala Trp Val
  1               5                  10                  15

Glu Arg Leu Phe Gly Tyr Asp Trp Ala Gln Gln Thr Ile Gly Cys Ser
             20                  25                  30

Asp Ala Ala Val Phe Arg Leu Ser Ala Gln Gly Arg Pro Val Leu Phe
         35                  40                  45

Val Lys Thr Asp Leu Ser Gly Ala Leu Asn Glu Leu Gln Asp Glu Ala
     50                  55                  60

Ala Arg Leu Ser Trp Leu Ala Thr Thr Gly Val Pro Cys Ala Ala Val
65                  70                  75                  80

Leu Asp Val Val Thr Glu Ala Gly Arg Asp Trp Leu Leu Leu Gly Glu
                85                  90                  95

Val Pro Gly Gln Asp Leu Leu Ser Ser His Leu Ala Pro Ala Glu Lys
            100                 105                 110

Val Ser Ile Met Ala Asp Ala Met Arg Arg Leu His Thr Leu Asp Pro
        115                 120                 125

Ala Thr Cys Pro Phe Asp His Gln Ala Lys His Arg Ile Glu Arg Ala
    130                 135                 140

Arg Thr Arg Met Glu Ala Gly Leu Val Asp Gln Asp Asp Leu Asp Glu
145                 150                 155                 160

Glu His Gln Gly Leu Ala Pro Ala Glu Leu Phe Ala Arg Leu Lys Ala
                165                 170                 175

Ser Met Pro Asp Gly Glu Asp Leu Val Val Thr His Gly Gly Ala Cys
            180                 185                 190

Leu Pro Asn Ile Met Val Glu Asn Gly Arg Phe Ser Gly Phe Ile Asp
        195                 200                 205

Cys Gly Arg Leu Gly Val Ala Asp Arg Tyr Gln Asp Ile Ala Leu Ala
    210                 215                 220

Thr Arg Asp Ile Ala Glu Glu Leu Gly Gly Glu Trp Ala Asp Arg Phe
225                 230                 235                 240

Leu Val Leu Tyr Gly Ile Ala Ala Pro Asp Ser Gln Arg Ile Ala Phe
                245                 250                 255

Tyr Arg Leu Leu Asp Glu Phe Phe
            260

<210> 25
<211> 795
<212> DNA
<213> Künstliche Sequenz

<220>
<223> Beschreibung der künstlichen Sequenz:
      Neomycin-Mutante D208G

<400> 25
atgattgaac aagatggatt gcacgcaggt tctccggccg cttgggtgga gaggctattc 60
ggctatgact gggcacaaca gacaatcggc tgctctgatg ccgccgtgtt ccggctgtca 120
gcgcaggggc gcccggttct ttttgtcaag accgacctgt ccggtgccct gaatgaactg 180
caagacgagg cagcgcggct atcgtggctg gccacgacgg gcgttccttg cgcagctgtg 240
ctcgacgttg tcactgaagc gggaagggac tggctgctat tgggcgaagt gccggggcag 300
gatctcctgt catctcacct tgctcctgcc gagaaagtat ccatcatggc tgatgcaatg 360

cggcggctgc atacgcttga tccggctacc tgcccattcg accaccaagc gaaacatcgc 420
atcgagcgag cacgtactcg gatggaagcc ggtcttgtcg atcaggatga tctggacgaa 480
gagcatcagg ggctcgcgcc agccgaactg ttcgccaggc tcaaggcgag catgcccgac 540
ggcgaggatc tcgtcgtgac ccatggcgat gcctgcttgc cgaatatcat ggtggaaaat 600
ggccgctttt ctggattcat cggctgtggc cggctgggtg tggcggaccg ctatcaggac 660
atagcgttgg ctacccgtga tattgctgaa gagcttggcg gcgaatgggc tgaccgcttc 720
ctcgtgcttt acggtatcgc cgctcccgat tcgcagcgca tcgccttcta tcgccttctt 780
gacgagttct tctga 795

<210> 26
<211> 264
<212> PRT
<213> Künstliche Sequenz

<220>
<223> Beschreibung der künstlichen Sequenz:
      Neomycin-Mutante D208G

<400> 26
Met Ile Glu Gln Asp Gly Leu His Ala Gly Ser Pro Ala Ala Trp Val
  1               5                  10                  15

Glu Arg Leu Phe Gly Tyr Asp Trp Ala Gln Gln Thr Ile Gly Cys Ser
             20                  25                  30

Asp Ala Ala Val Phe Arg Leu Ser Ala Gln Gly Arg Pro Val Leu Phe
         35                  40                  45

Val Lys Thr Asp Leu Ser Gly Ala Leu Asn Glu Leu Gln Asp Glu Ala
     50                  55                  60

Ala Arg Leu Ser Trp Leu Ala Thr Thr Gly Val Pro Cys Ala Ala Val
 65                  70                  75                  80

Leu Asp Val Val Thr Glu Ala Gly Arg Asp Trp Leu Leu Leu Gly Glu
                 85                  90                  95

Val Pro Gly Gln Asp Leu Leu Ser Ser His Leu Ala Pro Ala Glu Lys
            100                 105                 110

Val Ser Ile Met Ala Asp Ala Met Arg Arg Leu His Thr Leu Asp Pro
        115                 120                 125

Ala Thr Cys Pro Phe Asp His Gln Ala Lys His Arg Ile Glu Arg Ala
    130                 135                 140

Arg Thr Arg Met Glu Ala Gly Leu Val Asp Gln Asp Asp Leu Asp Glu
145                 150                 155                 160

Glu His Gln Gly Leu Ala Pro Ala Glu Leu Phe Ala Arg Leu Lys Ala
                165                 170                 175

Ser Met Pro Asp Gly Glu Asp Leu Val Val Thr His Gly Asp Ala Cys
            180                 185                 190

Leu Pro Asn Ile Met Val Glu Asn Gly Arg Phe Ser Gly Phe Ile Gly

195                 200                 205

Cys Gly Arg Leu Gly Val Ala Asp Arg Tyr Gln Asp Ile Ala Leu Ala
    210                 215                 220

Thr Arg Asp Ile Ala Glu Glu Leu Gly Gly Glu Trp Ala Asp Arg Phe
225                 230                 235                 240

Leu Val Leu Tyr Gly Ile Ala Ala Pro Asp Ser Gln Arg Ile Ala Phe
                245                 250                 255

Tyr Arg Leu Leu Asp Glu Phe Phe
            260


<210> 27
<211> 21
<212> DNA
<213> Künstliche Sequenz

<220>
<223> Beschreibung der künstlichen Sequenz:
      Oligonukleotid Neofor5

<400> 27
ttccagaagt agtgaggagg c                                                21


<210> 28
<211> 19
<212> DNA
<213> Künstliche Sequenz

<220>
<223> Beschreibung der künstlichen Sequenz:
      Oligonukleotid Neorev5

<400> 28
atggcaggtt gggcgtcgc                                                   19


<210> 29
<211> 21
<212> DNA
<213> Künstliche Sequenz

<220>
<223> Beschreibung der künstlichen Sequenz:
      Oligonukleotid Neofor2

<400> 29
gaactgttcg ccaggctcaa g                                                21


<210> 30
<211> 22
<212> DNA

<213> Künstliche Sequenz

<220>
<223> Beschreibung der künstlichen Sequenz:
      Oligonukleotid IC49

<400> 30
cggcaaaatc ccttataaat ca                                                22


<210> 31
<211> 20
<212> DNA
<213> Künstliche Sequenz

<220>
<223> Beschreibung der künstlichen Sequenz:
      Oligonukleotid E182Gfor

<400> 31
gacggcgggg atctcgtcgt                                                   20


<210> 32
<211> 20
<212> DNA
<213> Künstliche Sequenz

<220>
<223> Beschreibung der künstlichen Sequenz:
      Oligonukleotid E182Grev

<400> 32
acgacgagat ccccgccgtc                                                   20


<210> 33
<211> 23
<212> DNA
<213> Künstliche Sequenz

<220>
<223> Beschreibung der künstlichen Sequenz:
      Oligonukleotid W91Afor

<400> 33
gggaagggac gcgctgctat tgg                                               23


<210> 34
<211> 23
<212> DNA
<213> Künstliche Sequenz

<220>
<223> Beschreibung der künstlichen Sequenz:
      Oligonukleotid W91Arev

<400> 34
ccaatagcag cgcgtccctt ccc                                              23


<210> 35
<211> 24
<212> DNA
<213> Künstliche Sequenz

<220>
<223> Beschreibung der künstlichen Sequenz:
      Oligonukleotid V198Gfor

<400> 35
ccgaatatca tgggggaaaa tggc                                             24


<210> 36
<211> 24
<212> DNA
<213> Künstliche Sequenz

<220>
<223> Beschreibung der künstlichen Sequenz:
      Oligonukleotid V198Grev

<400> 36
gccattttcc cccatgatat tcgg                                             24


<210> 37
<211> 21
<212> DNA
<213> Künstliche Sequenz

<220>
<223> Beschreibung der künstlichen Sequenz:
      Oligonukleotid D227Afor

<400> 37
ctacccgtgc tattgctgaa g                                                21


<210> 38
<211> 21
<212> DNA
<213> Künstliche Sequenz

<220>
<223> Beschreibung der künstlichen Sequenz:
      Oligonukleotid D227Arev

<400> 38
cttcagcaat agcacgggta g                                                21

<210> 39
<211> 21
<212> DNA
<213> Künstliche Sequenz

<220>
<223> Beschreibung der künstlichen Sequenz:
      Oligonukleotid D227Vfor

<400> 39
ctacccgtgt tattgctgaa g                                                21


<210> 40
<211> 21
<212> DNA
<213> Künstliche Sequenz

<220>
<223> Beschreibung der künstlichen Sequenz:
      Oligonukleotid D227Vrev

<400> 40
cttcagcaat aacacgggta g                                                21


<210> 41
<211> 24
<212> DNA
<213> Künstliche Sequenz

<220>
<223> Beschreibung der künstlichen Sequenz:
      Oligonukleotid D261Gfor

<400> 41
gccttcttgg cgagttcttc tgag                                             24


<210> 42
<211> 24
<212> DNA
<213> Künstliche Sequenz

<220>
<223> Beschreibung der künstlichen Sequenz:
      Oligonukleotid D261Grev

<400> 42
ctcagaagaa ctcgccaaga aggc                                             24


<210> 43
<211> 24
<212> DNA
<213> Künstliche Sequenz

<220>
<223> Beschreibung der künstlichen Sequenz:
      Oligonukleotid D261Nfor

<400> 43
gccttcttaa cgagttcttc tgag                                              24


<210> 44
<211> 24
<212> DNA
<213> Künstliche Sequenz

<220>
<223> Beschreibung der künstlichen Sequenz:
      Oligonukleotid D261Nrev

<400> 44
ctcagaagaa ctcgttaaga aggc                                              24


<210> 45
<211> 22
<212> DNA
<213> Künstliche Sequenz

<220>
<223> Beschreibung der künstlichen Sequenz:
      Oligonukleotid F240Ifor

<400> 45
ggctgaccgc atcctcgtgc tt                                                22


<210> 46
<211> 22
<212> DNA
<213> Künstliche Sequenz

<220>
<223> Beschreibung der künstlichen Sequenz:
      Oligonukleotid F240Irev

<400> 46
aagcacgagg atgcggtcag cc                                                22


<210> 47
<211> 20
<212> DNA
<213> Künstliche Sequenz

<220>
<223> Beschreibung der künstlichen Sequenz:
      Oligonucleotid E182Dfor

<400> 47

gacggcgatg atctcgtcgt                                                    20


<210> 48
<211> 20
<212> DNA
<213> Künstliche Sequenz

<220>
<223> Beschreibung der künstlichen Sequenz:
      Oligonucleotid E182Drev

<400> 48
acgacgagat catcgccgtc                                                    20


<210> 49
<211> 19
<212> DNA
<213> Künstliche Sequenz

<220>
<223> Beschreibung der künstlichen Sequenz:
      Oligonucleotid D190Gfor

<400> 49
catggcggtg cctgcttgc                                                     19


<210> 50
<211> 19
<212> DNA
<213> Künstliche Sequenz

<220>
<223> Beschreibung der künstlichen Sequenz:
      Oligonucleotid D190Grev

<400> 50
gcaagcaggc accgccatg                                                     19


<210> 51
<211> 19
<212> DNA
<213> Künstliche Sequenz

<220>
<223> Beschreibung der künstlichen Sequenz:
      Oligonucleotid D208Gfor

<400> 51
gattcatcgg ctgtggccg                                                     19


<210> 52
<211> 19

<212> DNA
<213> Künstliche Sequenz

<220>
<223> Beschreibung der künstlichen Sequenz:
      Oligonucleotid D208Grev

<400> 52
cggccacagc cgatgaatc                                                19


<210> 53
<211> 21
<212> DNA
<213> Künstliche Sequenz

<220>
<223> Beschreibung der künstlichen Sequenz:
      Oligonucleotid D227Gfor

<400> 53
ctacccgtgg tattgctgaa g                                             21


<210> 54
<211> 21
<212> DNA
<213> Künstliche Sequenz

<220>
<223> Beschreibung der künstlichen Sequenz:
      Oligonucleotid D227Grev

<400> 54
cttcagcaat accacgggta g                                             21


<210> 55
<211> 2406
<212> DNA
<213> Cricetulus griseus

<300>
<310> PCT/EP/96/04631
<311> 1996-10-24
<312> 1997-05-01

<400> 55
gatctccagg acagccatgg ctattacaca gagaaaccct gtctggaaaa acaaaaaatt 60
agtgtccatg tgtaaatgtg tggagtatgc ttgtcatgcc acatacagag gtagagggca 120
gtttatggga gtcagttcct attcttcctt tatgggggac ctggggactg aactcaggtc 180
atcaggcttg gcagaaagtg cattagctca cggagcctta tcattggcga aagctctctc 240
aagtagaaaa tcaatgtgtt tgctcatagt gcaatcatta tgtttcgaga ggggaagggt 300
acaatcgttg gggcatgtgt ggtcacatct gaatagcagt agctccctag gagaattcca 360
agttctttgg tggtgtatca atgcccttaa aggggtcaac aacttttttt ccctctgaca 420
aaactatctt cttatgtcct tgtccctcat atttgaagta ttttattctt tgcagtgttg 480
aatatcaatt ctagcacctc agacatgtta ggtaagtacc ctacaactca ggttaactaa 540

tttaatttaa ctaatttaac cccaacactt tttctttgtt tatccacatt tgtggagtgt 600
gtgtgtgtgt gtgtgtgtgt gtgtgtgtgt gtgtgtgtgt gtgtgtgtgt gtgtgtgtgc 660
gcgcgcgcgc gcgctcggat cattctacct tttgtttaaa aaatgttagt ccaggggtgg 720
ggtgcactgt gaaagtctga gggtaacttg ctggggtcag ttctttccac tataggacag 780
aactccaggt gtcaactctt tactgacaga accatccaaa tagccctatc taattttagt 840
ttttatttta tttatttttt gtttttcgag acagggtttc tctgtggctt tggaggctgt 900
cctggaacta gctcttgtag accaggctgg tctcgaactc agagatccac ctgcctctgc 960
ctcctgagtg ctgggattaa aggcatgcgc caccaacgct tggctctacc taattttaaa 1020
agagattgtg tgtcacaagg gtgtcatgtc gccctgcaac cacccccccc ccaaaaaaaa 1080
aaaaaaaaaa acttcactga agctgaagca cgatgatttg gttactctgg ctggccaatg 1140
agctctaggg agtctcctgt caaacagaat ctcaacaggc gcagcagtct tttttaaagt 1200
ggggttacaa cacaggtttt tgcatatcag gcattttatc taagctattt cccagccaaa 1260
aatgtgtatt ttggaggcag cagagctaat agattaaaat gagggaagag cccacacagg 1320
ttattaggaa gataagcatc ttctttatat aaaacaaaac caaaccaaac tggaggaggt 1380
ctacctttag ggatggaaga aaagacattt agagggtgca atagaaaggg cactgagttt 1440
gtgaggtgga ggactgggag agggcgcaac cgctttaact gtcctgtttt gcctattttt 1500
tggggacagc acatgttcct atttttccca ggatgggcaa tctccacgtc caaacttgcg 1560
gtcgaggact acagtcattt tgcaggtttc cttactgtat ggcttttaaa acgtgcaaag 1620
gtgaccatta accgtttcac gctgggaggg cacgtgcggc tcagatgctt cctctgactg 1680
agggccagga gggggctaca cggaagaggc cacacccgca cttgggaaga ctcgatttgg 1740
gcttcagctg gctgagacgc cccagcaggc tcctcggcta caccttcagc cccgaatgcc 1800
ttccggccca taacccttcc cttctaggca tttccggcga ggacccaccc tcgcgccaaa 1860
cattcggccc catcccccgg tcctcacctg aatctctaac tctgactcca gagtttagag 1920
actataacca gatagcccgg atgtgtggaa ctgcatcttg ggacgagtag ttttagcaaa 1980
aagaaagcga cgaaaaacta caattcccag acagacttgt gttacctctc ttctcatgct 2040
aaacaagccc cctttaaagg aaagcccctc ttagtcgcat cgactgtgta agaaaggcgt 2100
ttgaaacatt ttaatgttgg gcacaccgtt tcgaggaccg aaatgagaaa gagcataggg 2160
aaacggagcg cccgagctag tctggcactg cgttagacag ccgcggtcgt tgcagcgggc 2220
aggcacttgc gtggacgcct aaggggcggg tctttcggcc gggaagcccc gttggtccgc 2280
gcggctcttc ctttccgatc cgccatccgt ggtgagtgtg tgctgcgggc tgccgctccg 2340
gcttggggct tcccgcgtcg ctctcaccct ggtcggcggc tctaatccgt ctcttttcga 2400
atgtag 2406

SEQUENZPROTOKOLL

<110> BOEHRINGER INGELHEIM PHARMA GMBH & CO KG

<120> NEUE NEOMYCIN-PHOSPHOTRANSFERASE-GENE UND VERFAHREN
      ZUR SELEKTION VON HOCHPRODUZIERENDEN REKOMBINANTEN ZELLEN

<130> Case 1-1411

<140>
<141>

<160> 55

<170> PatentIn Ver. 3.1

<210> 1
<211> 795
<212> DNA
<213> Escherichia coli

<400> 1
atgattgaac aagatggatt gcacgcaggt tctccggccg cttgggtgga gaggctattc 60
ggctatgact gggcacaaca gacaatcggc tgctctgatg ccgccgtgtt ccggctgtca 120
gcgcaggggc gcccggttct ttttgtcaag accgacctgt ccggtgccct gaatgaactg 180
caagacgagg cagcgcggct atcgtggctg gccacgacgg gcgttccttg cgcagctgtg 240
ctcgacgttg tcactgaagc gggaagggac tggctgctat tgggcgaagt gccggggcag 300
gatctcctgt catctcacct tgctcctgcc gagaaagtat ccatcatggc tgatgcaatg 360
cggcggctgc atacgcttga tccggctacc tgcccattcg accaccaagc gaaacatcgc 420
atcgagcgag cacgtactcg gatggaagcc ggtcttgtcg atcaggatga tctggacgaa 480
gagcatcagg ggctcgcgcc agccgaactg ttcgccaggc tcaaggcgag catgcccgac 540
ggcgaggatc tcgtcgtgac ccatggcgat gcctgcttgc cgaatatcat ggtggaaaat 600
ggccgctttt ctggattcat cgactgtggc cggctgggtg tggcggaccg ctatcaggac 660
atagcgttgg ctacccgtga tattgctgaa gagcttggcg gcgaatgggc tgaccgcttc 720
ctcgtgcttt acggtatcgc cgctcccgat tcgcagcgca tcgccttcta tcgccttctt 780
gacgagttct tctga                                                  795


<210> 2
<211> 264
<212> PRT
<213> Escherichia coli

<400> 2
Met Ile Glu Gln Asp Gly Leu His Ala Gly Ser Pro Ala Ala Trp Val
1               5                   10                  15

Glu Arg Leu Phe Gly Tyr Asp Trp Ala Gln Gln Thr Ile Gly Cys Ser
            20                  25                  30

Asp Ala Ala Val Phe Arg Leu Ser Ala Gln Gly Arg Pro Val Leu Phe
        35                  40                  45

Val Lys Thr Asp Leu Ser Gly Ala Leu Asn Glu Leu Gln Asp Glu Ala
    50                  55                  60

Ala Arg Leu Ser Trp Leu Ala Thr Thr Gly Val Pro Cys Ala Ala Val
65                  70                  75                  80

Leu Asp Val Val Thr Glu Ala Gly Arg Asp Trp Leu Leu Leu Gly Glu
                85                  90                  95

Val Pro Gly Gln Asp Leu Leu Ser Ser His Leu Ala Pro Ala Glu Lys
            100                 105                 110

Val Ser Ile Met Ala Asp Ala Met Arg Arg Leu His Thr Leu Asp Pro
        115                 120                 125

Ala Thr Cys Pro Phe Asp His Gln Ala Lys His Arg Ile Glu Arg Ala
    130                 135                 140

Arg Thr Arg Met Glu Ala Gly Leu Val Asp Gln Asp Asp Leu Asp Glu
145                 150                 155                 160

Glu His Gln Gly Leu Ala Pro Ala Glu Leu Phe Ala Arg Leu Lys Ala
                165                 170                 175

Ser Met Pro Asp Gly Glu Asp Leu Val Val Thr His Gly Asp Ala Cys
            180                 185                 190

Leu Pro Asn Ile Met Val Glu Asn Gly Arg Phe Ser Gly Phe Ile Asp
        195                 200                 205

Cys Gly Arg Leu Gly Val Ala Asp Arg Tyr Gln Asp Ile Ala Leu Ala
    210                 215                 220

Thr Arg Asp Ile Ala Glu Glu Leu Gly Gly Glu Trp Ala Asp Arg Phe
225                 230                 235                 240

Leu Val Leu Tyr Gly Ile Ala Ala Pro Asp Ser Gln Arg Ile Ala Phe
                245                 250                 255

Tyr Arg Leu Leu Asp Glu Phe Phe
            260


<210> 3
<211> 795
<212> DNA
<213> Künstliche Sequenz

<220>
<223> Beschreibung der künstlichen Sequenz:
      Neomycin-Mutante E182G

<400> 3
atgattgaac aagatggatt gcacgcaggt tctccggccg cttgggtgga gaggctattc 60
ggctatgact gggcacaaca gacaatcggc tgctctgatg ccgccgtgtt ccggctgtca 120
gcgcaggggc gcccggttct ttttgtcaag accgacctgt ccggtgccct gaatgaactg 180
caagacgagg cagcgcggct atcgtggctg gccacgacgg gcgttccttg cgcagctgtg 240
ctcgacgttg tcactgaagc gggaagggac tggctgctat tgggcgaagt gccggggcag 300
gatctcctgt catctcacct tgctcctgcc gagaaagtat ccatcatggc tgatgcaatg 360
cggcggctgc atacgcttga tccggctacc tgcccattcg accaccaagc gaaacatcgc 420
atcgagcgag cacgtactcg gatggaagcc ggtcttgtcg atcaggatga tctggacgaa 480
gagcatcagg ggctcgcgcc agccgaactg ttcgccaggc tcaaggcgag catgcccgac 540
ggcggggatc tcgtcgtgac ccatggcgat gcctgcttgc cgaatatcat ggtggaaaat 600
ggccgctttt ctggattcat cgactgtggc cggctgggtg tggcggaccg ctatcaggac 660
atagcgttgg ctacccgtga tattgctgaa gagcttggcg gcgaatgggc tgaccgcttc 720
ctcgtgcttt acggtatcgc cgctcccgat tcgcagcgca tcgccttcta tcgccttctt 780
gacgagttct tctga                                                  795


<210> 4
<211> 264
<212> PRT
<213> Künstliche Sequenz

<220>
<223> Beschreibung der künstlichen Sequenz:
      Neomycin-Mutante E182G

<400> 4
Met Ile Glu Gln Asp Gly Leu His Ala Gly Ser Pro Ala Ala Trp Val
  1               5                  10                  15

Glu Arg Leu Phe Gly Tyr Asp Trp Ala Gln Gln Thr Ile Gly Cys Ser
             20                  25                  30

Asp Ala Ala Val Phe Arg Leu Ser Ala Gln Gly Arg Pro Val Leu Phe
        35                  40                  45

Val Lys Thr Asp Leu Ser Gly Ala Leu Asn Glu Leu Gln Asp Glu Ala
    50                  55                  60

Ala Arg Leu Ser Trp Leu Ala Thr Thr Gly Val Pro Cys Ala Ala Val
65                  70                  75                  80

Leu Asp Val Val Thr Glu Ala Gly Arg Asp Trp Leu Leu Leu Gly Glu
                85                  90                  95

Val Pro Gly Gln Asp Leu Leu Ser Ser His Leu Ala Pro Ala Glu Lys
            100                 105                 110

Val Ser Ile Met Ala Asp Ala Met Arg Arg Leu His Thr Leu Asp Pro
        115                 120                 125

Ala Thr Cys Pro Phe Asp His Gln Ala Lys His Arg Ile Glu Arg Ala
    130                 135                 140

Arg Thr Arg Met Glu Ala Gly Leu Val Asp Gln Asp Asp Leu Asp Glu
145                 150                 155                 160

Glu His Gln Gly Leu Ala Pro Ala Glu Leu Phe Ala Arg Leu Lys Ala
                165                 170                 175

Ser Met Pro Asp Gly Gly Asp Leu Val Val Thr His Gly Asp Ala Cys
            180                 185                 190

Leu Pro Asn Ile Met Val Glu Asn Gly Arg Phe Ser Gly Phe Ile Asp
        195                 200                 205

Cys Gly Arg Leu Gly Val Ala Asp Arg Tyr Gln Asp Ile Ala Leu Ala
    210                 215                 220

Thr Arg Asp Ile Ala Glu Glu Leu Gly Gly Glu Trp Ala Asp Arg Phe
225                 230                 235                 240

Leu Val Leu Tyr Gly Ile Ala Ala Pro Asp Ser Gln Arg Ile Ala Phe
                245                 250                 255

Tyr Arg Leu Leu Asp Glu Phe Phe
            260


<210> 5
<211> 795
<212> DNA
<213> Künstliche Sequenz

<220>
<223> Beschreibung der künstlichen Sequenz:
      Neomycin-Mutante W91A

<400> 5
atgattgaac aagatggatt gcacgcaggt tctccggccg cttgggtgga gaggctattc 60
ggctatgact gggcacaaca gacaatcggc tgctctgatg ccgccgtgtt ccggctgtca 120
gcgcaggggc gcccggttct ttttgtcaag accgacctgt ccggtgccct gaatgaactg 180
caagacgagg cagcgcggct atcgtggctg gccacgacgg gcgttccttg cgcagctgtg 240
ctcgacgttg tcactgaagc gggaagggac gcgctgctat tgggcgaagt gccggggcag 300
gatctcctgt catctcacct tgctcctgcc gagaaagtat ccatcatggc tgatgcaatg 360
cggcggctgc atacgcttga tccggctacc tgcccattcg accaccaagc gaaacatcgc 420
atcgagcgag cacgtactcg gatggaagcc ggtcttgtcg atcaggatga tctggacgaa 480
gagcatcagg ggctcgcgcc agccgaactg ttcgccaggc tcaaggcgag catgcccgac 540
ggcgaggatc tcgtcgtgac ccatggcgat gcctgcttgc cgaatatcat ggtggaaaat 600
ggccgctttt ctggattcat cgactgtggc cggctgggtg tggcggaccg ctatcaggac 660
atagcgttgg ctacccgtga tattgctgaa gagcttggcg gcgaatgggc tgaccgcttc 720

ctcgtgcttt acggtatcgc cgctcccgat tcgcagcgca tcgccttcta tcgccttctt 780
gacgagttct tctga 795

<210> 6
<211> 264
<212> PRT
<213> Künstliche Sequenz

<220>
<223> Beschreibung der künstlichen Sequenz:
Neomycin-Mutante W91A

<400> 6
Met Ile Glu Gln Asp Gly Leu His Ala Gly Ser Pro Ala Ala Trp Val
1               5                   10                  15

Glu Arg Leu Phe Gly Tyr Asp Trp Ala Gln Gln Thr Ile Gly Cys Ser
            20                  25                  30

Asp Ala Ala Val Phe Arg Leu Ser Ala Gln Gly Arg Pro Val Leu Phe
        35                  40                  45

Val Lys Thr Asp Leu Ser Gly Ala Leu Asn Glu Leu Gln Asp Glu Ala
    50                  55                  60

Ala Arg Leu Ser Trp Leu Ala Thr Thr Gly Val Pro Cys Ala Ala Val
65                  70                  75                  80

Leu Asp Val Val Thr Glu Ala Gly Arg Asp Ala Leu Leu Leu Gly Glu
                85                  90                  95

Val Pro Gly Gln Asp Leu Leu Ser Ser His Leu Ala Pro Ala Glu Lys
            100                 105                 110

Val Ser Ile Met Ala Asp Ala Met Arg Arg Leu His Thr Leu Asp Pro
        115                 120                 125

Ala Thr Cys Pro Phe Asp His Gln Ala Lys His Arg Ile Glu Arg Ala
    130                 135                 140

Arg Thr Arg Met Glu Ala Gly Leu Val Asp Gln Asp Asp Leu Asp Glu
145                 150                 155                 160

Glu His Gln Gly Leu Ala Pro Ala Glu Leu Phe Ala Arg Leu Lys Ala
                165                 170                 175

Ser Met Pro Asp Gly Glu Asp Leu Val Val Thr His Gly Asp Ala Cys
            180                 185                 190

Leu Pro Asn Ile Met Val Glu Asn Gly Arg Phe Ser Gly Phe Ile Asp
        195                 200                 205

Cys Gly Arg Leu Gly Val Ala Asp Arg Tyr Gln Asp Ile Ala Leu Ala
    210                 215                 220

Thr Arg Asp Ile Ala Glu Glu Leu Gly Gly Glu Trp Ala Asp Arg Phe
225                 230                 235                 240

Leu Val Leu Tyr Gly Ile Ala Ala Pro Asp Ser Gln Arg Ile Ala Phe
                245                 250                 255

Tyr Arg Leu Leu Asp Glu Phe Phe
            260

<210> 7
<211> 795
<212> DNA

<213> Künstliche Sequenz

<220>
<223> Beschreibung der künstlichen Sequenz:
      Neomycin-Mutante V198G

<400> 7
atgattgaac aagatggatt gcacgcaggt tctccggccg cttgggtgga gaggctattc 60
ggctatgact gggcacaaca gacaatcggc tgctctgatg ccgccgtgtt ccggctgtca 120
gcgcaggggc gcccggttct ttttgtcaag accgacctgt ccggtgccct gaatgaactg 180
caagacgagg cagcgcggct atcgtggctg gccacgacgg gcgttccttg cgcagctgtg 240
ctcgacgttg tcactgaagc gggaagggac tggctgctat tgggcgaagt gccggggcag 300
gatctcctgt catctcacct tgctcctgcc gagaaagtat ccatcatggc tgatgcaatg 360
cggcggctgc atacgcttga tccggctacc tgcccattcg accaccaagc gaaacatcgc 420
atcgagcgag cacgtactcg gatggaagcc ggtcttgtcg atcaggatga tctggacgaa 480
gagcatcagg ggctcgcgcc agccgaactg ttcgccaggc tcaaggcgag catgcccgac 540
ggcgaggatc tcgtcgtgac ccatggcgat gcctgcttgc cgaatatcat gggggaaaat 600
ggccgctttt ctggattcat cgactgtggc cggctgggtg tggcggaccg ctatcaggac 660
atagcgttgg ctacccgtga tattgctgaa gagcttggcg gcgaatgggc tgaccgcttc 720
ctcgtgcttt acggtatcgc cgctcccgat tcgcagcgca tcgccttcta tcgccttctt 780
gacgagttct tctga                                                  795


<210> 8
<211> 264
<212> PRT
<213> Künstliche Sequenz

<220>
<223> Beschreibung der künstlichen Sequenz:
      Neomycin-Mutante V198G

<400> 8
Met Ile Glu Gln Asp Gly Leu His Ala Gly Ser Pro Ala Ala Trp Val
  1               5                  10                  15

Glu Arg Leu Phe Gly Tyr Asp Trp Ala Gln Gln Thr Ile Gly Cys Ser
             20                  25                  30

Asp Ala Ala Val Phe Arg Leu Ser Ala Gln Gly Arg Pro Val Leu Phe
         35                  40                  45

Val Lys Thr Asp Leu Ser Gly Ala Leu Asn Glu Leu Gln Asp Glu Ala
     50                  55                  60

Ala Arg Leu Ser Trp Leu Ala Thr Thr Gly Val Pro Cys Ala Ala Val
 65                  70                  75                  80

Leu Asp Val Val Thr Glu Ala Gly Arg Asp Trp Leu Leu Leu Gly Glu
                 85                  90                  95

Val Pro Gly Gln Asp Leu Leu Ser Ser His Leu Ala Pro Ala Glu Lys
            100                 105                 110

Val Ser Ile Met Ala Asp Ala Met Arg Arg Leu His Thr Leu Asp Pro
        115                 120                 125

Ala Thr Cys Pro Phe Asp His Gln Ala Lys His Arg Ile Glu Arg Ala
    130                 135                 140

Arg Thr Arg Met Glu Ala Gly Leu Val Asp Gln Asp Asp Leu Asp Glu
145                 150                 155                 160

Glu His Gln Gly Leu Ala Pro Ala Glu Leu Phe Ala Arg Leu Lys Ala
                165                 170                 175

Ser Met Pro Asp Gly Glu Asp Leu Val Val Thr His Gly Asp Ala Cys
            180                 185                 190

Leu Pro Asn Ile Met Gly Glu Asn Gly Arg Phe Ser Gly Phe Ile Asp
        195                 200                 205

Cys Gly Arg Leu Gly Val Ala Asp Arg Tyr Gln Asp Ile Ala Leu Ala
    210                 215                 220

Thr Arg Asp Ile Ala Glu Glu Leu Gly Gly Glu Trp Ala Asp Arg Phe
225                 230                 235                 240

Leu Val Leu Tyr Gly Ile Ala Ala Pro Asp Ser Gln Arg Ile Ala Phe
                245                 250                 255

Tyr Arg Leu Leu Asp Glu Phe Phe
            260


<210> 9
<211> 795
<212> DNA
<213> Künstliche Sequenz

<220>
<223> Beschreibung der künstlichen Sequenz:
      Neomycin-Mutante D227A

<400> 9
atgattgaac aagatggatt gcacgcaggt tctccggccg cttgggtgga gaggctattc 60
ggctatgact gggcacaaca gacaatcggc tgctctgatg ccgccgtgtt ccggctgtca 120
gcgcaggggc gcccggttct ttttgtcaag accgacctgt ccggtgccct gaatgaactg 180
caagacgagg cagcgcggct atcgtggctg gccacgacgg gcgttccttg cgcagctgtg 240
ctcgacgttg tcactgaagc gggaagggac tggctgctat tgggcgaagt gccggggcag 300
gatctcctgt catctcacct tgctcctgcc gagaaagtat ccatcatggc tgatgcaatg 360
cggcggctgc atacgcttga tccggctacc tgcccattcg accaccaagc gaaacatcgc 420
atcgagcgag cacgtactcg gatggaagcc ggtcttgtcg atcaggatga tctggacgaa 480
gagcatcagg ggctcgcgcc agccgaactg ttcgccaggc tcaaggcgag catgcccgac 540
ggcgaggatc tcgtcgtgac ccatggcgat gcctgcttgc cgaatatcat ggtggaaaat 600
ggccgctttt ctggattcat cgactgtggc cggctgggtg tggcggaccg ctatcaggac 660
atagcgttgg ctacccgtgc tattgctgaa gagcttggcg gcgaatgggc tgaccgcttc 720
ctcgtgcttt acggtatcgc cgctcccgat tcgcagcgca tcgccttcta tcgccttctt 780
gacgagttct tctga                                                  795


<210> 10
<211> 264
<212> PRT
<213> Künstliche Sequenz

<220>
<223> Beschreibung der künstlichen Sequenz:
      Neomycin-Mutante D227A

<400> 10
Met Ile Glu Gln Asp Gly Leu His Ala Gly Ser Pro Ala Ala Trp Val
  1               5                  10                  15

Glu Arg Leu Phe Gly Tyr Asp Trp Ala Gln Gln Thr Ile Gly Cys Ser
             20                  25                  30

Asp Ala Ala Val Phe Arg Leu Ser Ala Gln Gly Arg Pro Val Leu Phe
         35                  40                  45

Val Lys Thr Asp Leu Ser Gly Ala Leu Asn Glu Leu Gln Asp Glu Ala
     50                  55                  60

Ala Arg Leu Ser Trp Leu Ala Thr Thr Gly Val Pro Cys Ala Ala Val
 65                  70                  75                  80

Leu Asp Val Val Thr Glu Ala Gly Arg Asp Trp Leu Leu Leu Gly Glu
                 85                  90                  95

Val Pro Gly Gln Asp Leu Leu Ser Ser His Leu Ala Pro Ala Glu Lys
            100                 105                 110

Val Ser Ile Met Ala Asp Ala Met Arg Arg Leu His Thr Leu Asp Pro
        115                 120                 125

Ala Thr Cys Pro Phe Asp His Gln Ala Lys His Arg Ile Glu Arg Ala
    130                 135                 140

Arg Thr Arg Met Glu Ala Gly Leu Val Asp Gln Asp Asp Leu Asp Glu
145                 150                 155                 160

Glu His Gln Gly Leu Ala Pro Ala Glu Leu Phe Ala Arg Leu Lys Ala
                165                 170                 175

Ser Met Pro Asp Gly Glu Asp Leu Val Val Thr His Gly Asp Ala Cys
            180                 185                 190

Leu Pro Asn Ile Met Val Glu Asn Gly Arg Phe Ser Gly Phe Ile Asp
        195                 200                 205

Cys Gly Arg Leu Gly Val Ala Asp Arg Tyr Gln Asp Ile Ala Leu Ala
    210                 215                 220

Thr Arg Ala Ile Ala Glu Glu Leu Gly Gly Glu Trp Ala Asp Arg Phe
225                 230                 235                 240

Leu Val Leu Tyr Gly Ile Ala Ala Pro Asp Ser Gln Arg Ile Ala Phe
                245                 250                 255

Tyr Arg Leu Leu Asp Glu Phe Phe
            260

<210> 11
<211> 795
<212> DNA
<213> Künstliche Sequenz

<220>
<223> Beschreibung der künstlichen Sequenz:
      Neomycin-Mutante D227V

<400> 11
atgattgaac aagatggatt gcacgcaggt tctccggccg cttgggtgga gaggctattc 60
ggctatgact gggcacaaca gacaatcggc tgctctgatg ccgccgtgtt ccggctgtca 120
gcgcaggggc gcccggttct ttttgtcaag accgacctgt ccggtgccct gaatgaactg 180
caagacgagg cagcgcggct atcgtggctg gccacgacgg gcgttccttg cgcagctgtg 240
ctcgacgttg tcactgaagc gggaagggac tggctgctat tgggcgaagt gccggggcag 300
gatctcctgt catctcacct tgctcctgcc gagaaagtat ccatcatggc tgatgcaatg 360
cggcggctgc atacgcttga tccggctacc tgcccattcg accaccaagc gaaacatcgc 420
atcgagcgag cacgtactcg gatggaagcc ggtcttgtcg atcaggatga tctggacgaa 480
gagcatcagg ggctcgcgcc agccgaactg ttcgccaggc tcaaggcgag catgcccgac 540
ggcgaggatc tcgtcgtgac ccatggcgat gcctgcttgc cgaatatcat ggtggaaaat 600
ggccgctttt ctggattcat cgactgtggc cggctgggtg tggcggaccg ctatcaggac 660
atagcgttgg ctacccgtgt tattgctgaa gagcttggcg gcgaatgggc tgaccgcttc 720
ctcgtgcttt acggtatcgc cgctcccgat tcgcagcgca tcgccttcta tcgccttctt 780
gacgagttct tctga                                                  795

<210> 12
<211> 264
<212> PRT
<213> Künstliche Sequenz

<220>
<223> Beschreibung der künstlichen Sequenz:
      Neomycin-Mutante D227V

<400> 12
Met Ile Glu Gln Asp Gly Leu His Ala Gly Ser Pro Ala Ala Trp Val
1               5                   10                  15

Glu Arg Leu Phe Gly Tyr Asp Trp Ala Gln Gln Thr Ile Gly Cys Ser
            20                  25                  30

Asp Ala Ala Val Phe Arg Leu Ser Ala Gln Gly Arg Pro Val Leu Phe
        35                  40                  45

Val Lys Thr Asp Leu Ser Gly Ala Leu Asn Glu Leu Gln Asp Glu Ala
    50                  55                  60

Ala Arg Leu Ser Trp Leu Ala Thr Thr Gly Val Pro Cys Ala Ala Val
65                  70                  75                  80

Leu Asp Val Val Thr Glu Ala Gly Arg Asp Trp Leu Leu Leu Gly Glu
                85                  90                  95

Val Pro Gly Gln Asp Leu Leu Ser Ser His Leu Ala Pro Ala Glu Lys
            100                 105                 110

Val Ser Ile Met Ala Asp Ala Met Arg Arg Leu His Thr Leu Asp Pro
        115                 120                 125

Ala Thr Cys Pro Phe Asp His Gln Ala Lys His Arg Ile Glu Arg Ala
    130                 135                 140

Arg Thr Arg Met Glu Ala Gly Leu Val Asp Gln Asp Asp Leu Asp Glu
145                 150                 155                 160

Glu His Gln Gly Leu Ala Pro Ala Glu Leu Phe Ala Arg Leu Lys Ala
                165                 170                 175

Ser Met Pro Asp Gly Glu Asp Leu Val Val Thr His Gly Asp Ala Cys
            180                 185                 190

Leu Pro Asn Ile Met Val Glu Asn Gly Arg Phe Ser Gly Phe Ile Asp
        195                 200                 205

Cys Gly Arg Leu Gly Val Ala Asp Arg Tyr Gln Asp Ile Ala Leu Ala
    210                 215                 220

Thr Arg Val Ile Ala Glu Glu Leu Gly Gly Glu Trp Ala Asp Arg Phe
225                 230                 235                 240

Leu Val Leu Tyr Gly Ile Ala Ala Pro Asp Ser Gln Arg Ile Ala Phe
                245                 250                 255

Tyr Arg Leu Leu Asp Glu Phe Phe
            260


<210> 13
<211> 795
<212> DNA
<213> Künstliche Sequenz

<220>
<223> Beschreibung der künstlichen Sequenz:
      Neomycin-Mutante D261G

<400> 13
atgattgaac aagatggatt gcacgcaggt tctccggccg cttgggtgga gaggctattc 60
ggctatgact gggcacaaca gacaatcggc tgctctgatg ccgccgtgtt ccggctgtca 120
gcgcaggggc gcccggttct ttttgtcaag accgacctgt ccggtgccct gaatgaactg 180
caagacgagg cagcgcggct atcgtggctg gccacgacgg gcgttccttg cgcagctgtg 240
ctcgacgttg tcactgaagc gggaagggac tggctgctat tgggcgaagt gccggggcag 300

gatctcctgt catctcacct tgctcctgcc gagaaagtat ccatcatggc tgatgcaatg 360
cggcggctgc atacgcttga tccggctacc tgcccattcg accaccaagc gaaacatcgc 420
atcgagcgag cacgtactcg gatggaagcc ggtcttgtcg atcaggatga tctggacgaa 480
gagcatcagg ggctcgcgcc agccgaactg ttcgccaggc tcaaggcgag catgcccgac 540
ggcgaggatc tcgtcgtgac ccatggcgat gcctgcttgc cgaatatcat ggtggaaaat 600
ggccgctttt ctggattcat cgactgtggc cggctgggtg tggcggaccg ctatcaggac 660
atagcgttgg ctacccgtga tattgctgaa gagcttggcg gcgaatgggc tgaccgcttc 720
ctcgtgcttt acggtatcgc cgctcccgat tcgcagcgca tcgccttcta tcgccttctt 780
ggcgagttct tctga 795

<210> 14
<211> 264
<212> PRT
<213> Künstliche Sequenz

<220>
<223> Beschreibung der künstlichen Sequenz:
      Neomycin-Mutante D261G

<400> 14
Met Ile Glu Gln Asp Gly Leu His Ala Gly Ser Pro Ala Ala Trp Val
1               5                   10                  15

Glu Arg Leu Phe Gly Tyr Asp Trp Ala Gln Gln Thr Ile Gly Cys Ser
            20                  25                  30

Asp Ala Ala Val Phe Arg Leu Ser Ala Gln Gly Arg Pro Val Leu Phe
        35                  40                  45

Val Lys Thr Asp Leu Ser Gly Ala Leu Asn Glu Leu Gln Asp Glu Ala
    50                  55                  60

Ala Arg Leu Ser Trp Leu Ala Thr Thr Gly Val Pro Cys Ala Ala Val
65                  70                  75                  80

Leu Asp Val Val Thr Glu Ala Gly Arg Asp Trp Leu Leu Leu Gly Glu
                85                  90                  95

Val Pro Gly Gln Asp Leu Leu Ser Ser His Leu Ala Pro Ala Glu Lys
            100                 105                 110

Val Ser Ile Met Ala Asp Ala Met Arg Arg Leu His Thr Leu Asp Pro
        115                 120                 125

Ala Thr Cys Pro Phe Asp His Gln Ala Lys His Arg Ile Glu Arg Ala
    130                 135                 140

Arg Thr Arg Met Glu Ala Gly Leu Val Asp Gln Asp Asp Leu Asp Glu
145                 150                 155                 160

Glu His Gln Gly Leu Ala Pro Ala Glu Leu Phe Ala Arg Leu Lys Ala
                165                 170                 175

Ser Met Pro Asp Gly Glu Asp Leu Val Val Thr His Gly Asp Ala Cys
            180                 185                 190

Leu Pro Asn Ile Met Val Glu Asn Gly Arg Phe Ser Gly Phe Ile Asp
        195                 200                 205

Cys Gly Arg Leu Gly Val Ala Asp Arg Tyr Gln Asp Ile Ala Leu Ala
    210                 215                 220

Thr Arg Asp Ile Ala Glu Glu Leu Gly Gly Glu Trp Ala Asp Arg Phe
225                 230                 235                 240

Leu Val Leu Tyr Gly Ile Ala Ala Pro Asp Ser Gln Arg Ile Ala Phe
                245                 250                 255

Tyr Arg Leu Leu Gly Glu Phe Phe
            260


<210> 15
<211> 795
<212> DNA
<213> Künstliche Sequenz

<220>
<223> Beschreibung der künstlichen Sequenz:
      Neomycin-Mutante D261N

<400> 15
atgattgaac aagatggatt gcacgcaggt tctccggccg cttgggtgga gaggctattc 60
ggctatgact gggcacaaca gacaatcggc tgctctgatg ccgccgtgtt ccggctgtca 120
gcgcaggggc gcccggttct ttttgtcaag accgacctgt ccggtgccct gaatgaactg 180
caagacgagg cagcgcggct atcgtggctg gccacgacgg gcgttccttg cgcagctgtg 240
ctcgacgttg tcactgaagc gggaagggac tggctgctat tgggcgaagt gccggggcag 300
gatctcctgt catctcacct tgctcctgcc gagaaagtat ccatcatggc tgatgcaatg 360
cggcggctgc atacgcttga tccggctacc tgcccattcg accaccaagc gaaacatcgc 420
atcgagcgag cacgtactcg gatggaagcc ggtcttgtcg atcaggatga tctggacgaa 480
gagcatcagg ggctcgcgcc agccgaactg ttcgccaggc tcaaggcgag catgcccgac 540
ggcgaggatc tcgtcgtgac ccatggcgat gcctgcttgc cgaatatcat ggtggaaaat 600
ggccgctttt ctggattcat cgactgtggc cggctgggtg tggcggaccg ctatcaggac 660
atagcgttgg ctacccgtga tattgctgaa gagcttggcg gcgaatgggc tgaccgcttc 720
ctcgtgcttt acggtatcgc cgctcccgat tcgcagcgca tcgccttcta tcgccttctt 780
aacgagttct tctga                                                  795


<210> 16
<211> 264
<212> PRT
<213> Künstliche Sequenz

<220>
<223> Beschreibung der künstlichen Sequenz:
      Neomycin-Mutante D261N

<400> 16
Met Ile Glu Gln Asp Gly Leu His Ala Gly Ser Pro Ala Ala Trp Val
  1               5                  10                  15

Glu Arg Leu Phe Gly Tyr Asp Trp Ala Gln Gln Thr Ile Gly Cys Ser
             20                  25                  30

Asp Ala Ala Val Phe Arg Leu Ser Ala Gln Gly Arg Pro Val Leu Phe
         35                  40                  45

Val Lys Thr Asp Leu Ser Gly Ala Leu Asn Glu Leu Gln Asp Glu Ala
     50                  55                  60

Ala Arg Leu Ser Trp Leu Ala Thr Thr Gly Val Pro Cys Ala Ala Val
 65                  70                  75                  80

Leu Asp Val Val Thr Glu Ala Gly Arg Asp Trp Leu Leu Leu Gly Glu
                 85                  90                  95

Val Pro Gly Gln Asp Leu Leu Ser Ser His Leu Ala Pro Ala Glu Lys
            100                 105                 110

Val Ser Ile Met Ala Asp Ala Met Arg Arg Leu His Thr Leu Asp Pro
        115                 120                 125

Ala Thr Cys Pro Phe Asp His Gln Ala Lys His Arg Ile Glu Arg Ala
    130                 135                 140

Arg Thr Arg Met Glu Ala Gly Leu Val Asp Gln Asp Asp Leu Asp Glu
145                 150                 155                 160

Glu His Gln Gly Leu Ala Pro Ala Glu Leu Phe Ala Arg Leu Lys Ala
                165                 170                 175

Ser Met Pro Asp Gly Glu Asp Leu Val Val Thr His Gly Asp Ala Cys
            180                 185                 190

Leu Pro Asn Ile Met Val Glu Asn Gly Arg Phe Ser Gly Phe Ile Asp
        195                 200                 205

Cys Gly Arg Leu Gly Val Ala Asp Arg Tyr Gln Asp Ile Ala Leu Ala
    210                 215                 220

Thr Arg Asp Ile Ala Glu Glu Leu Gly Gly Glu Trp Ala Asp Arg Phe
225                 230                 235                 240

Leu Val Leu Tyr Gly Ile Ala Ala Pro Asp Ser Gln Arg Ile Ala Phe
                245                 250                 255

Tyr Arg Leu Leu Asn Glu Phe Phe
            260


<210> 17
<211> 795
<212> DNA
<213> Künstliche Sequenz

<220>
<223> Beschreibung der künstlichen Sequenz:
      Neomycin-Mutante F240I

<400> 17
atgattgaac aagatggatt gcacgcaggt tctccggccg cttgggtgga gaggctattc 60
ggctatgact gggcacaaca gacaatcggc tgctctgatg ccgccgtgtt ccggctgtca 120
gcgcaggggc gcccggttct ttttgtcaag accgacctgt ccggtgccct gaatgaactg 180
caagacgagg cagcgcggct atcgtggctg gccacgacgg gcgttccttg cgcagctgtg 240
ctcgacgttg tcactgaagc gggaagggac tggctgctat tgggcgaagt gccggggcag 300
gatctcctgt catctcacct tgctcctgcc gagaaagtat ccatcatggc tgatgcaatg 360
cggcggctgc atacgcttga tccggctacc tgcccattcg accaccaagc gaaacatcgc 420
atcgagcgag cacgtactcg gatggaagcc ggtcttgtcg atcaggatga tctggacgaa 480
gagcatcagg ggctcgcgcc agccgaactg ttcgccaggc tcaaggcgag catgcccgac 540
ggcgaggatc tcgtcgtgac ccatggcgat gcctgcttgc cgaatatcat ggtggaaaat 600
ggccgctttt ctggattcat cgactgtggc cggctgggtg tggcggaccg ctatcaggac 660
atagcgttgg ctacccgtga tattgctgaa gagcttggcg gcgaatgggc tgaccgcatc 720
ctcgtgcttt acggtatcgc cgctcccgat tcgcagcgca tcgccttcta tcgccttctt 780
gacgagttct tctga                                                  795


<210> 18
<211> 264
<212> PRT
<213> Künstliche Sequenz

<220>
<223> Beschreibung der künstlichen Sequenz:
      Neomycin-Mutante F240I

<400> 18
Met Ile Glu Gln Asp Gly Leu His Ala Gly Ser Pro Ala Ala Trp Val
  1               5                  10                  15

Glu Arg Leu Phe Gly Tyr Asp Trp Ala Gln Gln Thr Ile Gly Cys Ser
             20                  25                  30

Asp Ala Ala Val Phe Arg Leu Ser Ala Gln Gly Arg Pro Val Leu Phe
         35                  40                  45

Val Lys Thr Asp Leu Ser Gly Ala Leu Asn Glu Leu Gln Asp Glu Ala

50                  55                  60

Ala Arg Leu Ser Trp Leu Ala Thr Thr Gly Val Pro Cys Ala Ala Val
65                  70                  75                  80

Leu Asp Val Val Thr Glu Ala Gly Arg Asp Trp Leu Leu Leu Gly Glu
                85                  90                  95

Val Pro Gly Gln Asp Leu Leu Ser Ser His Leu Ala Pro Ala Glu Lys
            100                 105                 110

Val Ser Ile Met Ala Asp Ala Met Arg Arg Leu His Thr Leu Asp Pro
        115                 120                 125

Ala Thr Cys Pro Phe Asp His Gln Ala Lys His Arg Ile Glu Arg Ala
    130                 135                 140

Arg Thr Arg Met Glu Ala Gly Leu Val Asp Gln Asp Asp Leu Asp Glu
145                 150                 155                 160

Glu His Gln Gly Leu Ala Pro Ala Glu Leu Phe Ala Arg Leu Lys Ala
                165                 170                 175

Ser Met Pro Asp Gly Glu Asp Leu Val Val Thr His Gly Asp Ala Cys
            180                 185                 190

Leu Pro Asn Ile Met Val Glu Asn Gly Arg Phe Ser Gly Phe Ile Asp
        195                 200                 205

Cys Gly Arg Leu Gly Val Ala Asp Arg Tyr Gln Asp Ile Ala Leu Ala
    210                 215                 220

Thr Arg Asp Ile Ala Glu Glu Leu Gly Gly Glu Trp Ala Asp Arg Ile
225                 230                 235                 240

Leu Val Leu Tyr Gly Ile Ala Ala Pro Asp Ser Gln Arg Ile Ala Phe
                245                 250                 255

Tyr Arg Leu Leu Asp Glu Phe Phe
            260


<210> 19
<211> 795
<212> DNA
<213> Künstliche Sequenz

<220>
<223> Beschreibung der künstlichen Sequenz:
      Neomycin-Mutante E182D

<400> 19
atgattgaac aagatggatt gcacgcaggt tctccggccg cttgggtgga gaggctattc 60
ggctatgact gggcacaaca gacaatcggc tgctctgatg ccgccgtgtt ccggctgtca 120
gcgcaggggc gcccggttct ttttgtcaag accgacctgt ccggtgccct gaatgaactg 180
caagacgagg cagcgcggct atcgtggctg gccacgacgg gcgttccttg cgcagctgtg 240
ctcgacgttg tcactgaagc gggaagggac tggctgctat tgggcgaagt gccggggcag 300
gatctcctgt catctcacct tgctcctgcc gagaaagtat ccatcatggc tgatgcaatg 360
cggcggctgc atacgcttga tccggctacc tgcccattcg accaccaagc gaaacatcgc 420
atcgagcgag cacgtactcg gatggaagcc ggtcttgtcg atcaggatga tctggacgaa 480
gagcatcagg ggctcgcgcc agccgaactg ttcgccaggc tcaaggcgag catgcccgac 540
ggcgatgatc tcgtcgtgac ccatggcgat gcctgcttgc cgaatatcat ggtggaaaat 600
ggccgctttt ctggattcat cgactgtggc cggctgggtg tggcggaccg ctatcaggac 660
atagcgttgg ctacccgtga tattgctgaa gagcttggcg gcgaatgggc tgaccgcttc 720
ctcgtgcttt acggtatcgc cgctcccgat tcgcagcgca tcgccttcta tcgccttctt 780
gacgagttct tctga                                                  795


<210> 20

<211> 264
<212> PRT
<213> Künstliche Sequenz

<220>
<223> Beschreibung der künstlichen Sequenz:
      Neomycin-Mutante E182D

<400> 20
Met Ile Glu Gln Asp Gly Leu His Ala Gly Ser Pro Ala Ala Trp Val
 1               5                  10                  15

Glu Arg Leu Phe Gly Tyr Asp Trp Ala Gln Gln Thr Ile Gly Cys Ser
            20                  25                  30

Asp Ala Ala Val Phe Arg Leu Ser Ala Gln Gly Arg Pro Val Leu Phe
        35                  40                  45

Val Lys Thr Asp Leu Ser Gly Ala Leu Asn Glu Leu Gln Asp Glu Ala
    50                  55                  60

Ala Arg Leu Ser Trp Leu Ala Thr Thr Gly Val Pro Cys Ala Ala Val
65                  70                  75                  80

Leu Asp Val Val Thr Glu Ala Gly Arg Asp Trp Leu Leu Leu Gly Glu
                85                  90                  95

Val Pro Gly Gln Asp Leu Leu Ser Ser His Leu Ala Pro Ala Glu Lys
            100                 105                 110

Val Ser Ile Met Ala Asp Ala Met Arg Arg Leu His Thr Leu Asp Pro
        115                 120                 125

Ala Thr Cys Pro Phe Asp His Gln Ala Lys His Arg Ile Glu Arg Ala
    130                 135                 140

Arg Thr Arg Met Glu Ala Gly Leu Val Asp Gln Asp Asp Leu Asp Glu
145                 150                 155                 160

Glu His Gln Gly Leu Ala Pro Ala Glu Leu Phe Ala Arg Leu Lys Ala
                165                 170                 175

Ser Met Pro Asp Gly Asp Asp Leu Val Val Thr His Gly Asp Ala Cys
            180                 185                 190

Leu Pro Asn Ile Met Val Glu Asn Gly Arg Phe Ser Gly Phe Ile Asp
        195                 200                 205

Cys Gly Arg Leu Gly Val Ala Asp Arg Tyr Gln Asp Ile Ala Leu Ala
    210                 215                 220

Thr Arg Asp Ile Ala Glu Glu Leu Gly Gly Glu Trp Ala Asp Arg Phe
225                 230                 235                 240

Leu Val Leu Tyr Gly Ile Ala Ala Pro Asp Ser Gln Arg Ile Ala Phe
                245                 250                 255

Tyr Arg Leu Leu Asp Glu Phe Phe
            260


<210> 21
<211> 795
<212> DNA
<213> Künstliche Sequenz

<220>
<223> Beschreibung der künstlichen Sequenz:
      Neomycin-Mutante D227G

<400> 21
atgattgaac aagatggatt gcacgcaggt tctccggccg cttgggtgga gaggctattc 60
ggctatgact gggcacaaca gacaatcggc tgctctgatg ccgccgtgtt ccggctgtca 120
gcgcaggggc gcccggttct ttttgtcaag accgacctgt ccggtgccct gaatgaactg 180
caagacgagg cagcgcggct atcgtggctg gccacgacgg gcgttccttg cgcagctgtg 240
ctcgacgttg tcactgaagc gggaagggac tggctgctat tgggcgaagt gccggggcag 300
gatctcctgt catctcacct tgctcctgcc gagaaagtat ccatcatggc tgatgcaatg 360
cggcggctgc atacgcttga tccggctacc tgcccattcg accaccaagc gaaacatcgc 420
atcgagcgag cacgtactcg gatggaagcc ggtcttgtcg atcaggatga tctggacgaa 480
gagcatcagg ggctcgcgcc agccgaactg ttcgccaggc tcaaggcgag catgcccgac 540
ggcgaggatc tcgtcgtgac ccatggcgat gcctgcttgc cgaatatcat ggtggaaaat 600
ggccgctttt ctggattcat cgactgtggc cggctgggtg tggcggaccg ctatcaggac 660
atagcgttgg ctacccgtgg tattgctgaa gagcttggcg gcgaatgggc tgaccgcttc 720
ctcgtgcttt acggtatcgc cgctcccgat tcgcagcgca tcgccttcta tcgccttctt 780
gacgagttct tctga 795

<210> 22
<211> 264
<212> PRT
<213> Künstliche Sequenz

<220>
<223> Beschreibung der künstlichen Sequenz:
      Neomycin-Mutante D227G

<400> 22
Met Ile Glu Gln Asp Gly Leu His Ala Gly Ser Pro Ala Ala Trp Val
1               5                   10                  15

Glu Arg Leu Phe Gly Tyr Asp Trp Ala Gln Gln Thr Ile Gly Cys Ser
            20                  25                  30

Asp Ala Ala Val Phe Arg Leu Ser Ala Gln Gly Arg Pro Val Leu Phe
        35                  40                  45

Val Lys Thr Asp Leu Ser Gly Ala Leu Asn Glu Leu Gln Asp Glu Ala
    50                  55                  60

Ala Arg Leu Ser Trp Leu Ala Thr Thr Gly Val Pro Cys Ala Ala Val
65                  70                  75                  80

Leu Asp Val Val Thr Glu Ala Gly Arg Asp Trp Leu Leu Leu Gly Glu
                85                  90                  95

Val Pro Gly Gln Asp Leu Leu Ser Ser His Leu Ala Pro Ala Glu Lys
            100                 105                 110

Val Ser Ile Met Ala Asp Ala Met Arg Arg Leu His Thr Leu Asp Pro
        115                 120                 125

Ala Thr Cys Pro Phe Asp His Gln Ala Lys His Arg Ile Glu Arg Ala
    130                 135                 140

Arg Thr Arg Met Glu Ala Gly Leu Val Asp Gln Asp Asp Leu Asp Glu
145                 150                 155                 160

Glu His Gln Gly Leu Ala Pro Ala Glu Leu Phe Ala Arg Leu Lys Ala
                165                 170                 175

Ser Met Pro Asp Gly Glu Asp Leu Val Val Thr His Gly Asp Ala Cys
            180                 185                 190

Leu Pro Asn Ile Met Val Glu Asn Gly Arg Phe Ser Gly Phe Ile Asp
        195                 200                 205

Cys Gly Arg Leu Gly Val Ala Asp Arg Tyr Gln Asp Ile Ala Leu Ala
    210                 215                 220

Thr Arg Gly Ile Ala Glu Glu Leu Gly Gly Glu Trp Ala Asp Arg Phe
225                 230                 235                 240

Leu Val Leu Tyr Gly Ile Ala Ala Pro Asp Ser Gln Arg Ile Ala Phe
                245                 250                 255

Tyr Arg Leu Leu Asp Glu Phe Phe
            260


<210> 23
<211> 795
<212> DNA
<213> Künstliche Sequenz

<220>
<223> Beschreibung der künstlichen Sequenz:
      Neomycin-Mutante D190G

<400> 23
atgattgaac aagatggatt gcacgcaggt tctccggccg cttgggtgga gaggctattc 60
ggctatgact gggcacaaca gacaatcggc tgctctgatg ccgccgtgtt ccggctgtca 120
gcgcaggggc gcccggttct ttttgtcaag accgacctgt ccggtgccct gaatgaactg 180
caagacgagg cagcgcggct atcgtggctg gccacgacgg gcgttccttg cgcagctgtg 240
ctcgacgttg tcactgaagc gggaagggac tggctgctat tgggcgaagt gccggggcag 300
gatctcctgt catctcacct tgctcctgcc gagaaagtat ccatcatggc tgatgcaatg 360
cggcggctgc atacgcttga tccggctacc tgcccattcg accaccaagc gaaacatcgc 420
atcgagcgag cacgtactcg gatggaagcc ggtcttgtcg atcaggatga tctggacgaa 480
gagcatcagg ggctcgcgcc agccgaactg ttcgccaggc tcaaggcgag catgcccgac 540
ggcgaggatc tcgtcgtgac ccatggcggt gcctgcttgc cgaatatcat ggtggaaaat 600
ggccgctttt ctggattcat cgactgtggc cggctgggtg tggcggaccg ctatcaggac 660
atagcgttgg ctacccgtga tattgctgaa gagcttggcg gcgaatgggc tgaccgcttc 720
ctcgtgcttt acggtatcgc cgctcccgat tcgcagcgca tcgccttcta tcgccttctt 780
gacgagttct tctga                                                  795


<210> 24
<211> 264
<212> PRT
<213> Künstliche Sequenz

<220>
<223> Beschreibung der künstlichen Sequenz:
      Neomycin-Mutante D190G

<400> 24
Met Ile Glu Gln Asp Gly Leu His Ala Gly Ser Pro Ala Ala Trp Val
  1               5                  10                  15

Glu Arg Leu Phe Gly Tyr Asp Trp Ala Gln Gln Thr Ile Gly Cys Ser
             20                  25                  30

Asp Ala Ala Val Phe Arg Leu Ser Ala Gln Gly Arg Pro Val Leu Phe
         35                  40                  45

Val Lys Thr Asp Leu Ser Gly Ala Leu Asn Glu Leu Gln Asp Glu Ala
     50                  55                  60

Ala Arg Leu Ser Trp Leu Ala Thr Thr Gly Val Pro Cys Ala Ala Val
 65                  70                  75                  80

Leu Asp Val Val Thr Glu Ala Gly Arg Asp Trp Leu Leu Leu Gly Glu
                 85                  90                  95

Val Pro Gly Gln Asp Leu Leu Ser Ser His Leu Ala Pro Ala Glu Lys
            100                 105                 110

Val Ser Ile Met Ala Asp Ala Met Arg Arg Leu His Thr Leu Asp Pro

115                 120                 125

Ala Thr Cys Pro Phe Asp His Gln Ala Lys His Arg Ile Glu Arg Ala
    130                 135                 140

Arg Thr Arg Met Glu Ala Gly Leu Val Asp Gln Asp Asp Leu Asp Glu
145                 150                 155                 160

Glu His Gln Gly Leu Ala Pro Ala Glu Leu Phe Ala Arg Leu Lys Ala
                165                 170                 175

Ser Met Pro Asp Gly Glu Asp Leu Val Val Thr His Gly Gly Ala Cys
            180                 185                 190

Leu Pro Asn Ile Met Val Glu Asn Gly Arg Phe Ser Gly Phe Ile Asp
        195                 200                 205

Cys Gly Arg Leu Gly Val Ala Asp Arg Tyr Gln Asp Ile Ala Leu Ala
    210                 215                 220

Thr Arg Asp Ile Ala Glu Glu Leu Gly Gly Glu Trp Ala Asp Arg Phe
225                 230                 235                 240

Leu Val Leu Tyr Gly Ile Ala Ala Pro Asp Ser Gln Arg Ile Ala Phe
                245                 250                 255

Tyr Arg Leu Leu Asp Glu Phe Phe
            260


<210> 25
<211> 795
<212> DNA
<213> Künstliche Sequenz

<220>
<223> Beschreibung der künstlichen Sequenz:
      Neomycin-Mutante D208G

<400> 25
atgattgaac aagatggatt gcacgcaggt tctccggccg cttgggtgga gaggctattc 60
ggctatgact gggcacaaca gacaatcggc tgctctgatg ccgccgtgtt ccggctgtca 120
gcgcaggggc gcccggttct ttttgtcaag accgacctgt ccggtgccct gaatgaactg 180
caagacgagg cagcgcggct atcgtggctg gccacgacgg gcgttccttg cgcagctgtg 240
ctcgacgttg tcactgaagc gggaagggac tggctgctat tgggcgaagt gccggggcag 300
gatctcctgt catctcacct tgctcctgcc gagaaagtat ccatcatggc tgatgcaatg 360
cggcggctgc atacgcttga tccggctacc tgcccattcg accaccaagc gaaacatcgc 420
atcgagcgag cacgtactcg gatggaagcc ggtcttgtcg atcaggatga tctggacgaa 480
gagcatcagg ggctcgcgcc agccgaactg ttcgccaggc tcaaggcgag catgcccgac 540
ggcgaggatc tcgtcgtgac ccatggcgat gcctgcttgc cgaatatcat ggtggaaaat 600
ggccgctttt ctggattcat cggctgtggc cggctgggtg tggcggaccg ctatcaggac 660
atagcgttgg ctacccgtga tattgctgaa gagcttggcg gcgaatgggc tgaccgcttc 720
ctcgtgcttt acggtatcgc cgctcccgat tcgcagcgca tcgccttcta tcgccttctt 780
gacgagttct tctga                                                  795


<210> 26
<211> 264
<212> PRT
<213> Künstliche Sequenz

<220>
<223> Beschreibung der künstlichen Sequenz:
      Neomycin-Mutante D208G

<400> 26
Met Ile Glu Gln Asp Gly Leu His Ala Gly Ser Pro Ala Ala Trp Val
1               5                   10                  15

Glu Arg Leu Phe Gly Tyr Asp Trp Ala Gln Gln Thr Ile Gly Cys Ser
                20                  25                  30

Asp Ala Ala Val Phe Arg Leu Ser Ala Gln Gly Arg Pro Val Leu Phe
            35                  40                  45

Val Lys Thr Asp Leu Ser Gly Ala Leu Asn Glu Leu Gln Asp Glu Ala
    50                  55                  60

Ala Arg Leu Ser Trp Leu Ala Thr Thr Gly Val Pro Cys Ala Ala Val
65                  70                  75                  80

Leu Asp Val Val Thr Glu Ala Gly Arg Asp Trp Leu Leu Leu Gly Glu
                85                  90                  95

Val Pro Gly Gln Asp Leu Leu Ser Ser His Leu Ala Pro Ala Glu Lys
            100                 105                 110

Val Ser Ile Met Ala Asp Ala Met Arg Arg Leu His Thr Leu Asp Pro
        115                 120                 125

Ala Thr Cys Pro Phe Asp His Gln Ala Lys His Arg Ile Glu Arg Ala
    130                 135                 140

Arg Thr Arg Met Glu Ala Gly Leu Val Asp Gln Asp Asp Leu Asp Glu
145                 150                 155                 160

Glu His Gln Gly Leu Ala Pro Ala Glu Leu Phe Ala Arg Leu Lys Ala
                165                 170                 175

Ser Met Pro Asp Gly Glu Asp Leu Val Val Thr His Gly Asp Ala Cys
            180                 185                 190

Leu Pro Asn Ile Met Val Glu Asn Gly Arg Phe Ser Gly Phe Ile Gly
        195                 200                 205

Cys Gly Arg Leu Gly Val Ala Asp Arg Tyr Gln Asp Ile Ala Leu Ala
    210                 215                 220

Thr Arg Asp Ile Ala Glu Glu Leu Gly Gly Glu Trp Ala Asp Arg Phe
225                 230                 235                 240

Leu Val Leu Tyr Gly Ile Ala Ala Pro Asp Ser Gln Arg Ile Ala Phe
                245                 250                 255

Tyr Arg Leu Leu Asp Glu Phe Phe
            260


<210> 27
<211> 21
<212> DNA
<213> Künstliche Sequenz

<220>
<223> Beschreibung der künstlichen Sequenz:
      Oligonukleotid Neofor5

<400> 27
ttccagaagt agtgaggagg c                                                21


<210> 28
<211> 19
<212> DNA
<213> Künstliche Sequenz

<220>
<223> Beschreibung der künstlichen Sequenz:

Oligonukleotid Neorev5

<400> 28
atggcaggtt gggcgtcgc                                                    19


<210> 29
<211> 21
<212> DNA
<213> Künstliche Sequenz

<220>
<223> Beschreibung der künstlichen Sequenz:
      Oligonukleotid Neofor2

<400> 29
gaactgttcg ccaggctcaa g                                                 21


<210> 30
<211> 22
<212> DNA
<213> Künstliche Sequenz

<220>
<223> Beschreibung der künstlichen Sequenz:
      Oligonukleotid IC49

<400> 30
cggcaaaatc ccttataaat ca                                                22


<210> 31
<211> 20
<212> DNA
<213> Künstliche Sequenz

<220>
<223> Beschreibung der künstlichen Sequenz:
      Oligonukleotid E182Gfor

<400> 31
gacggcgggg atctcgtcgt          20


<210> 32
<211> 20
<212> DNA
<213> Künstliche Sequenz

<220>
<223> Beschreibung der künstlichen Sequenz:
      Oligonukleotid E182Grev

<400> 32
acgacgagat ccccgccgtc                                                   20


<210> 33
<211> 23
<212> DNA
<213> Künstliche Sequenz

<220>
<223> Beschreibung der künstlichen Sequenz:
      Oligonukleotid W91Afor

<400> 33
gggaagggac gcgctgctat tgg                                               23

<210> 34
<211> 23
<212> DNA
<213> Künstliche Sequenz

<220>
<223> Beschreibung der künstlichen Sequenz:
      Oligonukleotid W91Arev

<400> 34
ccaatagcag cgcgtccctt ccc                                              23


<210> 35
<211> 24
<212> DNA
<213> Künstliche Sequenz

<220>
<223> Beschreibung der künstlichen Sequenz:
      Oligonukleotid V198Gfor

<400> 35
ccgaatatca tgggggaaaa tggc                                             24


<210> 36
<211> 24
<212> DNA
<213> Künstliche Sequenz

<220>
<223> Beschreibung der künstlichen Sequenz:
      Oligonukleotid V198Grev

<400> 36
gccattttcc cccatgatat tcgg                                             24


<210> 37
<211> 21
<212> DNA
<213> Künstliche Sequenz

<220>
<223> Beschreibung der künstlichen Sequenz:
      Oligonukleotid D227Afor

<400> 37
ctacccgtgc tattgctgaa g                                                21


<210> 38
<211> 21
<212> DNA
<213> Künstliche Sequenz

<220>
<223> Beschreibung der künstlichen Sequenz:
      Oligonukleotid D227Arev

<400> 38
cttcagcaat agcacgggta g                                                21


<210> 39
<211> 21

<212> DNA
<213> Künstliche Sequenz

<220>
<223> Beschreibung der künstlichen Sequenz:
      Oligonukleotid D227Vfor

<400> 39
ctacccgtgt tattgctgaa g                                                21


<210> 40
<211> 21
<212> DNA
<213> Künstliche Sequenz

<220>
<223> Beschreibung der künstlichen Sequenz:
      Oligonukleotid D227Vrev

<400> 40
cttcagcaat aacacgggta g                                                21


<210> 41
<211> 24
<212> DNA
<213> Künstliche Sequenz

<220>
<223> Beschreibung der künstlichen Sequenz:
      Oligonukleotid D261Gfor

<400> 41
gccttcttgg cgagttcttc tgag                                             24


<210> 42
<211> 24
<212> DNA
<213> Künstliche Sequenz

<220>
<223> Beschreibung der künstlichen Sequenz:
      Oligonukleotid D261Grev

<400> 42
ctcagaagaa ctcgccaaga aggc                                             24


<210> 43
<211> 24
<212> DNA
<213> Künstliche Sequenz

<220>
<223> Beschreibung der künstlichen Sequenz:
      Oligonukleotid D261Nfor

<400> 43
gccttcttaa cgagttcttc tgag                                             24


<210> 44
<211> 24
<212> DNA
<213> Künstliche Sequenz

<220>

<223> Beschreibung der künstlichen Sequenz:
      Oligonukleotid D261Nrev

<400> 44
ctcagaagaa ctcgttaaga aggc                                              24


<210> 45
<211> 22
<212> DNA
<213> Künstliche Sequenz

<220>
<223> Beschreibung der künstlichen Sequenz:
      Oligonukleotid F240Ifor

<400> 45
ggctgaccgc atcctcgtgc tt                                                22


<210> 46
<211> 22
<212> DNA
<213> Künstliche Sequenz

<220>
<223> Beschreibung der künstlichen Sequenz:
      Oligonukleotid F240Irev

<400> 46
aagcacgagg atgcggtcag cc                                                22


<210> 47
<211> 20
<212> DNA
<213> Künstliche Sequenz

<220>
<223> Beschreibung der künstlichen Sequenz:
      Oligonucleotid E182Dfor

<400> 47
gacggcgatg atctcgtcgt                                                20


<210> 48
<211> 20
<212> DNA
<213> Künstliche Sequenz

<220>
<223> Beschreibung der künstlichen Sequenz:
      Oligonucleotid E182Drev

<400> 48
acgacgagat catcgccgtc                                                20


<210> 49
<211> 19
<212> DNA
<213> Künstliche Sequenz

<220>
<223> Beschreibung der künstlichen Sequenz:
      Oligonucleotid D190Gfor

<400> 49

catggcggtg cctgcttgc                                                    19


<210> 50
<211> 19
<212> DNA
<213> Künstliche Sequenz

<220>
<223> Beschreibung der künstlichen Sequenz:
      Oligonucleotid D190Grev

<400> 50
gcaagcaggc accgccatg                                                    19


<210> 51
<211> 19
<212> DNA
<213> Künstliche Sequenz

<220>
<223> Beschreibung der künstlichen Sequenz:
      Oligonucleotid D208Gfor

<400> 51
gattcatcgg ctgtggccg                                                    19


<210> 52
<211> 19
<212> DNA
<213> Künstliche Sequenz

<220>
<223> Beschreibung der künstlichen Sequenz:
      Oligonucleotid D208Grev

<400> 52
cggccacagc cgatgaatc                                                    19


<210> 53
<211> 21
<212> DNA
<213> Künstliche Sequenz

<220>
<223> Beschreibung der künstlichen Sequenz:
      Oligonucleotid D227Gfor

<400> 53
ctacccgtgg tattgctgaa g                                                 21


<210> 54
<211> 21
<212> DNA
<213> Künstliche Sequenz

<220>
<223> Beschreibung der künstlichen Sequenz:
      Oligonucleotid D227Grev

<400> 54
cttcagcaat accacgggta g                                                 21


<210> 55

<211> 2406
<212> DNA
<213> Cricetulus griseus

<300>
<310> PCT/EP/96/04631
<311> 1996-10-24
<312> 1997-05-01

<400> 55
gatctccagg acagccatgg ctattacaca gagaaaccct gtctggaaaa acaaaaaatt 60
agtgtccatg tgtaaatgtg tggagtatgc ttgtcatgcc acatacagag gtagagggca 120
gtttatggga gtcagttcct attcttcctt tatgggggac ctggggactg aactcaggtc 180
atcaggcttg gcagaaagtg cattagctca cggagcctta tcattggcga aagctctctc 240
aagtagaaaa tcaatgtgtt tgctcatagt gcaatcatta tgtttcgaga ggggaagggt 300
acaatcgttg gggcatgtgt ggtcacatct gaatagcagt agctccctag gagaattcca 360
agttctttgg tggtgtatca atgcccttaa aggggtcaac aacttttttt ccctctgaca 420
aaactatctt cttatgtcct tgtccctcat atttgaagta ttttattctt tgcagtgttg 480
aatatcaatt ctagcacctc agacatgtta ggtaagtacc ctacaactca ggttaactaa 540
tttaatttaa ctaatttaac cccaacactt tttctttgtt tatccacatt tgtggagtgt 600
gtgtgtgtgt gtgtgtgtgt gtgtgtgtgt gtgtgtgtgt gtgtgtgtgt gtgtgtgtgc 660
gcgcgcgcgc gcgctcggat cattctacct tttgtttaaa aaatgttagt ccaggggtgg 720
ggtgcactgt gaaagtctga gggtaacttg ctggggtcag ttctttccac tataggacag 780
aactccaggt gtcaactctt tactgacaga accatccaaa tagccctatc taattttagt 840
tttttattta tttatttttt gtttttcgag acagggtttc tctgtggctt tggaggctgt 900
cctggaacta gctcttgtag accaggctgg tctcgaactc agagatccac ctgcctctgc 960
ctcctgagtg ctgggattaa aggcatgcgc caccaacgct tggctctacc taattttaaa 1020
agagattgtg tgtcacaagg gtgtcatgtc gccctgcaac cacccccccc ccaaaaaaaa 1080
aaaaaaaaaa acttcactga agctgaagca cgatgatttg gttactctgg ctggccaatg 1140
agctctaggg agtctcctgt caaacagaat ctcaacaggc gcagcagtct tttttaaagt 1200
ggggttacaa cacaggtttt tgcatatcag gcattttatc taagctattt cccagccaaa 1260
aatgtgtatt ttggaggcag cagagctaat agattaaaat gagggaagag cccacacagg 1320
ttattaggaa gataagcatc ttctttatat aaaacaaaac caaaccaaac tggaggaggt 1380
ctacctttag ggatggaaga aaagacattt agagggtgca atagaaaggg cactgagttt 1440
gtgaggtgga ggactgggag agggcgcaac cgctttaact gtcctgtttt gcctattttt 1500
tggggacagc acatgttcct atttttccca ggatgggcaa tctccacgtc caaacttgcg 1560
gtcgaggact acagtcattt tgcaggtttc cttactgtat ggcttttaaa acgtgcaaag 1620
gtgaccatta accgtttcac gctgggaggg cacgtgcggc tcagatgctt cctctgactg 1680
agggccagga gggggctaca cggaagaggc cacacccgca cttgggaaga ctcgatttgg 1740
gcttcagctg gctgagacgc cccagcaggc tcctcggcta caccttcagc cccgaatgcc 1800
ttccggccca taacccttcc cttctaggca tttccggcga ggacccaccc tcgcgccaaa 1860
cattcggccc catcccccgg tcctcacctg aatctctaac tctgactcca gagtttagag 1920
actataacca gatagcccgg atgtgtggaa ctgcatcttg ggacgagtag ttttagcaaa 1980
aagaaagcga cgaaaaacta caattcccag acagacttgt gttacctctc ttctcatgct 2040
aaacaagccc cctttaaagg aaagcccctc ttagtcgcat cgactgtgta agaaaggcgt 2100
ttgaaacatt ttaatgttgg gcacaccgtt tcgaggaccg aaatgagaaa gagcataggg 2160
aaacggagcg cccgagctag tctggcactg cgttagacag ccgcggtcgt tgcagcgggc 2220
aggcacttgc gtggacgcct aaggggcggg tctttcggcc gggaagcccc gttggtccgc 2280
gcggctcttc ctttccgatc cgccatccgt ggtgagtgtg tgctgcgggc tgccgctccg 2340
gcttggggct tcccgcgtcg ctctcaccct ggtcggcggc tctaatccgt ctcttttcga 2400
atgtag 2406

## Beschreibung

### Gebiet der Erfindung

Die Erfindung betrifft neue modifizierte Neomycin-Phosphotransferase-Gene sowie deren Verwendung in Selektionsverfahren für hochproduzierende rekombinante Zellen. Dementsprechend betrifft die vorliegende Erfindung auch neue Expressionsvektoren, die ein erfindungsgemäßes modifiziertes Neomycin-Phosphotransferase-Gen enthalten, vorzugsweise in Kombination mit einem Gen von Interesse in funktioneller Verknüpfung mit einem heterologen Promotor. Die Erfindung betrifft ferner Verfahren zur Herstellung von heterologen Genprodukten mit Hilfe der entsprechenden hochproduzierenden rekombinanten Zellen.

### Hintergrund der Erfindung

Säugerzellen sind die bevorzugten Wirtszellen zur Produktion komplexer biopharmazeutischer Proteine, da die post-translational durchgeführten Modifikationen sowohl in funktionaler als auch in pharmakokinetischer Hinsicht humankompatibel sind. Vornehmlich relevante Zelltypen sind Hybridomas, Myelomas, CHO- *(Chinese Hamster Ovary)* Zellen und BHK- *(Baby Hamster Kidney)* Zellen. Die Kultivierung der Wirtszellen erfolgt zunehmend unter serum- und proteinfreien Produktionsbedingungen. Gründe hierfür sind die damit verbundene Kostenreduktion, die geringere Interferenz bei der Aufreinigung des rekombinanten Proteins sowie die Reduktion des Potentials zur Einführung von Pathogenen (z.B. Prionen, Viren). Der Einsatz von CHO-Zellen als Wirtszellen findet immer weitere Verbreitung, da diese Zellen sich an Suspensionswachstum in serum- und proteinfreiem Medium adaptieren lassen und zudem von den regulatorischen Behörden als sichere Produktionszellen angesehen und akzeptiert werden.

Zur Erzeugung einer stabilen Säugerzelllinie, die ein heterologes Gen von Interesse (GOI) exprimiert, wird das heterologe Gen in der Regel gemeinsam mit einem selektierbaren Markergen, wie z.B. Neomycin-Phosphotransferase (NPT), durch Transfektion in die gewünschten Zelllinie eingebracht. Das heterologe Gen und das selektierbare Markergen lassen sich in einer Wirtszelle, ausgehend von einem einzelnen oder von separaten, co-transfizierten Vektoren, exprimieren. Zwei bis drei Tage nach der Transfektion werden die transfizierten Zellen in Medium überführt, das ein selektives Agens enthält, z.B. G418 bei Verwendung des Neomycin-Phosphotransferase-Gens (NPT-Gen), und für einige Wochen unter diesen selektiven Bedingungen kultiviert. Die hochwachsenden resistenten Zellen, die die exogene DNA integriert haben, können isoliert und hinsichtlich der Expression des gewünschten Genprodukts (des GOI) untersucht werden.

Ein großes Problem bei der Etablierung von Zelllinien mit hoher Expression des gewünschten Proteins ergibt sich aus der willkürlichen und ungerichteten Integration des rekombinanten Vektors in transkriptionsaktive oder -inaktive Loci des Wirtszellgenoms. Dadurch erhält man eine Population von Zellen, die völlig unterschiedliche Expressionsraten des heterologen Gens aufweisen, wobei die Produktivität der Zellen in der Regel einer Normalverteilung folgt. Zur Identifizierung von Zellklonen, die eine sehr hohe Expression des heterologen Gens von Interesse aufweisen, muss deshalb eine Vielzahl von Klonen überprüft und getestet werden, resultierend in einem hohen Zeit-, Arbeits- und Kostenaufwand. Optimierungen des zur Transfektion eingesetzten Vektorsystems zielen deshalb darauf ab, durch geeignete Selektionsstrategien den Anteil von Hochproduzenten in der transfizierten Zellpopulation zu erhöhen und somit den Aufwand in der Klonidentifizierung zu reduzieren. Die Entwicklung eines derartigen Expressionssystems ist Gegenstand der vorliegenden Erfindung.

Das Aminoglycosid-3'-Phosphotransferase II Enzym (Neomycin-Phosphotransferase) (EC 2.7.1.95), dessen Gen in *Escherichia coli* Transposon 5-assoziiert ist, wird als Selektionsmarker in einer Vielzahl von Organismen (z.B. Bakterien, Hefen, Pflanzen, Säugerzellen) verwendet. Dieses Enzym vermittelt Resistenz gegenüber verschiedenen aminoglykosidischen Antibiotika wie Neomycin, Kanamycin und G418, indem es die Antibiotika durch den Transfer des terminalen Phosphats von ATP auf die 3'Hydroxylgruppe des Aminohexose-Rings I inaktiviert.

Neben der Wildtyp-Neomycin-Phosphotransferase (wt NPT) sind einige Mutanten bekannt, die eine reduzierte Phosphotransferase-Aktivität und damit eine verminderten Resistenz gegenüber aminoglykosidischen Antibiotika in Bakterien (Blázquez et al., 1991; Kocabiyik et al., 1992; Yenofsky et al., 1990) und in Blattscheiben von Tabak (Yenofsky et al., 1990) aufweisen.

Eine dieser Mutanten (Glu182Asp) wurde als Marker zur Selektion embryonaler Stammzellen der Ratte verwendet, wobei das Neomycin-Phosphotransferase-Gen über gezielte homologe Rekombination *(gene targeting)* in das c-myc Gen integriert wurde (Hanson et al., 1995). Die Autoren beschränken sich auf die Verwendung des modifizierten Enzyms für Gentargeting.

In der Patentanmeldung WO 99/53046 wird die Expression eines modifizierten Neomycin-Phosphotransferase-Gens (Asp261Asn) in produktionsrelevanten Säugerzellen beschrieben. Die Autoren beschreiben hierbei eine Nicht-Klonierungsmethode zur Expression eines Gens von Interesse in Säugerzellen. Durch Co-Transfektion der Zellen mit drei individuellen DNA-Fragmenten, die für ein Promotorelement, ein Gen von Interesse und ein mit einem IRES ("internal ribosomal entry site")-Element gekoppelten Selektionsmarker kodieren, lassen sich unter Selektionsdruck gezielt Zellen heranziehen, in denen alle drei DNA-Fragmente als funktionelle bicistronische Transkriptionseinheit kombiniert wurden (Promotor-Gen von Interesse-IRES-Neomycin-Phosphotransferase-Gen). Das Arrangement der Elemente erfolgt erst in der transfizierten Zelle, so dass lediglich wenige Zellen eine richtige Anordnung der Elemente zeigen. Ferner lassen sich nach Genamplifikation unter Verwendung eines amplifizierbaren selektierbaren Markers keine Hochproduzentenklone generieren. Die generierten Zellen zeigten nach mehrfacher Selektion und Genamplifikation maximal 6 pg Protein pro Zelle und Tag (6 pg/Zelle/Tag).

Keine der Publikationen offenbart modifzierte Neomycin-Phosphotransferase-Gene mit besonderer Eignung für die Bereitstellung eines Hochexpressionsvektorsystem für Säugerzellen, welches die Entwicklung von hochproduzierenden Zellen zur Herstellung rekombinanter biopharmazeutischer Proteine ermöglicht, die eine oder mehrere komplette funktionelle Transkriptionseinheiten sowohl für ein oder mehrere Gene von Interesse als auch für ein modifiziertes Neomycin-Phosphotransferase-Gen mit verminderter Antibiotikaresistenz enthalten. Das in WO 99/53046 beschrieben DNA-Konstrukt enthält lediglich ein promotorloses Neomycin-Gen funktionell verknüpft mit dem Gen für die Dihydrofolat-Reduktase (DHFR).

Es besteht somit ein Bedarf geeignete modifizierte Neomycin-Phosphotransferase-Gene, insbesondere für die Entwicklung entsprechender Hochexpressionsvektorsysteme für biopharmazeutische Prozesse, zur Verfügung zu stellen. Aufgabe der vorliegenden Erfindung war von daher, entsprechende neue modifizierte Neomycin-Phosphotransferase-Gene, Expressionsvektoren, die ein modifiziertes Neomycin-Phosphotransferase-Gen und ein Gen von Interesse in funktioneller Verknüpfung mit einem heterologen Promotor enthalten, ein Selektionsverfahren für hochproduzierende rekombinante Zellen, vorzugsweise für Säugerzellen und ein Verfahren zur Herstellung von heterologen Genprodukten zur Verfügung zustellen.

Überraschenderweise konnten im Rahmen der vorliegenden Erfindung neue modifizierte hochselektive Neomycin-Phosphotransferase-Gene erzeugt und identifiziert werden, die sich durch ihre besondere Eignung zur Selektion von hochproduzierenden Zellen auszeichnen.

### Zusammenfassung der Erfindung

Die vorliegende Erfindung stellt neue modifizierte Neomycin-Phosphotransferase-Gene zur Verfügung. Überraschenderweise wurde gefunden, dass bei Verwendung der im weiteren beschriebenen modifizierten Neomycin-Phosphotransferase-Gene als Selektionsmarker eine Anreicherung von transfizierten Säugerzellen mit hohen Expressionsraten des co-integrierten Gens von Interesse erzielt werden konnte. Im Vergleich zur Verwendung der Wildtyp-Neomycin-Phosphotransferase als Selektionsmarker wiesen die Zellen nach Transfektion mit einer der erfindungsgemäßen neuen Neomycin-Phosphotransferase-Genen eine um Faktor 1,4 bis 14,6 erhöhte Produktivität eines Proteins (eines Antikörpers) auf.

Bei den modifizierten erfindungsgemäßen Neomycin-Phosphotransferase-Genen handelt es sich vorzugsweise um Mutanten, die an Aminosäureposition 91, 182, 198, 227, 240 oder 261 für eine andere Aminosäure als das Wildtyp-Gen kodieren. In einer bevorzugten Ausführungsform handelt es sich bei dem erfindungsgemäßen Neomycin-Phosphotransferase-Gen um die Mutante Glu182Gly, Glu182Asp, Trp91Ala, Val198Gly, Asp227Ala, Asp227Val, Asp227Gly, Asp261Asn, Asp261Gly oder Phe240lle. Als besonders geeignet zur Selektion von hochproduzierenden Säugerzellen stellte sich hierbei die Verwendung der Mutanten Trp91Ala, Asp227Val, Asp261Asn, Asp261Gly und Phe240lle heraus, wobei wiederum die Mutanten Asp227Val und Asp261Gly zu Zellklonen mit der höchsten Produktivität führten und von daher besonders bevorzugt sind.

Die Herstellung der hochproduzierenden Zellen wurde durch die Verwendung eines eukaryontischen Expressionsvektors erreicht, der ein heterologes Gen von Interesse in funktioneller Verknüpfung mit einem heterologen Promotor und ein erfindungsgemäßes modifiziertes Neomycin-Phosphotransferase-Gen enthält. Der Expressionsvektor enthält vorzugsweise weitere regulatorische Elemente, beispielsweise einen oder mehrere Enhancer in funktioneller Verknüpfung mit dem oder den Promotoren. Ferner sind Expressionsvektoren bevorzugt, die zusätzlich ein Gen für ein fluoreszierendes Protein enthalten, welches funktionell mit dem Gen von Interesse und dem heterologen Promotor verknüpft ist, vorzugsweise über eine interne Ribosomenbindungsstelle (IRES), welche eine bicistronische Expression des Gens, das für ein fluoreszierendes Protein kodiert, und des Gens, das für ein Protein/Produkt von Interesse kodiert, unter der Kontrolle des heterologen Promotors ermöglicht. Besonders geeignet sind Expressionsvektoren, bei dem das heterologe Gen von Interesse unter der Kontrolle des Ubiquitin/S27a-Promotors steht.

Die Erfindung betrifft auch Expressionsvektoren, die anstelle des Gens von Interesse eine multiple Klonierungsstelle zum Einbau eines solchen Gens aufweisen, d.h. einen Sequenzbereich mit multiplen Erkennungssequenzen für Restriktionsendonukleasen.

Ein weiterer Aspekt der Erfindung betrifft rekombinante Säugerzellen, die eines der oben beschriebenen erfindungsgemäßen modifizierten Neomycin-Phosphotransferase-Gene enthalten. Weiterhin betrifft die vorliegende Erfindung rekombinante Säugerzellen, die mit einem der erfindungsgemäßen Expressionsvektoren transfiziert wurden. Hierbei handelt es sich vorzugsweise um rekombinante Nagerzellen, wobei rekombinante Hamsterzellen wie z.B. CHO-Zellen oder BHK-Zellen besonders bevorzugt sind. In einer weiteren bevorzugten Ausführungsform sind die besagten rekombinanten Zellen zusätzlich mit dem Gen für einen amplizifierbaren Selektionsmarker transfiziert, z.B. mit dem Gen der Dihydrofolat-Reduktase (DHFR).

Ferner betrifft die Erfindung ein Verfahren zur Anreicherung von rekombinanten Säugerzellen, die ein modifiziertes Neomycin-Phosphotransferase-Gens exprimieren, dadurch gekennzeichnet, dass (i) ein Pool an Säugerzellen mit einem Gen für eine modifizierte Neomycin-Phosphotransferase transfiziert wird, die im Vergleich zur Wildtyp-Neomycin-Phosphotransferase lediglich 1 bis 80%, vorzugsweise lediglich 1 bis 60%, weiter bevorzugt lediglich 1,5 bis 30%, noch weiter bevorzugt lediglich 1,5 bis 26% der Aktivität aufweist und/oder eine der oben beschriebenen Modifikationen aufweist; (ii) die Säugerzellen unter Bedingungen kultiviert werden, die eine Expression des modifizierten Neomycin-Phosphotransferase-Gens erlauben; und (iii) die Säugerzellen in Anwesenheit zumindest eines Selektionsmittels kultiviert werden, das selektiv auf das Wachstum von Säugerzellen wirkt, und solche Zellen im Wachstum bevorzugt, die das Neomycin-Phosphotransferase-Gen exprimieren.

Ebenfalls erfindungsgemäß ist ein Verfahren zur Expression von mindestens einem Gen von Interesse in rekombinanten Säugerzellen, dadurch gekennzeichnet, dass (i) ein Pool von Säugerzellen mit mindestens einem Gen von Interesse und einem Gen für eine modifizierte Neomycin-Phosphotransferase transfiziert wird, die im Vergleich zur Wildtyp-Neomycin-Phosphotransferase lediglich 1 bis 80%, vorzugsweise lediglich 1 bis 60%, weiter bevorzugt lediglich 1,5 bis 30%, noch weiter bevorzugt lediglich 1,5 bis 26% der Aktivität aufweist und/oder eine der oben beschriebenen Modifikationen aufweist; (ii) die Zellen unter Bedingungen kultiviert werden, die eine Expression des (der) Gens (Gene) von Interesse und des modifizierten Neomycin-Phosphotransferase-Gens erlauben; (iii) die Säugerzellen in Anwesenheit zumindest eines Selektionsmittels kultiviert werden, das selektiv auf das Wachstum von Säugerzellen wirkt, und solche Zellen im Wachstum bevorzugt, die das Neomycin-Phosphotransferase-Gen exprimieren; und (iv) das (die) Protein(e) von Interesse aus den Säugerzellen oder dem Kulturüberstand gewonnen wird (werden).

Im Weiteren betrifft die vorliegende Erfindung ein Verfahren zur Gewinnung und Selektion von rekombinanten Säugerzellen, die mindestens ein heterologes Gen von Interesse exprimieren, das dadurch gekennzeichnet ist, dass (i) rekombinante Säugerzellen mit einem erfindungsgemäßen Expressionsvektor transfiziert werden, der neben dem Gen von Interesse und dem modifizierten Neomycin-Phosphotransferase-Gen für ein fluoreszierendes Protein kodiert; (ii) die Säugerzellen unter Bedingungen kultiviert werden, die eine Expression des (der) Gens (e) von Interesse, des Gens, das für ein fluoreszierendes Protein kodiert, und des modifizierten Neomycin-Phosphotransferase-Gens ermöglicht; (iii) die Säugerzellen in Anwesenheit zumindest eines Selektionsmittels kultiviert werden, das selektiv auf das Wachstum von Säugerzellen wirkt, und solche Zellen im Wachstum bevorzugt, die das Neomycin-Phosphotransferase-Gen exprimieren; und (iv) die Säugerzellen mittels flowzytometrischer Analyse sortiert werden.

Sofern die Säugerzellen zusätzlich mit einem Gen für ein amplifizierbares Selektionsmarkergen, z.B. dem DHFR-Gen transfiziert wurden, ist es möglich, die Säugerzellen unter Bedingungen zu kultivieren, unter denen auch das amplifizierbare Selektionsmarkergen exprimiert wird, und dem Kulturmedium ein Selektionsmittel zuzugeben, das die Amplifikation des amplifizierbaren Selektionsmarkergens zur Folge hat.

Vorzugsweise werden die erfindungsgemäßen Verfahren mit Säugerzellen durchgeführt, die an das Wachstum in Suspension adaptiert sind, also mit Säugerzellen, die in Suspensionskultur kultiviert werden. Weitere Ausführungsformen betreffen Verfahren, bei denen die Säugerzellen, bevorzugt solche, die an das Wachstum in Suspension adaptiert sind, unter serumfreien Bedingungen kultiviert werden.

### Beschreibung der Abbildungen

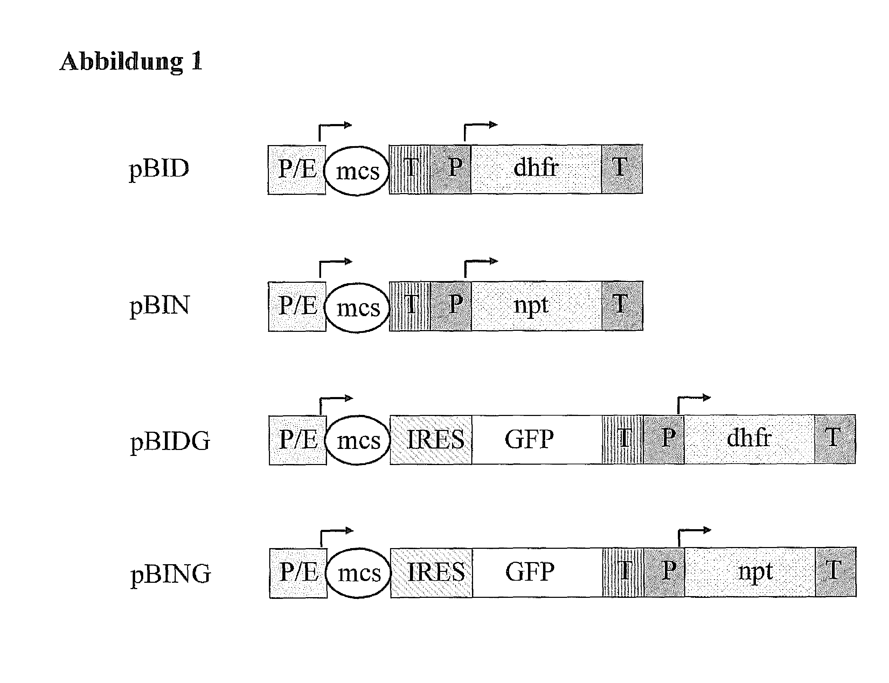

**Abbildung 1** zeigt eine schematische Darstellung der Basisvektoren, die zur Expression der rekombinanten Proteine in CHO-DG44 Zellen verwendet wurden. Bei "P/E" handelt es sich um eine Kombination aus CMV-Enhancer und Hamster-Ubiquitin/S27a-Promotor, bei "P" lediglich um ein Promotorelement und bei "T" um ein Terminationssignal für die Transkription, das zur Polyadenylierung der transkribierten mRNA benötigt wird. Die Position und Richtung der Transkriptionsinitiation innerhalb jeder Transkriptionseinheit wird durch einen Pfeil angezeigt. Zur Klonierung der heterologen Gene ist nach dem Promotorelement ein Sequenzbereich mit multiplen Schnittstellen für Restriktionsendonukleasen *(multiple cloning sites -* mcs) eingefügt. Der amplifizierbare Selektionsmarker Dihydrofolat-Reduktase ist mit "dhfr" und der Selektionsmarker Neomycin-Phosphotransferase mit "npt" (npt-Wildtyp oder npt-Mutante) abgekürzt. Das aus dem Encephalomyocarditis- Virus stammende "IRES"-Element dient als interne Ribosomenbindungsstelle innerhalb der bicistronischen Transkriptionseinheit und ermöglicht die Translation des nachfolgenden grünen fluoreszierenden Proteins "GFP".

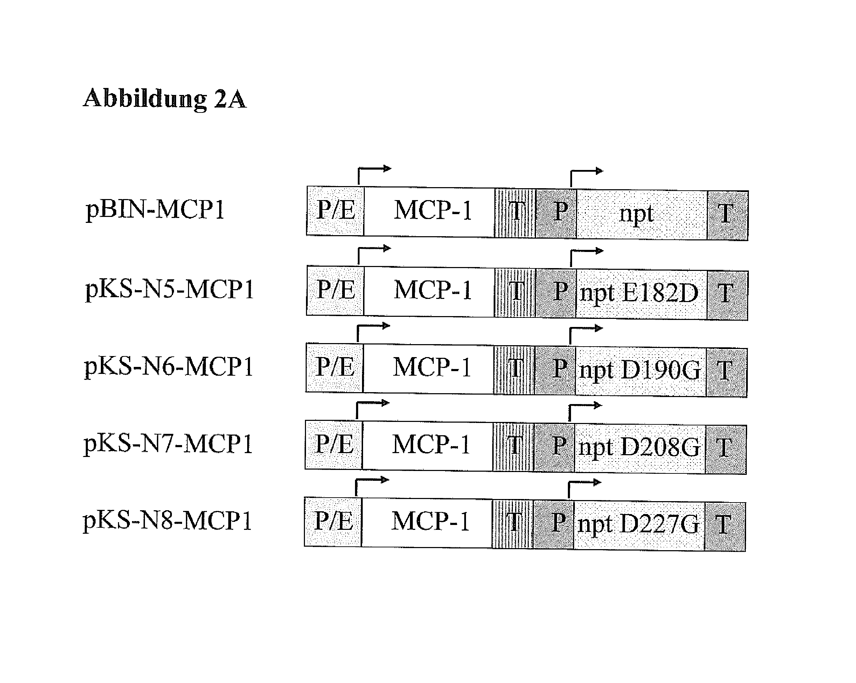

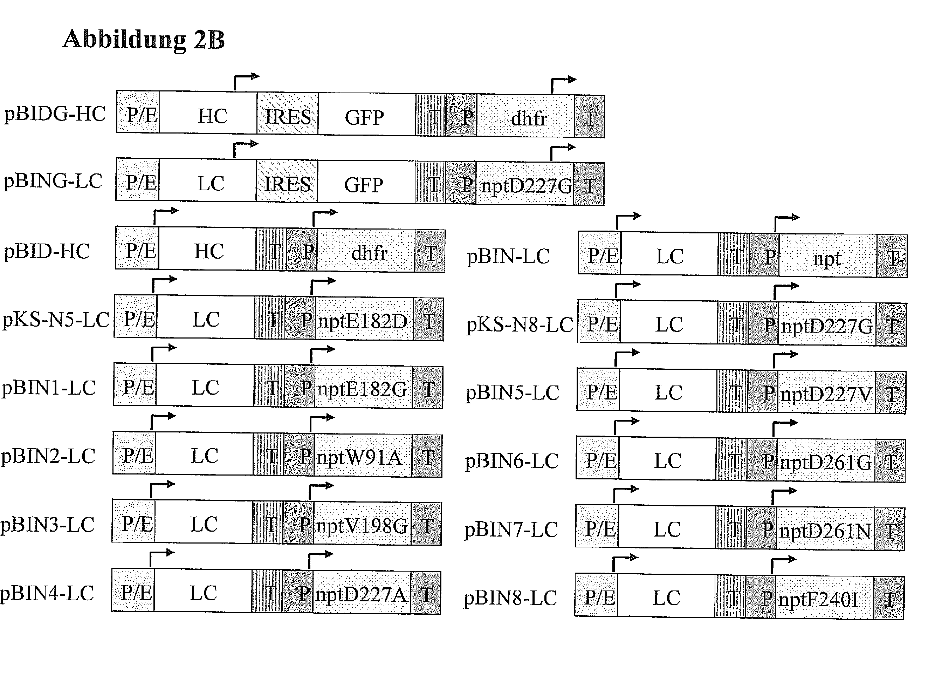

**Abbildung 2** zeigt eine schematische Darstellung der eukaryontischen Expressionsvektoren, die für ein einzelkettiges Protein (Abbidung 2A) oder jeweils für eine Untereinheit eines monoklonalen Antikörpers kodieren (Abbildung 2B) und zur Transfektion von CHO-DG44 Zellen eingesetzt wurden. Bei "P/E" handelt es sich um eine Kombination aus CMV-Enhancer und Hamster-Ubiquitin/S27a-Promotor, bei "P" lediglich um ein Promotorelement und bei "T" um ein Terminationssignal für die Transkription, das zur Polyadenylierung der transkribierten mRNA benötigt wird. Die Position und Richtung der Transkriptionsinitiation innerhalb jeder Transkriptionseinheit wird durch einen Pfeil angezeigt. Der amplifizierbare Selektionsmarker Dihydrofolat-Reduktase ist mit "dhfr" und der Selektionsmarker Neomycin-Phosphotransferase mit "npt" abgekürzt. Die NPT-Mutanten E182G (SEQ ID NO:3), E182D (SEQ ID NO:19), W91A (SEQ ID NO:5), D190G (SEQ ID NO:23), V198G (SEQ ID NO:7), D208G (SEQ ID NO:25), D227A (SEQ ID NO:9), D227V (SEQ ID NO:11), D227G (SEQ ID NO:21), D261G (SEQ ID NO:13), D261N (SEQ ID NO:15) und F2401 (SEQ ID NO:17) enthalten dabei eine Punktmutation, die an der indizierten Position in einer veränderten Aminosäure (in 1-Buchstaben-Code angegeben) resultiert. Das aus dem Encephalomyocarditis-Virus stammende "IRES"-Element dient als interne Ribosomenbindungsstelle innerhalb der bicistronischen Transkriptionseinheit und ermöglicht die Translation des nachfolgenden grünen fluoreszenten Proteins "GFP". "MCP-1" kodiert für das Monocyte Chemoattractant Protein-1, wohingegen "HC" und "LC" für die schwere bzw. leichte Kette eines humanisierten monoklonalen lgG2 Antikörpers kodieren.

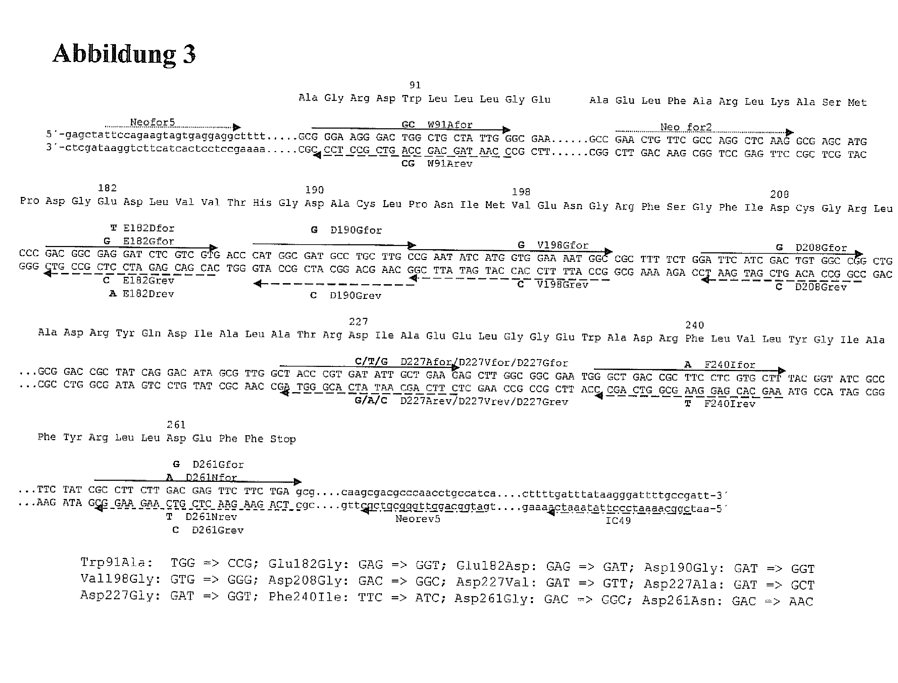

**Abbildung 3** zeigt den Sequenzausschnitt des Neomycin-Phosphotransferase (npt)-Gens, in dem durch PCR mit mutagenen Primern die Punktmutationen eingeführt wurden. Großbuchstaben kennzeichnen hierbei die Nukleotidsequenz der NPT-Kodierregion, kleine Buchstaben hingegen die flankierenden nicht-kodierenden Nukleotidsequenzen. Die aus der Nukleotidsequenz vorhergesagte Aminosäuresequenz (3-Buchstaben-Code) ist oberhalb der kodierenden Nukleotidsequenz angegeben. Pfeile geben die Richtung, Länge und Position der verwendeten Primer an, wobei Pfeile mit geschlossener Linie die mutagenen forward Primer, mit gebrochener Linie die mutagenen reverse Primer, mit gepunkteter Linie die stromaufwärts vom npt-Gen bzw. dem Mutationsort liegenden Primer Neofor5 (SEQ lD NO:27) bzw. Neofor2 (SEQ lD NO:29) und mit Strichpunkt-Linie die stromabwärts vom npt-Gen bzw. dem Mutationsort liegenden Primer Neorev5 (SEQ lD NO:28) bzw. lC49 (SEQ lD NO:30) anzeigen. Die gegenüber der Wildtyp-Sequenz ausgetauschten Nukleotide sind jeweils ober- bzw. unterhalb der Pfeile hervorgehoben.

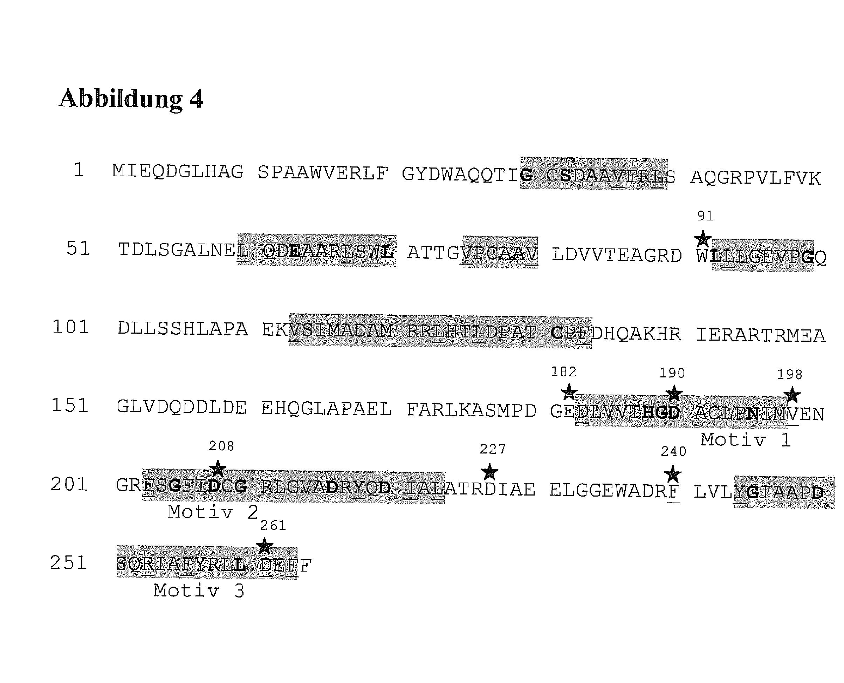

In **Abbildung 4** sind konservierte Domänen und die Position der eingeführten NPT-Mutationen innerhalb der NPT-Aminosäuresequenz dargestellt. Auf Basis von Sequenzhomologien zwischen verschiedenen Aminoglykosid-modifizierenden Enzymen wurden verschiedene konservierte Domänen innerhalb der NPT-Proteinsequenz identifiziert (grau unterlegt). Die drei Motive im C-terminalen Bereich des Enzyms haben dabei offensichtlich spezielle Funktionen. Motiv 1 und 2 sind vermutlich am katalytischen Transfer des terminalen Phosphats bei der ATP-Katalyse bzw. der Nukleotidbindung beteiligt, wohingegen Motiv 3 eine Funktion bei der ATP-Hydrolyse und/oder der Konformationsänderung im Enzym-Aminoglykosid-Komplex zugeschrieben wird. Aminosäuren, die in mindestens 70% der Aminoglykosid-modifizierenden Enzymen vorkommen, sind durch Fettdruck hervorgehoben. Die einfach unterstrichenen Aminosäuren werden auf Basis der Ähnlichkeit der gleichen Gruppe zugeordnet und kommen in mindestens 70% der Aminoglykosid-modifizierenden Enzymen vor. Mit einem Stern markierte Aminosäuren geben die Position der Mutationsorte wieder.

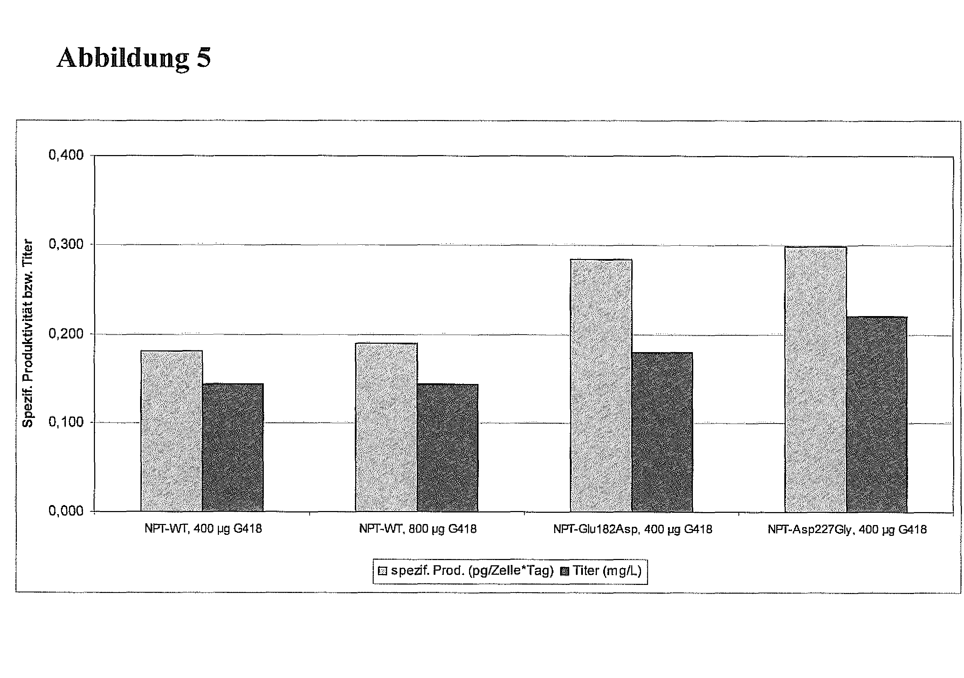

**Abbildung 5** zeigt den Einfluss der NPT-Mutationen auf die Selektion von stabil transfizierten MCP-1 exprimierenden Zellen. Dazu wurden CHO-DG44 Zellen mit den Vektoren pBIN-MCP1, pKS-N5-MCP1 bzw. pKS-N8-MCP1 transfiziert (Abb. 2A), die als Selektionsmarker entweder den NPT-Wildtyp (WT) oder die NPT-Mutanten GIu182Asp bzw. Asp227Gly enthielten. Zur Selektion von stabil transfizierten Zellen wurde dem Medium 400 µg/mL oder auch 800 µg/mL G418 als selektives Agens hinzugefügt. Die Konzentration des produzierten rekombinanten Proteins MCP-1 im Zellkulturüberstand wurde durch ELISA ermittelt und die spezifische Produktivität pro Zelle und Tag berechnet. Jeder Balken stellt dabei den Mittelwert der spezifischen Produktivität bzw. des Titers aus jeweils 18 Pools über vier Kultivierungspassagen in 6 Well-Schalen hinweg dar.

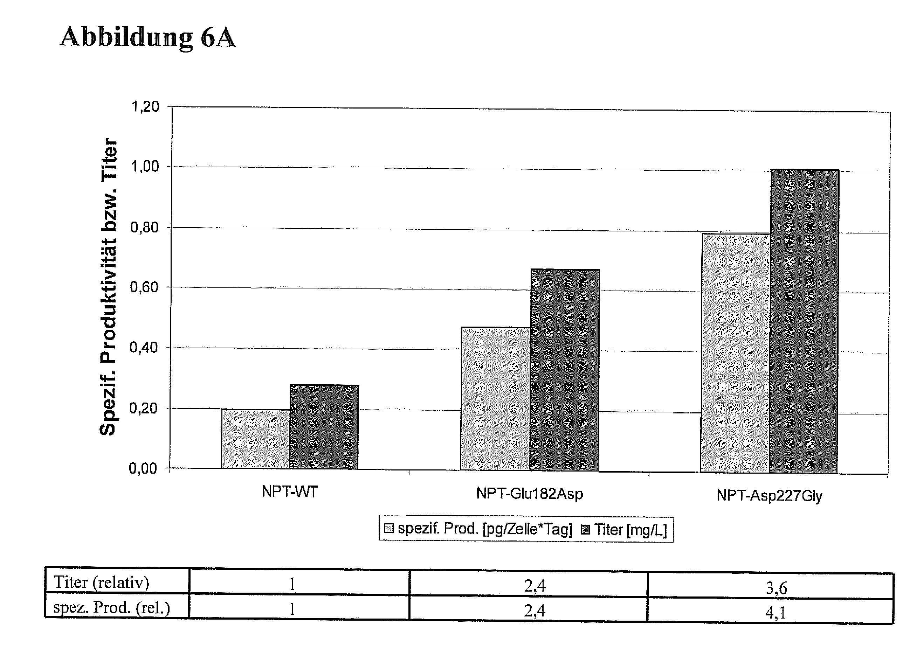

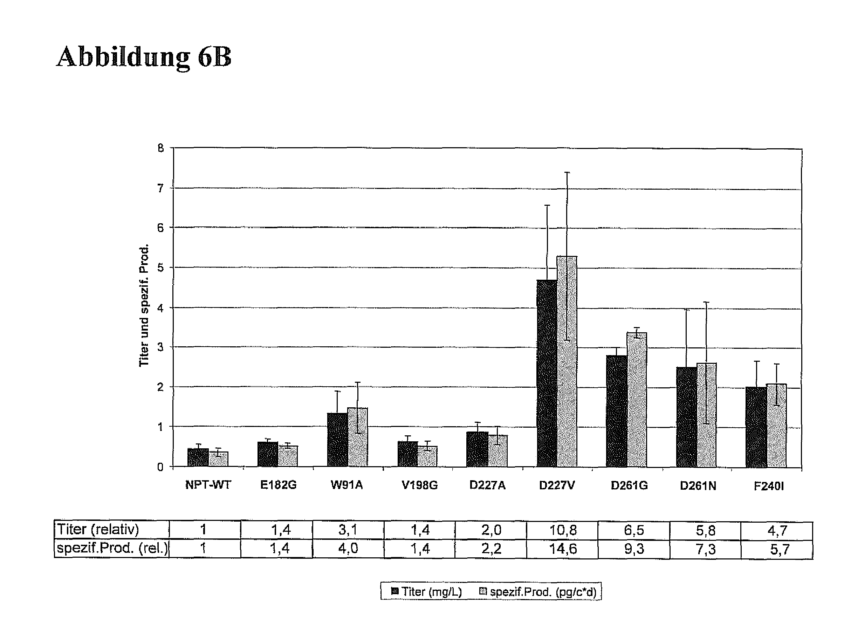

In **Abbildung 6** wurde der Einfluss der NPT-Mutationen auf die Selektion von stabil transfizierten mAk exprimierenden Zellen untersucht. Dazu wurden CHO-DG44 Zellen mit den Plasmidkombinationen pBIDG-HC/pBIN-LC (NPT-Wildtyp), pBIDG-HC/pKS-N5-LC (NPT-Mutante Glu182Asp) oder pBIDG-HC/pKS-N8-LC (NPT-Mutante Asp227Gly) (Abb. 6A) bzw. mit den Kombinationen pBIDG-HC/pBIN-LC (NPT-Wildtyp), pBIDG-HC/pBIN1-LC (NPT-Mutante Glu182Gly), pBIDG-HC/pBIN2-LC (NPT-Mutante Trp91Ala), pBIDG-HC/pBIN3-LC (NPT-Mutante Val198G), pBIDG-HC/pBlN4-LC (NPT-Mutante Asp227Ala), pBIDG-HC/pBIN5-LC (NPT-Mutante Asp227Val), pBIDG-HC/pBIN6-LC (NPT-Mutante Asp261Gly), pBIDG-HC/pBIN7-LC (NPT-Mutante Asp261Asn) oder pBIDG-HC/pBIN8-LC (NPT-Mutante Phe240IIe) transfiziert (Abb. 6B), die sich lediglich in dem als Selektionsmarker verwendeten NPT-Gen (Wildtyp oder Mutante) voneinander unterschieden. Die Konzentration des produzierten rekombinanten monoklonalen lgG2 Antikörpers im Zellkulturüberstand wurde durch ELISA ermittelt und die spezifische Produktivität pro Zelle und Tag berechnet. Insgesamt wurden für jede Vektorkombination 5 bis 9 Pools angesetzt. Die Balken repräsentieren die Mittelwerte der spezifischen Produktivität bzw. des Titers aller Pools im Test aus 6 Kultivierungspassagen in 75cm² Flaschen. Zur Berechnung der relativen Titer bzw. der relativen spezifische Produktivitäten wurden die Mittelwerte der mit dem NPT-Wildtypgen selektionierten Pools als 1 gesetzt.

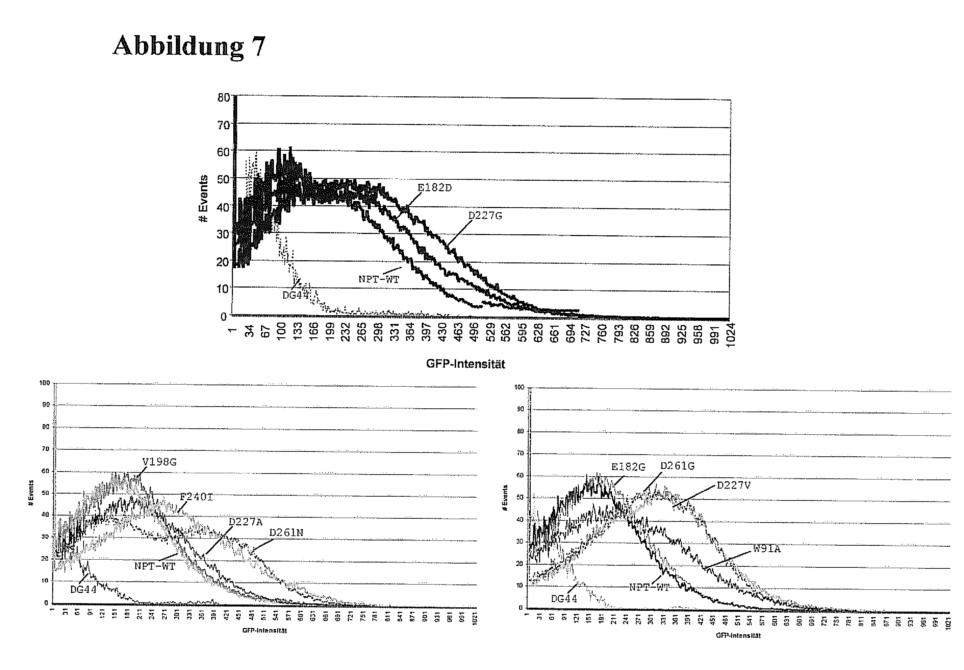

**Abbildung 7** zeigt die Anreicherung von Zellen mit höherer GFP-Expression in transfizierten Zellpools durch Verwendung der erfindungsgemäßen NPT-Mutanten als Selektionsmarker. Dazu wurden CHO-DG44 Zellen mit den Plasmidkombinationen pBIDG-HC/pBIN-LC (NPT-Wildtyp), pBIDG-HC/pKS-N5-LC (NPT-Mutante Glu182Asp), pBIDG-HC/pKS-N8-LC (NPT-Mutante Asp227Gly), pBIDG-HC/pBIN1-LC (NPT-Mutante Glu182Gly), pBIDG-HC/pBIN2-LC (NPT-Mutante Trp91Ala), pBIDG-HC/pBIN3-LC (NPT-Mutante Val198G), pBIDG-HC/pBIN4-LC (NPT-Mutante Asp227Ala), pBIDG-HC/pBIN5-LC (NPT-Mutante Asp227Val), pBIDG-HC/pBIN6-LC (NPT-Mutante Asp261Gly), pBIDG-HC/pBIN7-LC (NPT-Mutante Asp261Asn) oder pBIDG-HC/pBIN8-LC (NPT-Mutante Phe240Ile) transfiziert (jeweils 5 bis 9 Pools), die sich lediglich in dem als Selektionsmarker verwendeten NPT-Gen (Wildtyp oder Mutante) voneinander unterschieden. Außerdem enthielten die pBIDG-Vektoren auch noch das GFP als Markergen. Nach einer zwei- bis dreiwöchigen Selektion der transfizierten Zellpools in HT-freiem Medium mit Zusatz von G418 wurde die GFP-Fluoreszenz per FACS-Analyse gemessen. Jeder Graph, mit Ausnahme der als Negativkontrolle dienenden nicht-transfizierten CHO-DG44 Zellen, stellt dabei den Mittelwert der GFP-Fluoreszenz aus den Pools, die mit der gleichen Plasmidkombination transfiziert worden waren, dar.

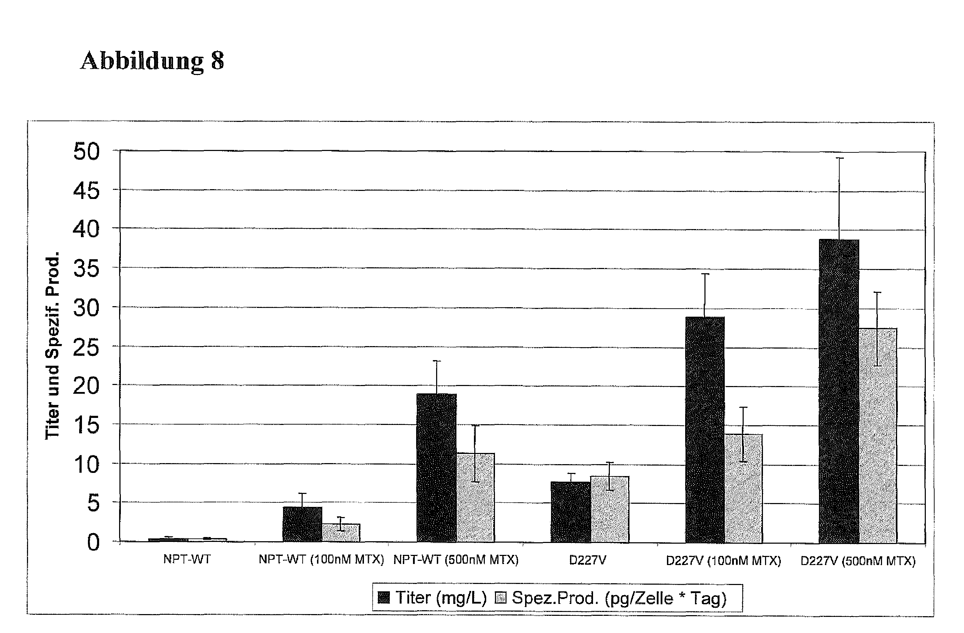

In **Abbildung 8** ist die Steigerung der mAk-Produktivität durch dhfr-vermittelte Genamplifikation am Beispiel je eines Zellpools dargestellt, der aus der Transfektion von CHO-DG44 mit der Vektorkombination pBIDG-HC/pBIN-LC (NPT-Wildtyp) bzw. pBIDG-HC/pBIN5-LC (NPT-Mutante D227V) erhalten wurde. Nach der ersten Selektion in Hypoxanthin/Thymidin-freiem CHO-S-SFMII-Medium in Anwesenheit von G418 wurde eine dhfr-vermittelte Genamplifikation durch Zusatz von 100 nM und nachfolgend von 500 nM MTX zum Kultivierungsmedium durchgeführt. Die Konzentration des mAks im Zellkulturüberstand der Pools wurde durch ELISA ermittelt und die spezifische Produktivität pro Zelle und Tag (pg/Zelle*Tag) berechnet. Jeder Datenpunkt stellt dabei den Durchschnitt aus sechs Kultivierungspassagen in 75cm² Flaschen dar.

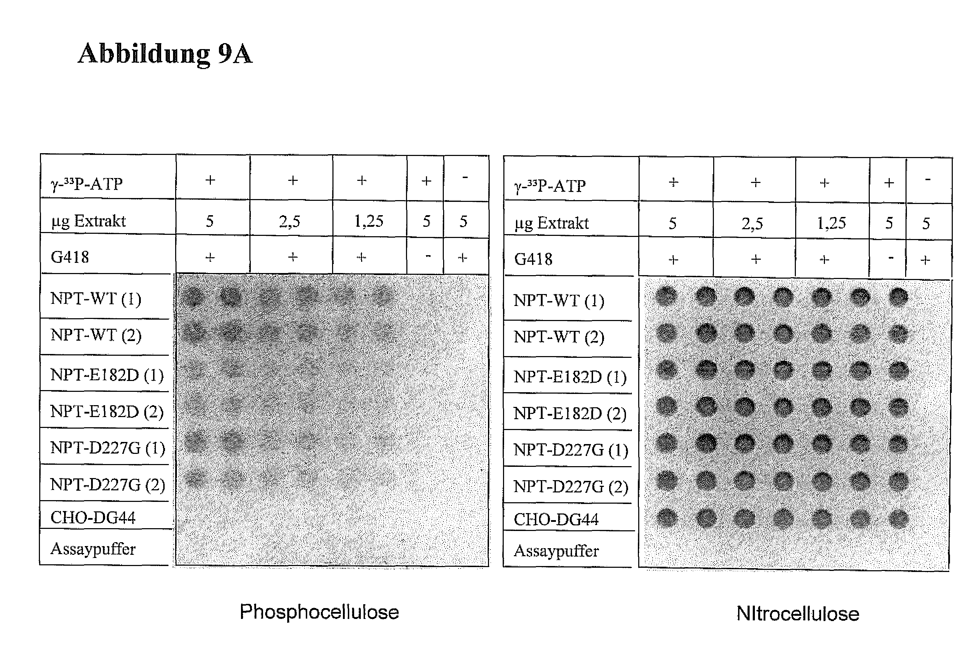

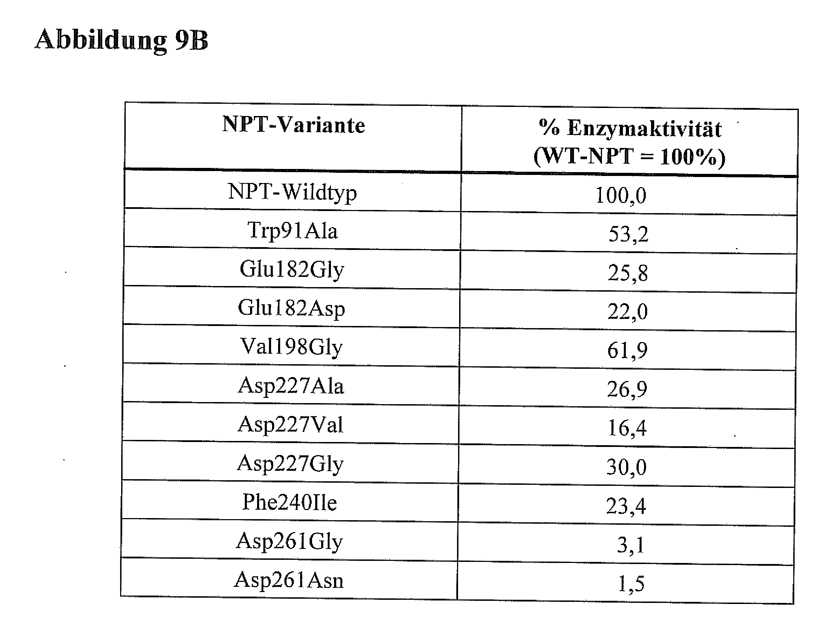

**Abbildung 9** zeigt die Enzymaktivität der erfindungsgemäßen NPT-Mutanten im Vergleich zum NPT-Wildtyp in einem Dot-Assay. Hierzu wurden Zellextrakte aus jeweils zwei verschiedenen mAk exprimierenden Zellpools (Pool 1 und 2) hergestellt, die entweder mit dem NPT-Wildtypgen (SEQ ID NO:1) oder mit den NPT-Mutanten E182G (SEQ ID NO:3), E182D (SEQ ID NO:19), W91A (SEQ ID NO:5), V198G (SEQ ID NO:7), D227A (SEQ ID NO:9), D227V (SEQ ID NO:11), D227G (SEQ ID NO: 21) D261G (SEQ ID NO:13), D261N (SEQ ID NO:15) und F2401 (SEQ ID NO:17) transfiziert und selektioniert worden waren. Nicht-transfizierte CHO-DG44 Zellen dienten als Negativkontrolle. Als Substrat im Phosphorylierungsassay wurde G418 eingesetzt. Die Extrakte wurden in einem 96 Well Vakuum Manifold durch einen Sandwich aus P81 Phosphocellulose- und Nitrocellulose-Membran filtriert. Durch Proteinkinasen phosphorylierte Proteine sowie nicht-phosphorylierte Proteine binden an die Nitrocellulose, während phosphoryliertes und nicht-phosphoryliertes G418 die Nitrocellulose passiert und an die Phosphocellulose bindet (Abb. 9A). Die Detektion und Quantifizierung der radioaktiven Signale erfolgte mittels eines Phospho Imagers. Zur Berechnung der prozentualen Enzymaktivität wurden die Signale, die mit 5 µg Extrakt erhalten wurden, herangezogen. Die prozentualen Enzymaktivitäten stellen dabei den Mittelwert der NPT-Mutanten aus jeweils 2 mAk-exprimierenden Zellpools dar, wobei die Enzymaktivität der Wildtyp-NPT als 100% gesetzt wurde (Abb. 9B).

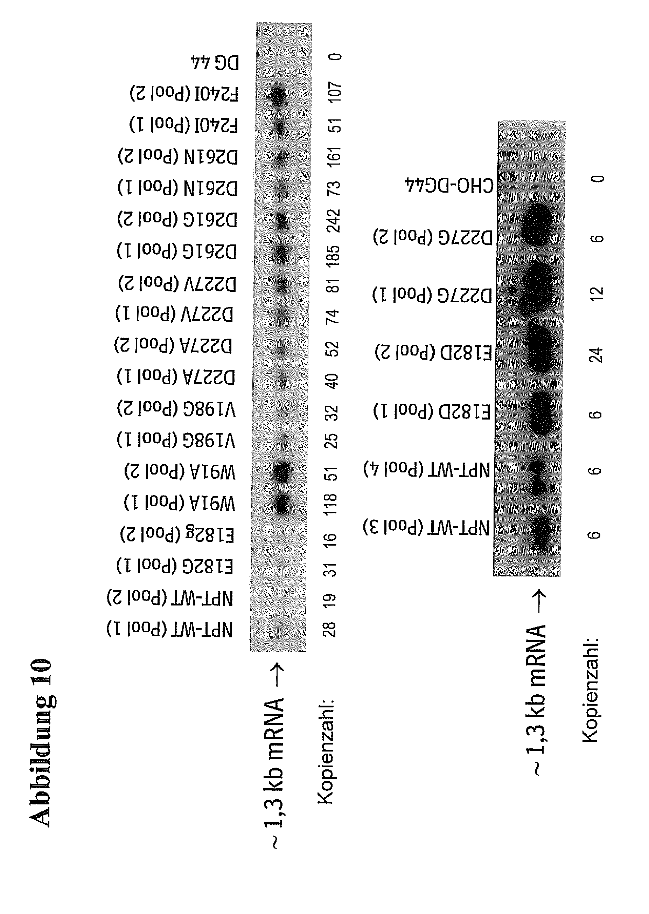

**Abbildung 10** zeigt die Northern Blot Analyse der NPT-Expression sowie die Zahl der NPT-Genkopien in den transfizierten Zellpools. Hierzu wurde Gesamt-RNA aus jeweils zwei verschiedenen mAk exprimierenden Zellpools hergestellt, die entweder mit dem NPT-Wildtypgen (SEQ ID NO:1) oder mit den NPT-Mutanten E182G (SEQ ID NO:3), E182D (SEQ ID NO: 19), W91A (SEQ ID NO:5), V198G (SEQ ID NO:7), D227A (SEQ ID NO:9), D227V (SEQ ID NO:11), D227G (SEQ ID NO: 21), D261G (SEQ ID NO:13), D261 N (SEQ ID NO:15) und F2401 (SEQ ID NO:17) transfiziert und selektioniert worden waren. Nicht-transfizierte CHO-DG44 Zellen dienten als Negativkontrolle. Es wurde jeweils 30 µg RNA mit einem FITC-dUTP-markierten PCR-Produkt, das die Kodierregion des NPT-Gens umfasste, hybridisiert. In allen transfizierten Zellen wurde ein spezifisches singuläres NPT-Transkript von ca. 1,3 kb detektiert. Zur Bestimmung der npt-Genkopienzahl wurde in einer Dot Blot Analyse genomische DNA aus den oben erwähnten mAk exprimierenden Zellpools isoliert. Es wurden jeweils 10 µg, 5 µg, 2,5 µg, 1,25 µg, 0,63 µg bzw. 0,32 µg genomische DNA mit einem FITC-dUTP-markierten PCR-Produkt, das die Kodierregion des NPT-Gens umfasste, hybridisiert. Nicht-transfizierte CHO-DG44 Zellen dienten als Negativkontrolle. Als Standard wurde das Plasmid pBIN-LC eingesetzt (320 pg, 160 pg, 80 pg, 40 pg, 20 pg, 10 pg, 5 pg, 2,5 pg). Die Kopienzahl der npt-Gene in den jeweiligen Zellpools wurde mit Hilfe der Standardreihe, die aus den ermittelten Signalintensitäten der titrierten Plasmid-DNA erstellt wurde, berechnet.

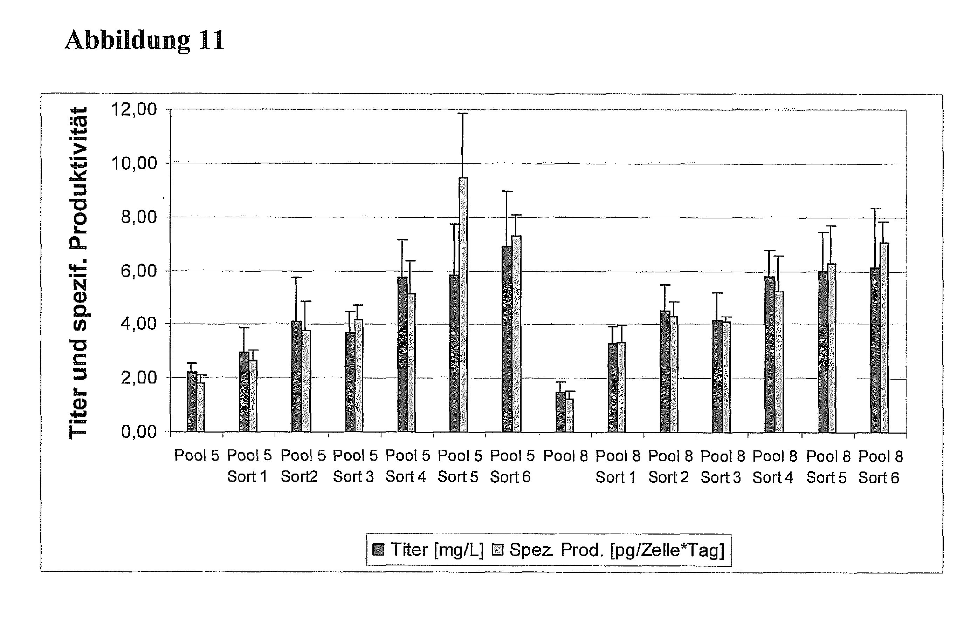

**Abbildung 11** zeigt die Isolierung von hochexprimierenden mAk Zellpools durch eine GFP-basierte Selektion mittels FACS am Beispiel von zwei Zellpools (Zellpool 5 und 8). Diese Zellpools, aus der Co-Transfektion mit den Vektoren pBID-HC und pBING-LC erhalten, wurden einem sequentiellen GFP-basiertem FACS-Sorting unterzogen. Die Konzentration des lgG2 Antikörpers im Zellkulturüberstand der Pools wurde nach jedem Sortierungsschritt durch ELISA ermittelt und die spezifische Produktivität pro Zelle und Tag (pg/Zelle*Tag) berechnet. Insgesamt wurden 6 Sortierungen durchgeführt, wobei jeweils die 5% Zellen mit der höchsten GFP-Fluoreszenz heraussortiert wurden. Jeder Datenpunkt stellt dabei den Durchschnitt aus mindestens sechs Kultivierungspassagen in 75 cm² Flaschen dar.

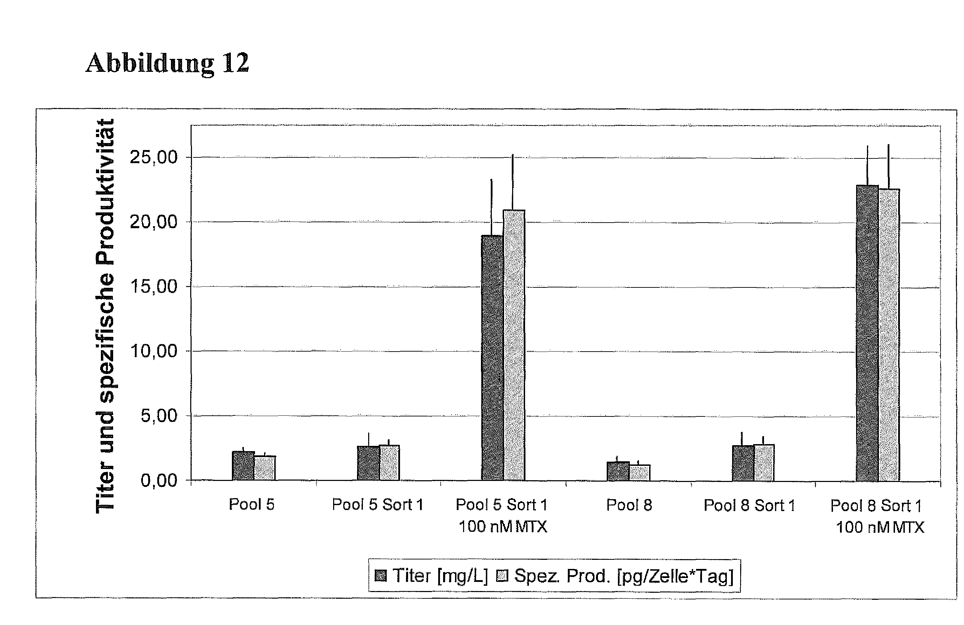

**Abbildung 12** zeigt die durch Kombination einer GFP-basierten Selektion mit einem MTX-Amplifikationsschritt erzielten Steigerungen in der mAk-Produktivität am Beispiel der Zellpools 5 und 8 (vgl. Abb. 11). Zwei Wochen nach der Co-Transfektion von CHO-DG44 mit den Vektoren pBID-HC und pBING-LC wurden aus den Pools 5 und 8 die 5% Zellen mit der höchsten GFP-Fluoreszenz heraussortiert. Danach wurde eine dhfr-vermittelte Genamplifikation durch Zusatz von 100 nM Methotrexat (MTX) zum Kultivierungsmedium durchgeführt. Die Konzentration des mAks im Zellkulturüberstand der Pools wurde durch ELISA ermittelt und die spezifische Produktivität pro Zelle und Tag (pg/Zelle*Tag) berechnet. Jeder Datenpunkt stellt dabei den Durchschnitt aus mindestens sechs Kultivierungspassagen in 75 cm² Flaschen dar.

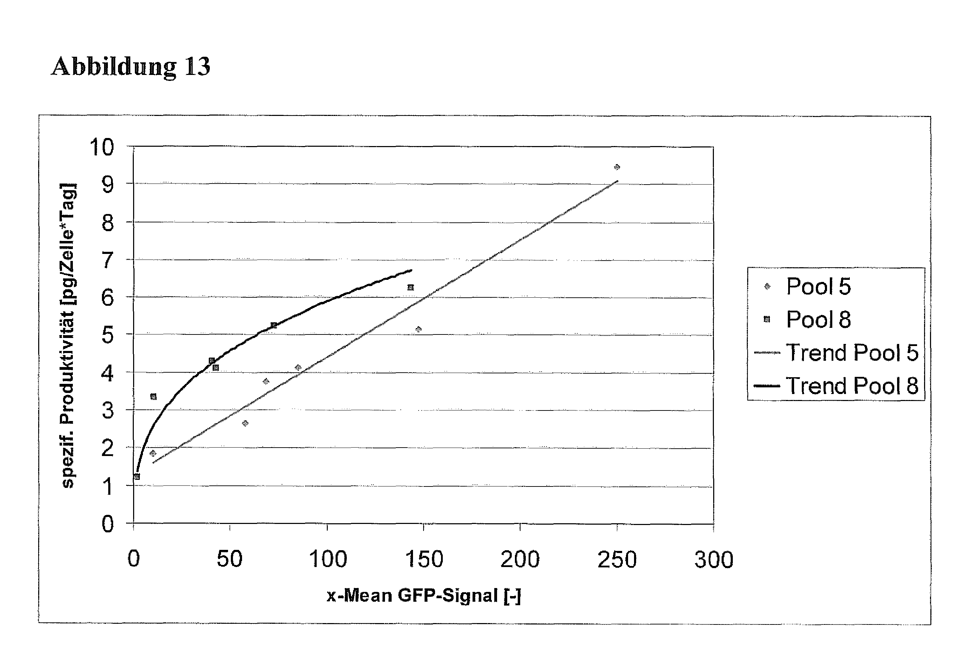

**Abbildung 13** zeigt die Korrelation zwischen der Antikörper-Produktivität und der GFP-Fluoreszenz am Beispiel der Zellpools 5 und 8 (vgl. Abb. 11). Diese Zellpools wurden aus der Transfektion von CHO-DG44 mit der Vektorkombination pBID-HC und pBING-LC erhalten. Sie wurden einem sequentiellen GFP-basiertem FACS-Sorting unterzogen, wobei jeweils die 5% Zellen mit der höchsten GFP-Fluoreszenz heraussortiert wurden. Nach jedem Sortierungsschritt wurde die Konzentration des lgG2 Antikörpers im Zellkulturüberstand des Pools durch ELISA ermittelt und die spezifische Produktivität pro Zelle und Tag (pg/c*d) berechnet. Jeder Datenpunkt stellt dabei den Durchschnitt aus mindestens sechs Kultivierungspassagen in 75 cm² Flaschen dar.

### Detaillierte Beschreibung der Erfindung und bevorzugte Ausführungsformen

Die nachfolgenden Angaben zu den Aminosäurepositionen beziehen sich jeweils auf die Position der Aminosäure, wie sie vom Wildtyp-Neomycin-Phosphotrasferase-Gen mit der SEQ ID NO:1 kodiert wird. Mit einem "modifizierten Neomycin-Phosphotransferase-Gen" ist eine Nukleinsäure gemeint, die für ein Polypeptid mit Neomycin-Phosphotransferase-Aktivität kodiert, wobei das Polypeptid wenigstens an einer der in der Beschreibung näher angegebenen Aminosäurepositionen, die zu dem Willdtyp-Protein mit der SEQ ID NO:2 homolog sind, eine andere Aminosäure als das Wildtyp-Protein aufweist. In diesem Zusammenhang meint der Ausdruck "homolog", dass der die Mutation tragende Sequenzbereich mit Hilfe sog. Standard "alignment" Algorithmen, wie beispielsweise "BLAST" (Altschul, S.F., Gish, W., Miller, W., Myers, E.W. & Lipman, D.J. (1990) "Basic local alignment search tool." J. Mol. Biol. 215:403-410; Gish, W. & States, D.J. (1993) "Identification of protein coding regions by database similarity search." Nature Genet. 3:266-272; Madden, T.L., Tatusov, R.L. & Zhang, J. (1996) "Applications of network BLAST server" Meth. Enzymol. 266:131-141; Zhang, J. & Madden, T.L. (1997) "PowerBLAST: A new network BLAST application for interactive or automated sequence analysis and annotation." Genome Res. 7:649-656; Altschul, Stephen F., Thomas L. Madden, Alejandro A. Schäffer, Jinghui Zhang, Zheng Zhang, Webb Miller, and David J. Lipman (1997), "Gapped BLAST and PSI-BLAST: a new generation of protein database search programs", Nucleic Acids Res. 25:3389-3402) mit einer Referenzsequenz, vorliegend mit der Sequenz der Wildtyp-Neomycin-Phosphotransferase nach SEQ ID NO:2 in Übereinstimmung gebracht werden kann. Sequenzen sind dann in Übereinstimmung gebracht, wenn sie sich in ihrer Sequenzabfolge entsprechen und mit Hilfe der Standard "alignment" Algorithmen identifizieren lassen.

Die vorliegende Erfindung stellt neue modifizierte Neomycin-Phosphotransferase-Gene sowie Verfahren zur Herstellung und Selektion von Säugerzelllinien zur Verfügung, die eine hohe Expression heterologer Genprodukte, vorzugsweise biopharmazeutisch relevanter Polypeptide oder Proteine, ermöglichen. Die erfindungsgemäßen Verfahren beruhen in erster Linie auf der Selektion von Zellen, die neben dem Gen von Interesse ein erfindungsgemäßes Neomycin-Phosphotransferase-Gen exprimieren, das den transfizierten Zellen gegenüber nicht transfizierten Zellen einen Selektionsvorteil vermittelt. Überraschenderweise hat sich gezeigt, dass die Verwendung der hier beschriebenen erfindungsgemäßen modifizierten Neomycin-Phosphotransferase-Gene (mNPT-Gene) gegenüber dem Wildtyp-Neomycin-Phosphotransferase-Gen (wtNPT-Gen) einen erheblichen selektiven Vorteil aufweist. Dies betrifft insbesondere die Verwendung von Mutanten, die eine geringere Enzymaktivität im Vergleich zur wtNPT aufweisen.

### Erfindungsgemäße modifizierte Neomycin-Phosphotransferase-Gene

Als besonders geeignet hat sich die Verwendung von modifizierten NPT-Genen herausgestellt, die für eine NPT kodieren, die lediglich 1 bis 80%, vorzugsweise 1 bis 60 % der Enzymaktivität der wtNPT aufweist. Weiter bevorzugt sind hierbei NPT-Mutanten, die lediglich 1 % bis 30% der Enzymaktivität der wtNPT aufweisen, besonders bevorzugt sind solche, die lediglich 1,5% bis 26% der Enzymaktivität der wtNPT aufweisen. Die Enzymaktivität einer NPT lässt sich beispielhaft in einem wie in Beispiel 4 beschriebenen und als Methode 5 angegebenen *"Dot Assay"* bestimmen.

Als "Wildtyp-Neomycin-Phosphotransferase" bezeichnet man ein Neomycin-Phosphotransferase-Gen, das für das Aminoglycosid-3'-Phosphotransferase ll Enzym (EC 2.7.1.95) kodiert, dessen Gen natürlicherweise in *Escherichia coli* Transposon 5-assoziiert ist, und z.B. die in SEQ ID NO:2 angegebene Aminosäuresequenz enthält oder durch die in SEQ ID NO:1 angegebene Nukleotidsequenz kodiert wird. Dieses Enzym vermittelt Resistenz gegenüber verschiedenen aminoglykosidischen Antibiotika wie Neomycin, Kanamycin und G418, indem es die Antibiotika durch den Transfer des terminalen Phosphats von ATP auf die 3'Hydroxylgruppe des Aminohexose-Rings I inaktiviert. Als wtNPT sind ferner alle NPT zu verstehen, die eine vergleichbare Enzymaktivät aufweisen, wie die durch SEQ ID NO:1 kodierte NPT. Dies schließt insbesondere solche NPT ein, bei denen das enzymatisch aktive Zentrum, welches den Transfer eines terminalen Phosphat von ATP auf ein Substrat katalysiert, in identischer oder annähernd identischer Konformation vorliegt (Shaw et al., 1993; Hon et al., 1997; Burk et al., 2001) und somit eine vergleichbare Enzymaktivität aufweist wie ein Enzym, welches die Aminosäuresequenz von SEQ ID NO:2 enthält. Eine wtNPT hat hierbei eine vergleichbare Enzymaktivität, wenn diese etwa 81 bis 150%, vorzugsweise 90 bis 120% der Enzymaktivität zeigt, die eine durch SEQ ID NO:2 definierte NPT aufweist, wobei die Aktivität in dem unter Beispiel 4 und als Methode 5 beschriebenen "Dot-Assay" bestimmt werden kann.

Grundsätzlich bevorzugt sind Mutanten, bei denen die Verringerung der Enzymaktivität im Vergleich zur wtNPT auf einer Modifikation der Aminosäuresequenz basiert, z.B. auf Substitution, Insertion oder Deletion zumindest einer oder mehrerer Aminosäuren. Deletions-, Insertions- und Substitutionsmutanten lassen sich mittels "ortsspezifischer Mutagenese" und / oder "PCR-basierten Mutagenese-Techniken" erzeugen. Entsprechende Methoden sind beispielhaft von Lottspeich und Zorbas (1998) (Kapitel 36.1 mit weiteren Verweisen) beschrieben.

Überraschenderweise zeigte sich, dass bei Verwendung von Neomycin-Phosphotransferase-Mutanten als Selektionsmarker, bei denen zumindest die Aminosäure Tryptophan an Aminosäureposition 91, die Aminosäure Glutaminsäure an Aminosäureposition 182, die Aminosäure Valin an Aminosäureposition 198, die Aminosäure Asparaginsäure an Aminosäureposition 227, die Aminosäure Asparaginsäure an Aminosäureposition 261 oder die Aminosäure Phenylalanin an Aminosäureposition 240 im Vergleich zur wtNPT verändert wurde, eine besonders effektive Anreicherung von transfizierten Säugerzellen mit hohem Expressionsraten des co-integrierten Gens von Interesse erzielt werden kann. Bevorzugt sind demnach Mutanten, die die Aminosäuren an Position 91, 182, 198, 227, 261 und/oder 240 betreffen. Besonders vorteilhaft sind hierbei Substitutionsmutanten, d.h. Mutanten bei denen die entsprechend im Wildtyp an dieser Stelle vorkommende Aminosäure gegen eine andere Aminosäure ausgetauscht wurde. Noch weiter bevorzugt sind entsprechende Substitutionsmutanten, bei denen eine Veränderung der entsprechenden Aminosäure zu einer Reduktion der Enzymaktivität im Vergleich zur wt-NPT auf 1 bis 80%, vorzugsweise auf 1 bis 60%, weiter bevorzugt auf 1,5 bis 30%, noch weiter bevorzugt auf 1,5% bis 26% führt. Insbesondere bevorzugt sind modifizierte NPT-Gene, bei denen die Aminosäure 91, 227, 261 und/oder 240 entsprechend modifiziert wurde, so dass die Enzymaktivität im Vergleich zur wt-NPT lediglich 1 bis 80%, vorzugsweise lediglich 1 bis 60%, weiter bevorzugt lediglich 1,5 bis 30%, noch weiter bevorzugt lediglich 1,5% bis 26% beträgt. Am meisten bevorzugt ist eine Substitutionsmutante, bei der die Aminosäure an Aminsäureposition 227 in der Form modifiziert wurde, dass die Enzymaktivität der modifizierten NPT weniger als 26%, vorzugsweise zwischen 1 und 20%, noch weiter bevorzugt zwischen 1 und 16% im Vergleich zur wt-NPT beträgt.

Nach einer weiteren Ausführungsform der vorliegenden Erfindung sind Mutanten vorteilhaft, die im Vergleich zur wtNPT an Aminosäureposition 91, 182 oder 227 jeweils für Glycin, Alanin, Valin, Leucin, Isoleucin, Phenylalanin, oder Tyrosin kodieren. Darüber hinaus kann die Glutaminsäure an Aminosäureposition 182 auch durch Asparaginsäure, Asparagin, Glutamin oder eine beliebige andere vorzugsweise negativ geladene Aminosäure ersetzt werden. Ferner sind modifizierte NPT-Gene bevorzugt, die an Aminosäureposition 198 im Vergleich zum wtNPT für Glycin, Alanin, Leucin, Isoleucin, Phenylalanin, Tyrosin oder Tryptophan kodieren. Darüber hinaus sind modifizierte NPT-Gene bevorzugt, die an Aminosäureposition 240 im Vergleich zum wtNPT für Glycin, Alanin, Valin, Isoleucin, Tyrosin oder Tryptophan kodieren. Ferner sind modifizierte NPT-Gene bevorzugt, die an Aminosäureposition 261 im Vergleich zum wtNPT für Glycin, Alanin, Leucin, Isoleucin, Phenylalanin, Tyrosin, Tryptophan, Asparagin, Glutamin oder Asparaginsäure kodieren. Insbesondere zeigte sich, dass mit den Mutanten Glu182Gly, Glu182Asp, Trp91Ala, Val198Gly, Asp227Ala, Asp227Val, Asp227Gly, Asp261Gly, Asp261Asn und Phe240lle als Selektionsmarker eine Anreicherung von transfizierten Säugerzellen mit hohen Expressionsraten des co-integrierten Gens von Interesse erzielt wurde, so dass diese Mutanten besonders bevorzugt sind. Noch weiter bevorzugt sind die Mutanten Asp227Val, Asp227Gly, Asp261Gly, Asp261Asn, Phe240lle und Trp91Ala, mit deren Verwendung die besten Anreicherungsraten erreicht werden konnten. Insbesondere bevorzugt ist die Mutante Asp227Val.

Im Gegensatz hierzu erwiesen sich die Asp190Gly- und die Asp208Gly-Mutanten als ungeeignete Marker für die Selektion von transfizierten CHO-DG44-Zellen unter serumfreien Kulturbedingungen. Bedingt durch die vermutlich sehr reduzierte Enzymfunktion dieser Mutanten (Asp190Gly, Asp208Gly) wurden nach der Selektionsphase nur wenige Zellen erhalten, die zudem in Wachstum und Vitalität stark beeinträchtigt waren.

Bei den Aminosäuren an Position 182 und 227 bezogen auf den Wildtyp handelt es sich um nicht-konservierte Aminosäuren, die außerhalb der drei konservierten Motive im C-terminalen Bereich der Aminoglykosid-3'-Phosphotransferasen lokalisiert sind. Die Aminosäure an Position 91 gehört ebenfalls zu den nicht-konservierten Aminosäuren und liegt außerhalb eines der konservierten Motive im N-terminalen Bereich der Aminoglykosid-3'-Phosphotransferasen. Bei den Aminosäuren an Position 198 und 240 handelt es sich hingegen um konservierte Aminosäuren im C-terminalen Bereich der NPT, die jedoch außerhalb der konservierten Motive liegen. Im Gegensatz hierzu ist die Aminosäure an Position 261 eine konservierte Aminosäure im dritten konservierten Motiv des C-terminalen Bereichs (Shaw et al., 1993; Hon et al., 1997; Burk et al., 2001).

Im Vergleich zur Verwendung der wtNPT als Selektionsmarker wiesen die Zellen im Fall der Glu182Gly-, Glu182Asp- und Val198Gly-Mutante eine um Faktor 1,4 - 2,4, im Fall der Asp227Gly-Mutante eine um Faktor 1,6 - 4,1, im Fall der Asp227Ala- bzw. Trp91Ala-Mutante eine um Faktor 2,2 bzw. 4, im Fall der Phe240lle- bzw. Asp261Asn-Mutante eine um Faktor 5,7 bzw. 7,3 und im Fall der Asp261Gly- bzw. Asp227Val-Mutante eine sogar um Faktor 9,3 bzw. 14,6 erhöhte Produktivität auf. Zur Expression des mehrkettigen Proteins(eines Antikörpers) wurde dabei eine Co-Transfektion durchgeführt. Die beiden Proteinketten wurden dabei jeweils von einem eigenen Vektor aus exprimiert, wobei der eine Vektor zusätzlich noch für das NPT-Gen und der andere Vektor für das amplifizierbare selektionierbare Dihydrofolat-Reduktase-Gen kodierte.

Die vorliegende Erfindung betrifft somit ein Verfahren zur Anreicherung von rekombinanten Säugerzellen, die ein modifiziertes Neomycin-Phosphotransferase-Gen exprimieren, dadurch gekennzeichnet, dass (i) ein Pool an Säugerzellen mit einem Gen für eine modifizierte Neomycin-Phosphotransferase transfiziert wird, die im Vergleich zur Wildtyp-Neomycin-Phosphotransferase lediglich 1 bis 80%, vorzugsweise lediglich 1 bis 60%, weiter bevorzugt lediglich 1,5 bis 30%, noch weiter bevorzugt 1,5 bis 26% und/oder eine der hier beschriebenen Modifikationen aufweist; (ii) die Säugerzellen unter Bedingungen kultiviert werden, die eine Expression des modifizierten Neomycin-Phosphotransferase-Gens erlauben; und (iii) die Säugerzellen in Anwesenheit zumindest eines Selektionsmittels kultiviert werden, das selektiv auf das Wachstum von Säugerzellen wirkt, und solche Zellen im Wachstum bevorzugt, die das Neomycin-Phosphotransferase-Gen exprimieren.

Besonders bevorzugt ist ein entsprechendes Verfahren, wenn ein in dieser Anmeldung näher beschriebenes modifiziertes NPT-Gen verwendet wird, insbesondere wenn das verwendete modifizierte NPT-Gen für eine modifizierte NPT kodiert, die im Vergleich zum Wildtyp-Gen an Aminosäureposition 91 für Alanin, an Aminosäureposition 182 für Glycin oder Asparaginsäure, an Aminosäureposition 198 für Glycin, an Aminosäureposition 227 für Alanin, Glycin bzw. Valin, an Aminosäureposition 261 für Glycin oder Asparagin oder an Aminosäureposition 240 für Isoleucin kodiert. Noch weiter bevorzugt sind NPT-Gene, die im Vergleich zum Wildtyp-Gen an Aminosäureposition 227 für Valin und /oder an Aminosäureposition 261 für Glycin und/oder Asparagin kodieren. Insbesondere bevorzugt sind solche NPT-Gene, die an Aminosäureposition 240 im Vergleich zum Wildtyp-Gen für ein Isoleucin oder an Aminosäureposition 227 für ein Valin kodieren, wobei ein NPT-Gen, das an Aminosäureposition 227 im Vergleich zum Wildtyp-Gen für Valin kodiert wiederum besonders bevorzugt ist. Natürlich umfasst die vorliegende Erfindung auch modifizierte NPT-Gene sowie die erfindungsgemäße Verwendung von modifizierten NPT-Genen, die eine Kombination aus den entsprechenden Aminosäureaustauschen aufweisen.

Die vorliegende Erfindung betrifft ferner eukaryontische Expressionsvektoren, die (i) ein heterologes Gen von Interesse in funktioneller Verknüpfung mit einem heterologen Promotor und (ii) ein hier beschriebenes erfindungsgemäßes modifiziertes Neomycin-Phosphotransferase-Gen enthalten, welches für eine Neomycin-Phosphotransferase kodiert, die im Vergleich zur Wildtyp-Neomycin-Phosphotransferase eine geringe Enzymaktivität aufweist. Unter einer "geringen" oder "geringeren" Enzymaktivität im Sinne der Erfindung ist eine Enzymaktivität zu verstehen, die maximal 80%, vorzugsweise 1 bis 80%, weiter bevorzugt lediglich 1 bis 60% der Enzymaktivität der wtNPT entspricht. Nach einer weiteren Ausführungsform der voliegenden Erfindung meint "geringere Enzymaktivität" eine Enzymaktivität von 1 bis 30%, vorzugsweise 1,5 bis 26% im Vergleich zur Wildtyp-Neomycin-Phosphotransferase.

Ein bevorzugter Expressionsvektor enthält ein modifiziertes NPT-Gen, das für eine modifizierte NPT kodiert, die lediglich 1 bis 80%, vorzugsweise lediglich 1 bis 60% der Enzymaktivität der wtNPT aufweist. Weiter bevorzugt sind Expressionsvektoren mit modifizierten NPT-Genen, die für Mutanten kodieren, die lediglich 1 bis 30% der Enzymaktivität der wtNPT aufweisen. Im Besonderen bevorzugt sind solche Expressionsvektoren, die ein modifiziertes NPT-Gen enthalten, die für Mutanten kodieren, die lediglich 1,5 bis 26% der Enzymaktivität der wtNPT aufweisen, wobei die Aktivität in dem unter Beispiel 4 und als Methode 5 beschriebenen "Dot-Assay" bestimmt wird.

In einer weiteren Ausführungsform der Erfindung enthalten die Expressionsvektoren Gene von modifizierten NPT, die im Vergleich zur wtNPT an Aminosäureposition Trp91, Glu182, Vall98, Asp227, Phe240 oder an Position Asp261 modifiziert wurden. Bevorzugt sind in diesem Zusammenhang NPT-Mutanten, die an Position Trp91, Glu182, Val198, Asp227, Phe240 oder Asp261 modifiziert sind und lediglich über 1 bis 80%, vorzugsweise lediglich über 1 bis 60%, weiter bevorzugt lediglich über 1,5 bis 30%, und besonders bevorzugt über lediglich 1,5 bis 26% der Enzymaktivität der wtNPT verfügen. Vorzugsweise können hierbei die Aminosäuren Tryp91, Glu182, oder Asp227 jeweils durch Glycin, Alanin, Valin, Leucin, Isoleucin, Phenylalanin oder Tyrosin an der entsprechenden Position ersetzt werden. Die Glutaminsäure an 182 kann ferner auch durch Asparaginsäure, Asparagin, Glutamin oder eine andere vorzugsweise negativ geladene Aminosäure ersetzt werden. Ferner sind modifizierte NPT-Gene bevorzugt, die an Aminosäureposition 198 im Vergleich zum wtNPT für Glycin, Alanin, Leucin, Isoleucin, Phenylalanin, Tyrosin oder Tryptophan kodieren. Darüberhinaus sind modifizierte NPT-Gene bevorzugt, die an Aminosäureposition 240 im Vergleich zum wtNPT für Glycin, Alanin, Valin, Isoleucin, Tyrosin oder Tryptophan kodieren. Ferner sind modifizierte NPT-Gene bevorzugt, die an Aminosäureposition 261 im Vergleich zum wtNPT für Glycin, Alanin, Leucin, Isoleucin, Phenylalanin, Tyrosin, Tryptophan, Asparagin, Glutamin oder Asparaginsäure kodieren. Besonders bevorzugt ist die Verwendung einer Mutante, bei der die Asparaginsäure an Position 227 durch Glycin, Alanin, Valin, Leucin oder Isoleucin ersetzt wird, die Asparaginsäure an Position 261 durch ein Alanin, Valin, Leucin, Isoleucin oder Glutamin, insbesondere durch Glycin oder Asparagin ersetzt wird.

Besonders bevorzugt sind Expressionsvektoren, die modifizierte NPT-Gene enthalten, die für eine Glu182Gly-, Glu182Asp-, Trp91Ala-, Val198Gly-, Asp227Ala-, Asp227Val-, Asp227Gly-, Asp261Gly-, Asp261Asn- oder Phe240Ile-Mutante kodieren, die im Fall der Glu182Gly-Mutante die Aminosäuresequenz der SEQ ID NO:4, im Fall der Glu182Asp-Mutante die Aminosäuresequenz der SEQ ID NO: 20, im Fall der Trp91Ala-Mutante die Aminosäuresequenz der SEQ ID NO:6, im Fall der Val198Gly-Mutante die Aminosäuresequenz der SEQ ID NO:8, im Fall der Asp227Ala-Mutante die Aminosäuresequenz der SEQ ID NO:10, im Fall der Asp227Val-Mutante die Aminosäuresequenz der SEQ ID NO:12, im Fall der Asp227Gly-Mutante die Aminosäuresequenz der SEQ ID NO:22, im Fall der Asp261Gly-Mutante die Aminosäuresequenz der SEQ ID NO:14, im Fall der Asp261Asn-Mutante die Aminosäuresequenz der SEQ ID NO:16 und im Fall der Phe240Ile-Mutante die Aminosäuresequenz der SEQ ID NO:18 enthält. Am meisten bevorzugt ist ein Expressionsvektor mit der Verwendung einer Asp227Val-, Asp227Gly, Asp261Gly-, Asp261Asn-, Phe240Ile oder Trp91Ala-Mutante, insbesondere wenn sie die in SEQ ID NO:12, SEQ ID NO:22, SEQ ID NO:14, SEQ ID NO:16, SEQ ID NO:18 oder SEQ ID NO:6 angegebene Aminosäuresequenz aufweist oder darüber hinaus, wenn sie durch die in SEQ ID NO:11, SEQ ID NO:21, SEQ ID NO:13, SEQ ID NO:15, SEQ ID NO:17 oder SEQ ID NO:5 angegebene Nukleinsäuresequenz kodiert wird oder diese enthält.

Darüber hinaus stellt die vorliegende Erfindung erstmals modifizierte Neomycin-Phosphotransferase-Gene sowie deren Genprodukte zur Verfügung, die im Vergleich zur wtNPT an Aminosäureposition Trp91, Val198 oder Phe240 für eine andere als die wt-Aminosäure kodieren. Dabei stellt die vorliegende Erfindung insbesondere erstmalig Trp91-, Val 198- oder Phe240-Mutanten zur Verfügung, die im Vergleich zur wtNPT ein geringere Enzymaktivität aufweisen. Die hier beschriebenen und im Rahmen der Erfindung zur Verfügung modifizierten NPT kodieren vorzugsweise an Aminosäureposition 91 für Alanin, an Position 198 für Glycin und an Position 240 für Isoleucin. Ferner stellt die vorliegende Erfindung erstmals NPT-Mutanten zur Verfügung, die im Vergleich zur wt-NPT an Position 182 für Glycin, an Position 227 für Alanin oder Valin und an Position 261 für Glycin kodieren. Hierbei werden sowohl die Gene als auch die Genprodukte (Enzyme) im Rahmen der vorliegenden Erfindung erstmalig zur Verfügung gestellt. In diesem Zusammenhang stellt die vorliegende Erfindung erstmalig modifizierte NPT mit den Aminosäuresequenzen gemäß SEQ ID NO:4, SEQ ID NO:6, SEQ ID NO:8, SEQ ID NO:10, SEQ ID NO:12, SEQ ID NO:14, und SEQ ID NO:18 zur Verfügung. Ferner stellt die vorliegende Erfindung modifizierte NPT-Gene mit den DNA-Sequenzen gemäß SEQ ID NO:3, SEQ ID NO:5, SEQ ID NO:7, SEQ ID NO:9, SEQ ID NO:11, SEQ ID NO:13, und SEQ ID NO:17 zur Verfügung.

### Gen von Interesse

Das im erfindungsgemäßen Expressionsvektor enthaltene Gen von Interesse umfasst eine Nukleotidsequenz beliebiger Länge, die für ein Produkt von Interesse kodiert. Das Genprodukt oder auch "Produkt von Interesse" ist in der Regel ein Protein, Polypeptid, Peptid bzw. Fragment oder Derivat davon. Es kann aber auch RNA oder antisense RNA sein. Das Gen von Interesse kann in voller Länge, in verkürzter Form, als Fusionsgen oder markiertes Gen vorliegen. Es kann sich um genomische DNA oder vorzugsweise cDNA bzw. entsprechende Fragmente oder Fusionen handeln. Das Gen von Interesse kann die native Gensequenz darstellen, mutiert oder auf sonstige Weise modifiziert sein. Derartige Modifikationen schließen Codon-Optimierungen zur Anpassung an eine bestimmte Wirtszelle und eine Humanisierung ein. Das Gen von Interesse kann z.B. für ein sekretiertes, zytoplasmatisches, kernlokalisiertes, membrangebundenes oder zelloberflächengebundenes Polypeptid kodieren.

Der Ausdruck "Nukleotidsequenz" oder "Nukleinsäuresequenz" bezeichnet ein Oligonukleotid, Nukleotide, Polynukleotide und deren Fragmente sowie DNA oder RNA genomischen oder synthetischen Ursprungs, die als Einzel- oder Doppelstrang vorliegen und den kodierenden oder den nicht-kodierenden Strang eines Gens repräsentieren kann. Zur Modifikation von Nukleinsäuresequenzen können Standardtechniken, wie z.B. ortsspezifische Mutagenese oder PCR-vermittelte Mutagenese ( z.B. in Sambrook et al., 1989 oder Ausubel et al., 1994 beschrieben), eingesetzt werden.

Unter "kodieren" versteht man die Eigenschaft oder Fähigkeit einer spezifischen Sequenz von Nukleotiden in einer Nukleinsäure, beispielsweise einem Gen in einem Chromosom oder einer mRNA, als Matrize für die Synthese von anderen Polymeren und Makromolekülen wie z.B. rRNA, tRNA, mRNA, anderen RNA-Molekülen, cDNA oder Polypeptiden in einem biologischen Prozess zu dienen. Demnach kodiert ein Gen für ein Protein, wenn durch Transkription und nachfolgende Translation der mRNA das gewünschte Protein in einer Zelle oder einem anderen biologischen System produziert wird. Sowohl der kodierende Strang, dessen Nukleotidsequenz identisch mit der mRNA-Sequenz ist und normalerweise auch in Sequenzdatenbanken, z.B. EMBL oder GenBank, angegeben wird, als auch der als Matrize für die Transkription dienende nicht-kodierende Strang eines Gens oder cDNA kann dabei als kodierend für ein Produkt oder Protein bezeichnet werden. Eine Nukleinsäure, die für ein Protein kodiert, schließt auch Nukleinsäuren mit ein, die auf Grund des degenerierten genetischen Codes eine andere Nukleotidsequenzabfolge aufweisen, aber in der gleichen Aminosäuresequenz des Proteins resultieren. Nukleinsäuresequenzen, die für Proteine kodieren, können auch Introns enthalten.

Mit dem Ausdruck "cDNA" werden Desoxyribonukleinsäuren bezeichnet, die durch reverse Transkription und Synthese des zweiten DNA-Strangs aus einer von einem Gen produzierten mRNA oder anderen RNA hergestellt werden. Liegt die cDNA als doppelstränges DNA-Molekül vor, dann enthält sie sowohl einen kodierenden als auch einen nicht-kodierenden Strang.

Mit dem Ausdruck "Intron" werden nicht-kodierende Nukleotidsequenzen beliebiger Länge bezeichnet. Sie kommen natürlicherweise in vielen eukaryontischen Genen vor und werden von einem zuvor transkribierten mRNA-Vorläufer durch einen als Spleissen bezeichneten Prozess entfernt. Hierfür ist ein exaktes Herausschneiden des Introns am 5'- und 3'-Ende und eine korrekte Verbindung der entstehenden mRNA-Enden erforderlich, damit eine reife prozessierte mRNA mit dem für die erfolgreiche Proteinsynthese richtigen Leseraster hergestellt wird. Viele der an diesem Spleissing-Prozess beteiligten Spleiss-Donor- und Spleiss-Akzeptor-Stellen, das sind die unmittelbar an den Exon-Intron- bzw. Intron-Exon-Grenzen vorliegenden Sequenzen, sind mittlerweile charakterisiert worden. Für einen Überblick siehe Ohshima et al., 1987.

### Protein/Produkt von Interesse

Biopharmazeutisch bedeutsame Proteine/Polypeptide umfassen z.B. Antikörper, Enzyme, Cytokine, Lymphokine, Adhäsionsmoleküle, Rezeptoren sowie deren Derivate bzw. Fragmente, sind aber nicht auf diese beschränkt. Im Allgemeinen sind alle Polypeptide bedeutsam, die als Agonisten oder Antagonisten wirken und/oder therapeutische oder diagnostische Anwendung finden können.

Der Ausdruck "Polypeptide" wird für Aminosäuresequenzen oder Proteine verwendet und bezeichnet Polymere von Aminosäuren beliebiger Länge. Dieser Ausdruck schließt auch Proteine ein, die posttranslational durch Reaktionen wie beispielsweise Glykosylierung, Phosphorylierung, Acetylierung oder Proteinprozessierung modifiziert werden. Die Struktur des Polypeptids kann z.B. durch Substitutionen, Deletionen oder Insertionen von Aminosäuren, Fusion mit anderen Proteinen, unter Beibehaltung seiner biologischen Aktivität modifiziert werden. Der Ausdruck Polypeptide schließt somit beispielsweise auch Fusionsproteine bestehend aus einem Immunglobulinanteil, z.B. dem Fc-Anteil, und einem Wachstumsfaktor, z.B. einem Interleukin, mit ein.

Beispiele für therapeutische Proteine sind Insulin, Insulin-ähnlicher Wachstumsfaktor, humanes Wachstumshormon (hGH) und andere Wachstumsfaktoren, Gewebeplasminogenaktivator (tPA), Erythropoetin (EPO), Cytokine, beispielsweise Interleukine (IL) wie IL-1, IL-2, IL-3, IL-4, IL-5, IL-6, IL-7, IL-8, IL-9, IL-10, IL-11, IL-12, IL-13, IL-14, IL-15, IL-16, IL-17, IL-18, Interferon (IFN)-alpha, beta, gamma, omega oder tau, Tumornekrosefaktor (TNF) wie z.B. TNF-alpha, beta oder gamma, TRAIL, G-CSF, GM-CSF, M-CSF, MCP-1 und VEGF. Weitere Beispiele sind monoklonale, polyklonale, multispezifische und einzelkettige *(single chain)* Antikörper und Fragmente davon, wie z.B. Fab, Fab', F(ab')₂, Fc und Fc'-Fragmente, leichte (L) und schwere (H) Immunglobulinketten und deren konstante, variable oder hypervariable Regionen sowie Fv- und Fd-Fragmente (Chamov et al., 1999). Die Antikörper können humanen oder nicht-humanen Ursprungs sein. Auch humanisierte und chimäre Antikörper kommen in Frage.

Fab-Fragmente (Fragment antigen-binding = Fab) bestehen aus den variablen Regionen beider Ketten, die durch die angrenzenden konstanten Regionen zusammengehalten werden. Sie können z.B. durch Behandlung mit einer Protease, wie beispielsweise Papain, aus konventionellen Antikörpern erzeugt werden oder aber auch durch DNA-Klonierung. Weitere Antikörperfragmente sind F(ab')₂-Fragmente, die durch proteolytischen Verdau mit Pepsin hergestellt werden können.

Durch Genklonierung können auch verkürzte Antikörperfragmente hergestellt werden, die nur aus den variablen Region der schweren (VH) und der leichten Kette (VL) bestehen. Diese werden als Fv-Fragmente (Fragment variable = Fragment des variablen Teils) bezeichnet. Da bei diesen Fv-Fragmenten die kovalente Verbindung über die Cysteinreste der konstanten Ketten nicht möglich ist, werden diese Fv-Fragmente oft anderweitig stabilisiert. Dazu werden die variablen Region der schweren und leichten Kette häufig mittels eines kurzen Peptidfragments von ca. 10 - 30 Aminosäuren, besonders bevorzugt 15 Aminosäuren, miteinander verknüpft. Auf diese Weise entsteht eine einzelne Polypeptidkette, in der VH und VL durch einen Peptidlinker miteinander verbunden sind. Solche Antikörperfragmente werden auch als *single-chain* Fv-Fragment (scFv) bezeichnet. Beispiele von scFv-Antikörpern sind bekannt und beschrieben, siehe z.B. Huston et al. (1988).

In den vergangenen Jahren wurden verschiedene Strategien entwickelt um multimere scFv-Derivate herzustellen. Die Intention besteht in der Erzeugung von rekombinanten Antikörpern mit verbesserten pharmakokinetischen Eigenschaften und verstärkter Bindungsavidität. Zur Erreichung der Multimerisierung der scFv-Fragmente werden diese als Fusionsproteine mit Multimerisierungsdomänen hergestellt. Als Multimerisierungsdomänen können dabei z.B. die CH3-Region eines lgGs oder Helixstrukturen *(,,coiled coil structure")* wie die Leucin-Zipper-Domänen fungieren. In anderen Strategien wird die Interaktion zwischen den VH- und VL-Regionen des scFv-Fragments für eine Multimerisierung genutzt (z.B. Dia-, Tri- und Pentabodies).

Als "Diabody" bezeichnet ein Fachmann ein bivalentes homodimeres scFv-Derivat. Die Verkürzung des Peptidlinkers im scFv-Moleküle auf 5 - 10 Aminosäuren resultiert in der Bildung von Homodimeren durch Überlagerung von VH/VL-Ketten. Die Diabodies können zusätzlich durch eingeführte Disulfidbrücken stabilisiert werden. Beispiele von Diabodies finden sich in der Literatur, z.B. bei Perisic et al. (1994).

Als "Minibody" bezeichnet der Fachmann ein bivalentes, homodimeres scFv-Derivat. Es besteht aus einem Fusionsprotein, das die CH3-Region eines Immunglobulins, vorzugsweise lgG, besonders bevorzugt lgG1, als Dimerisierungsregion enthält. Diese verbindet die scFv-Fragmente über eine Hinge-Region, ebenfalls von lgG, und eine Linker-Region. Beispiele solcher Minibodies sind bei Hu et al. (1996) beschrieben.

Mit "Triabody" bezeichnet der Fachmann ein trivalentes homotrimeres scFv-Derivat (Kortt et al., 1997). Die direkte Fusion von VH-VL ohne Verwendung einer Linkersequenz führt zur Ausbildung von Trimeren.

Bei den vom Fachmann als Mini-Antikörper bezeichneten Fragmenten, die eine bi-, tri- oder tetravalente Struktur haben, handelt es sich ebenfalls um Derivate von scFv-Fragmenten. Die Multimerisierung wird dabei über di-, tri- oder tetramere "coiled coil"-Strukturen erzielt (Pack et al., 1993 und 1995; Lovejoy et al., 1993).

### Gen, das für ein fluoreszierendes Protein kodiert

In einer weiteren Ausführungsform enthält der erfindungsgemäße Expressionsvektor ein für ein fluoreszierendes Protein kodierendes Gen, vorzugsweise in funktioneller Verknüpfung mit dem Gen von Interesse. Vorzugsweise erfolgt die Transkription beider Gene unter der Kontrolle eines einzigen heterologen Promotors, so dass das Protein/Produkt von Interesse und das fluoreszierende Protein durch eine bicistronische mRNA kodiert werden. Dies ermöglicht eine Identifizierung von Zellen, die das Protein/Produkt von Interesse in hohen Menge produzieren, über die Expressionsrate des fluoreszierenden Proteins.

Bei dem fluoreszierenden Protein kann es sich z.B. um ein grün, blaugrün, blau, gelb oder andersfarben fluoreszierendes Protein handeln. Ein spezielles Beispiel ist das grün fluoreszierende Protein (GFP) aus *Aequorea victoria* oder *Renilla reniformis* und daraus entwickelte Mutanten; siehe z.B. Bennet et al. (1998); Chalfie et al. (1994); WO 01/04306 und die dort zitierte Literatur.

Weitere fluoreszierende Proteine und dafür kodierende Gene sind in WO 00/34318, WO 00/34326, WO 00/34526 und WO 01/27150 beschrieben, die durch Bezugnahme hierin inkorporiert werden. Bei diesen fluoreszierenden Proteinen handelt es sich um Fluorophore von nicht-biolumineszierenden Organismen der Spezies Anthozoa, beispielsweise von *Anemonia majano, Clavularia sp., Zoanthus sp.* I*, Zoanthus sp.* II, *Discosoma striata, Discosoma sp.* "red", *Discosoma sp.* "green", *Discosoma sp.* "Magenta", *Anemonia sulcata.*

Die erfindungsgemäß eingesetzten Fluoreszenzproteine beinhalten neben den Wildtyp-Proteinen auch natürliche oder gentechnologisch hergestellte Mutanten und -varianten, deren Fragmente, Derivate oder z.B. mit anderen Proteinen oder Peptiden fusionierte Varianten. Die eingebrachten Mutationen können dabei beispielsweise das Exzitations- oder Emissionsspektrum, die Chromophorenbildung, den Extinktionskoeffizienten oder die Stabilität des Proteins verändern. Durch Codon-Optimierung kann zudem die Expression in Säugerzellen oder anderen Spezies verbessert werden. Erfindungsgemäß kann das fluoreszierende Protein auch in Fusion mit einem Selektionsmarker, bevorzugterweise mit einem amplifzierbaren Selektionsmarker wie beispielsweise der Dihydrofolat-Reduktase (DHFR), eingesetzt werden.

Die von den fluoreszierenden Proteinen emittierte Fluoreszenz ermöglicht die Detektion der Proteine z.B. durch Durchflusszytometrie mit einem Fluoreszenz-aktivierten Zellsortierer (FACS) oder durch Fluoreszenzmikroskopie.

### Weitere regulatorische Elemente

Der Expressionsvektor enthält zumindest einen heterologen Promotor, der die Expression des Gens von Interesse und vorzugsweise auch die des fluoreszierenden Proteins ermöglicht.

Als "Promotor" wird eine Polynukleotidsequenz bezeichnet, die die Transkription der mit ihr funktionell verknüpften Gene oder Sequenzen ermöglicht und kontrolliert. Ein Promotor enthält Erkennungssequenzen für die Bindung der RNA-Polymerase und die Initiationsstelle der Transkription (Transkriptionsinitiationsstelle). Zur Expression einer gewünschten Sequenz in einem bestimmten Zelltyp oder einer Wirtszelle muss jeweils ein geeigneter, funktionaler Promotor gewählt werden. Der Fachmann kennt eine Vielzahl von Promotoren aus verschiedenen Quellen, einschließlich konstitutiver, induzierbarer und reprimierbarer Promotoren. Sie sind in Datenbanken, z.B. GenBank, hinterlegt und können als eigenständige oder innerhalb von Polynukleotidsequenzen klonierte Elemente von kommerziellen oder individuellen Quellen bezogen werden. In induzierbaren Promotoren kann die Aktivität des Promotors in Reaktion auf ein Signal reduziert oder verstärkt werden. Ein Beispiel für einen induzierbaren Promotor stellt der Tetracyclin (tet)-Promotor dar. Dieser enthält Tetracyclin-Operatorsequenzen (tetO), die durch ein Tetracyclin-reguliertes Transaktivatorprotein (tTA) induziert werden können. In der Anwesenheit von Tetracyclin wird die Bindung von tTA an tetO inhibiert. Beispiele für weitere induzierbare Promotoren sind der jun-, fos-, Metallothionin- und Hitzeschockpromotor (siehe auch Sambrook et al., 1989; Gossen et al., 1994).

Unter den Promotoren, die für eine hohe Expression in Eukaryonten besonders gut geeignet sind, befinden sich beispielsweise der Ubiquitin/S27a-Promotor des Hamsters (WO 97/15664), der SV40 early Promotor, der Adenovirus major late Promotor, der Maus Metallothionin-I Promotor, die lange terminale Repeatregion des Rous Sarcoma Virus, der early Promotor des humanen Cytomegalie-Virus. Beispiele für andere heterologe Säugerpromotoren sind der/die Aktin-, Immunglobulin- oder Hitzeschockpromotor(en).

Ein entsprechender heterologer Promotor kann zur Steigerung/Regulierung der Transkriptionsaktivität in einer Expressionskassette in funktionellen Zusammenhang mit weiteren regulatorischen Sequenzen gebracht werden.

Beispielsweise kann der Promotor mit Enhancer-Sequenzen funktionell verknüpft werden, um die Transkriptionsaktivität zu steigern. Hierzu können ein oder mehrere Enhancer und/oder mehrere Kopien einer Enhancer-Sequenz verwendet werden, beispielsweise ein CMV- oder SV40-Enhancer. Dem entsprechend enthält ein erfindungsgemäßer Expressionsvektor in einer weiteren Ausführungsform einen oder mehrere Enhancer / Enhancer-Sequenzen, vorzugsweise einen CMV- oder SV40-Enhancer.

Mit dem Ausdruck "Enhancer" wird eine Polynukleotidsequenz bezeichnet, die in *cis-*Lokalisierung auf die Aktivität eines Promotors einwirkt und so die Transkription eines mit diesem Promotor funktionell verknüpften Gens stimuliert. Im Gegensatz zu Promotoren ist die Wirkung der Enhancer positions- und orientierungsunabhängig und können somit vor oder hinter eine Transkriptionseinheit, innerhalb eines Introns oder selbst innerhalb der Kodierregion positioniert werden. Der Enhancer kann dabei sowohl in unmittelbarer Nähe der Transkriptionseinheit als auch in beträchtlichem Abstand zum Promotor lokalisiert sein. Auch eine physikalische und funktionelle Überlappung mit dem Promotor ist möglich. Dem Fachmann sind eine Vielzahl von Enhancern aus verschiedenen Quellen bekannt (und in Datenbanken wie GenBank hinterlegt, z.B. SV40 Enhancer, CMV Enhancer, Polyoma Enhancer, Adenovirus Enhancer) und als eigenständige oder innerhalb von Polynukleotidsequenzen klonierte Elemente verfügbar (z.B. bei ATCC hinterlegt oder aus kommerziellen und individuellen Quellen). Eine Vielzahl von Promotorsequenzen beinhalten auch Enhancer-Sequenzen, wie z.B. der häufig verwendete CMV Promotor. Der humane CMV-Enhancer gehört dabei zu den stärksten bisher identifizierten Enhancern. Ein Beispiel für einen induzierbaren Enhancer ist der Metallothionein-Enhancer, der durch Glucocorticoide oder Schwermetalle stimuliert werden kann.

Eine weitere mögliche Modifikation ist z.B. die Einführung multipler Sp1-Bindungsstellen. Die Promotorsequenzen können ferner mit regulatorischen Sequenzen kombiniert werden, die eine Steuerung/Regulierung der Transkriptionsaktivität gestatten. So kann der Promotor reprimierbar/induzierbar gemacht werden. Dies kann beispielsweise durch die Verknüpfung mit Sequenzen geschehen, die Bindungsstellen für positiv oder negativ regulierende Transkriptionsfaktoren darstellen. Der oben genannte Transkriptionsfaktor SP-1 beispielsweise hat einen positiven Einfluss auf die Transkriptionsaktivität. Ein weiteres Beispiel ist die Bindungsstelle für das Aktivatorprotein AP-1, das sowohl in positiver als auch in negativer Weise auf die Transkription einwirken kann. Die Aktivität des AP-1 kann durch verschiedenste Faktoren, wie z.B. Wachstumsfaktoren, Cytokine und Serum, gesteuert werden (Faisst et al., 1992, und Referenzen darin). Die Transkriptionseffizienz kann auch dadurch gesteigert werden, dass die Promotorsequenz durch Mutation (Substitution, Insertion oder Deletion) von einer, zwei, drei oder mehr Basen verändert wird und dann in einem Reportergen-Assay bestimmt wird, ob sich dadurch die Promotoraktivität erhöht.

Grundsätzlich umfassen die zusätzlichen regulatorischen Elemente heterologe Promotoren, Enhancer, Terminations- und Polyadenylierungssignale und weitere Expressionskontrollelemente. Für die verschiedenen Zelltypen sind sowohl induzierbare als auch konstitutive regulatorische Sequenzen bekannt.

"Transkriptionsregulatorische Elemente" umfassen gewöhnlich einen Promotor stromaufwärts von der zu exprimierenden Gensequenz, Transkriptionsinitiations- und -terminationsstellen sowie ein Polyadenylierungssignal.

Der Ausdruck "Transkriptionsinitiationsstelle" bezieht sich auf eine Nukleinsäure in dem Konstrukt, die der ersten Nukleinsäure entspricht, welche in das primäre Transkript, d.h. den mRNA-Precursor, inkorporiert wird. Die Transkriptionsinitiationsstelle kann mit den Promotorsequenzen überlappen.

Der Ausdruck ,,Transkriptionsterminationsstelle" bezieht sich auf eine Nukleotidsequenz, die normalerweise am 3'-Ende des Gens von Interesse oder des zu transkribierenden Genabschnitts vorhanden ist und die den Abbruch der Transkription durch RNA-Polymerase bewirkt.

Das "Polyadenylierungssignal" ist eine Signalsequenz, welche die Spaltung an einer spezifischen Stelle am 3'-Ende der eukaryontischen mRNA und den posttranskriptionellen Einbau einer Sequenz von etwa 100-200 Adeninnukleotiden (polyA-Schwanz) am gespaltenen 3'-Ende verursacht. Das Polyadenylierungssignal umfasst die Sequenz AATAAA etwa 10-30 Nukleotide stromaufwärts von der Spaltstelle sowie eine stromabwärts gelegene Sequenz. Es sind verschiedene Polyadenylierungselemente bekannt, z.B. tk polyA, SV40 late und early polyA oder BGH polyA (z.B. beschrieben in US 5,122,458).

In einer bevorzugten Ausführungsform der vorliegenden Erfindung verfügt jede Transkriptionseinheit über einen Promotor oder ein Promotor/Enhancer-Element, ein Gen von Interesse und/oder ein Markergen, sowie über ein Transkriptionsterminationselement. In einer weiter bevorzugten Ausführungsform enthält die Transkriptionseinheit weitere translationsregulatorische Einheiten.

,,Translationsregulatorische Elemente" umfassen eine Translationsinitiationsstelle (AUG), ein Stoppcodon und ein polyA-Signal für jedes zu exprimierende Polypeptid. Für eine optimale Expression kann es günstig sein, 5'- und/oder 3'-nichttranslatierte Bereiche der zu exprimierenden Nukleinsäuresequenz zu entfernen, hinzuzufügen oder zu ändern, um potentielle zusätzliche ungeeignete Translationsinitiationscodons oder andere Sequenzen, welche die Expression auf dem Niveau der Transkription oder Expression beeinträchtigen können, zu eliminieren. Um die Expression zu fördern, kann man alternativ ribosomale Konsensus-Bindungsstellen unmittelbar stromaufwärts vom Startcodon inserieren. Um ein sekretiertes Polypeptid zu produzieren, enthält das Gen von Interesse gewöhnlich eine Signalsequenz, welche für ein Signalvorläuferpeptid kodiert, die das synthetisierte Polypeptid zu und durch die ER-Membran transportiert. Die Signalsequenz befindet sich oft, jedoch nicht immer, am Aminoterminus des sekretierten Proteins und wird durch Signal-Peptidasen abgespalten, nachdem das Protein durch die ER-Membran geschleust wurde. Die Gensequenz wird gewöhnlich, jedoch nicht notwendigerweise, eine eigene Signalsequenz enthalten. Wenn die native Signalsequenz nicht vorhanden ist, kann in bekannter Weise eine heterologe Signalsequenz eingeführt werden. Dem Fachmann sind zahlreiche solcher Signalsequenzen bekannt und in Sequenzdatenbanken wie GenBank und EMBL hinterlegt.

Ein erfindungsgemäß besonderes bedeutsames regulatorisches Element ist die interne Ribosomenbindungsstelle (IRES). Das "IRES-Element" umfasst eine Sequenz, welche die Translationsinitiation unabhängig von einer 5'-terminalen Methylguanosiniumkappe (CAP-Struktur) sowie dem stromaufwärts gelegenen Gen funktionell bewerkstelligt und in einer tierischen Zelle die Translation zweier Cistrone (offener Leseraster) von einem einzigen Transkript ermöglicht. Das IRES-Element stellt eine unabhängige Ribosomenbindungsstelle für die Translation des unmittelbar stromabwärts gelegenen offenen Leserasters zur Verfügung. Im Gegensatz zu bakterieller mRNA, die multicistronisch sein kann, d.h. für mehrere verschiedene Polypeptide oder Produkte kodieren kann, die nacheinander von der mRNA translatiert werden, sind die meisten mRNAs von Tierzellen monocistronisch und kodieren nur für ein einziges Protein oder Produkt. Bei einem multicistronischen Transkript in einer eukaryontischen Zelle würde die Translation von der stromaufwärts am nächsten gelegenen Translationsinitiationsstelle initiiert und vom ersten Stoppcodon beendet werden, worauf das Transkript aus dem Ribosom freigesetzt würde. Bei der Translation entstünde somit nur das erste von der mRNA kodierte Polypeptid oder Produkt. Dagegen ermöglicht ein multicistronisches Transkript mit einem IRES-Element, das mit dem zweiten oder weiteren offenen Leserastern in dem Transkript funktionell verknüpft ist, die anschließende Translation des stromabwärts gelegenen offenen Leserasters, so dass in der eukaryontischen Zelle zwei oder mehr, von demselben Transkript kodierte Polypeptide oder Produkte produziert werden.

Das IRES-Element kann von verschiedener Länge und von verschiedenem Ursprung sein und z.B. vom Encephalomyocarditisvirus (EMCV) oder anderen Picornaviren stammen. In der Literatur sind verschiedene IRES-Sequenzen und deren Anwendung bei der Konstruktion von Vektoren beschrieben worden; siehe z.B. Pelletier et al., 1988; Jang et al., 1989; Davies et al., 1992; Adam et al., 1991; Morgan et al., 1992; Sugimoto et al., 1994; Ramesh et al., 1996, Mosser et al., 1997.

Die stromabwärts gelegene Gensequenz ist mit dem 3'-Ende des IRES-Elements funktionell verknüpft, d.h. der Abstand wird so gewählt, dass die Expression des Gens nicht oder nur marginal beeinflusst wird bzw. eine für den Zweck ausreichende Expression aufweist. Der optimale und zulässige Abstand zwischen dem IRES-Element und dem Startcodon des stromabwärts gelegenen Gens für eine noch ausreichende Expression lässt sich in einfachen Versuchen durch Variation des Abstands und Bestimmung der Expressionsrate als Funktion des Abstandes mit Hilfe von Reportergen-Assays ermitteln.

Durch die geschilderten Maßnahmen kann eine optimierte Expressionskassette erhalten werden, die von hohem Nutzen für die Expression heterologer Genprodukte ist. Eine durch eine oder mehrere solcher Maßnahmen erhaltene Expressionskassette ist daher ebenfalls Gegenstand der Erfindung.

### Hamster-Ubiquitin/S27a-Promotor

In einer weiteren Ausführungsform enthält der erfindungsgemäße Expressionsvektor den Ubiquitin/S27a-Promotor des Hamsters, vorzugsweise in funktioneller Verknüpfung mit dem Gen von Interesse und noch mehr bevorzugt in funktioneller Verknüpfung mit dem Gen von Interesse und dem Gen, das für ein fluoreszierendes Protein kodiert.

Der Ubiquitin/S27a-Promotor des Hamsters ist ein starker homologer Promotor, der in der WO 97/15664 beschrieben ist. Ein solcher Promotor weist vorzugsweise mindestens eines der folgenden Merkmale auf: GC-reicher Sequenzbereich, Sp1-Bindungsstelle, Polypyrimidinelement, Abwesenheit einer TATA-Box. Besonders bevorzugt ist ein Promotor, der eine Sp1-Bindungsstelle bei Abwesenheit einer TATA-Box aufweist. Ferner ist ein solcher Promotor bevorzugt, der konstitutiv aktiviert ist und insbesondere unter serumhaltigen, serumarmen und serumfreien Zellkulturbedingungen gleichermaßen aktiv ist. In einer anderen Ausführungsform handelt es sich um einen induzierbaren Promotor, insbesondere um einen Promotor, der durch Serumentzug aktiviert wird.

Eine besonders vorteilhafte Ausführungsform ist ein Promotor mit einer Nukleotidsequenz, die in Fig. 5 der WO 97/15664 enthalten ist. Besonders bevorzugt sind dabei Promotorsequenzen, in denen die Sequenz von Position -161 bis - 45 von Fig. 5 enthalten ist.

Die in den Beispielen der vorliegenden Patentbeschreibung verwendeten Promotoren beinhalten jeweils ein DNA-Molekül mit der Sequenz von Position 1923 bis 2406 der SEQ ID NO:55 des beiliegenden Sequenzprotokolls. Diese Sequenz entspricht dem Fragment - 372 bis + 111 aus Fig. 5 der WO 97/15664 und repräsentiert den bevorzugten Promotor, d.h. ein bevorzugter Promotor sollte diesen Sequenzbereich umfassen. Ein anderes geeignetes Promotorfragment enthält die Sequenz von Position 2134 bis 2406 (entspricht - 161 bis + 111 in Fig. 5 der WO 97/15664). Ein Promotor, der lediglich die Sequenz von Position 2251 bis 2406 beinhaltet, ist nicht mehr funktionsfähig (entspricht Position - 45 bis + 111 in Fig. 5 der WO 97/15664). Eine Verlängerung der Promotorsequenz in 5'-Richtung ausgehend von Position 2134 ist möglich.

Es können auch funktionelle Subfragmente der vollständigen Hamster-Ubiquitin/S27a-Promotorsequenz sowie funktionelle Mutanten/Varianten der vollständigen Sequenz oder Subfragmente hiervon eingesetzt werden, die z.B. durch Substitutionen, Insertionen oder Deletionen modifiziert worden sind. Entsprechende Subfragmente, Mutanten oder Varianten werden nachfolgend auch als "modifizierter Promotor" bezeichnet.

Ein modifizierter Promotor, gegebenenfalls kombiniert mit weiteren regulatorischen Elementen, weist vorzugsweise eine Transkriptionsaktivität auf, die der des Promotorfragments von Position 1923 bis 2406 der in SEQ ID NO:55 angegebenen Nukleotidsequenz (- 372 bis + 111 aus Fig. 5 der WO 97/15664) entspricht. Ein modifizierter Promotor erweist sich als tauglich im Sinne der Erfindung, wenn er über eine Transkripitionsaktivität verfügt, die mindestens 50%, besser mindestens 80%, noch besser mindestens 90%, und noch mehr bevorzugt mindestens 100% der Aktivität des 1923 bis 2406 Fragments (- 372 bis + 111 Fragments) in einem vergleichenden Reportergen-Assay aufweist. Insbesondere bevorzugt sind modifizierte Promotoren, die eine minimale Sequenzhomologie zur Wildtyp-Sequenz SEQ ID NO:55 des Hamster-Ubiquitin/S27a-Promotors von mindestens 80%, besser mindestens 85%, bevorzugt mindestens 90%, weiter bevorzugt mindestens 95% und besonders bevorzugt mindestens 97% aufweisen und über eine entsprechende Promotoraktivität in einem vergleichenden Reportergen-Assay verfügen.

In einem entsprechenden vergleichenden Reportergen-Assay werden die zu testenden Promotorfragmente einschließlich der Referenzsequenz jeweils vor ein promotorloses Reportergen kloniert, das z.B. für Luciferase, sezernierte Alkalische Phosphatase oder grünes fluoreszierendes Protein (GFP) kodiert. Diese Konstrukte (Promotorsequenz + Reportergen) werden anschließend in die Testzellen, z.B. CHO-DG44, mittels Transfektion eingeführt und die Induktion der Reportergenexpression durch das jeweilige Promotorfragment über die Bestimmung des Proteingehalts des Reportergens ermittelt. Ein entsprechender Test findet sich beispielhaft auch in Ausubel et al., Current Protocols in Molecular Biology, 1994, updated.

Die Promotorsequenz des Hamster-Ubiquitin/S27a-Promotors sowie die modifizierter Promotoren, die z.B. auch die 5'-untranslatierte Region bzw. ausgewählte Fragmente hiervon umfassen können, und die kodierende Region sowie die 3'-untranslatierte Region des Ubiquitin/S27a-Gens bzw. ausgewählte Fragmente hiervon, können von einem Fachmann in Kenntnis der in der WO 97/15664 beschriebenen Sequenz mit verschiedenen Standardmethoden erhalten werden, wie z.B. in Sambrook et al., 1989; Ausubel et al., 1994 beschrieben. Ausgehend von der in WO 97/15664 beschriebenen Sequenz kann beispielsweise ein geeigneter Abschnitt ausgewählt und eine Oligonukleotid-Sonde chemisch synthetisiert werden, die die Sequenz dieses Abschnitts enthält. Mit einer solchen Sonde kann z.B. durch Hybridisierung aus einer genomischen Bibliothek des Hamsters das Ubiquitin/S27a-Gen bzw. dessen 5'-untranslatierte Region oder sonstige Fragmente kloniert werden. Mittels des oben beschrieben Reportergen-Assays ist der Fachmann in der Lage, ohne erheblichen Aufwand promotoraktive Fragmente zu identifizieren und im Sinne der vorliegenden Erfindung zu verwenden. Die 5'-untranslatierte Region bzw. spezielle Fragmente davon können auch leicht durch PCR-Amplifikation mit entsprechenden Primern aus genomischer DNA oder einer genomischen Bibliothek erhalten werden. Fragmente der 5'-untranslatierten Region können auch durch limitierten Exonuklease III-Verdau aus größeren DNA-Fragmenten erhalten werden. Solche DNA-Moleküle können auch chemisch synthetisiert oder aus chemisch synthetisierten Fragmenten durch Ligation erzeugt werden.

Deletions-, Insertions- und Substitutionsmutanten lassen sich mittels "ortsspezifischer Mutagenese" und / oder "PCR-basierten Mutagenese-Techniken" erzeugen. Entsprechende Methoden sind beispielhaft in Lottspeich und Zorbas (1998) (Kapitel 36.1), mit weiteren Verweisen, aufgeführt.

Es ist auch möglich über Kreuzhybridisierung mit Sonden aus dem 5'-untranslatierten Bereich des Hamster-Ubiquitin/S27a-Gens oder aus dem S27a-Anteil des Hamster-Ubiquitin/S27a-Gens bzw. der 3'-untranslatierten Region geeignete Promotorsequenzen aus korrespondierenden homologen Genen anderer, bevorzugt Säugerspezies, zu identifizieren und zu isolieren. Entsprechende Techniken sind beispielhaft in Lottspeich und Zorbas (1998) (Kapitel 23) beschrieben. "Homolog" im Sinne der Erfindung sind Gene, sofern ihre Nukleotidsequenz mindestens 70%, besser mindestens 80%, bevorzugt mindestens 90%, weiter bevorzugt mindestens 95% und besonders bevorzugt mindestens 97% Übereinstimmung mit der Nukleotidsequenz des Gens zeigt, zu dem es homolog ist.

Durch die geschilderten Maßnahmen kann eine optimierte Expressionskassette erhalten werden, die von hohem Nutzen für die Expression heterologer Genprodukte ist. Eine durch eine oder mehrere solcher Maßnahmen erhaltene Expressionskassette ist daher ebenfalls Gegenstand der Erfindung.

### Herstellung erfindungsgemäßer Expressionsvektoren

Die Herstellung des erfindungsgemäßen Expressionsvektors kann grundsätzlich nach herkömmlichen, dem Fachmann geläufigen Methoden, wie z.B. bei Sambrock et al. (1989) beschrieben, erfolgen. Dort findet sich auch eine Beschreibung der funktionellen Komponenten eines Vektors, z.B. geeigneter Promotoren (zusätzlich zum Hamster-Ubiquitin/S27a-Promotor), Enhancer, Terminations- und Polyadenylierungssignale, Antibiotikaresistenzgene, Selektionsmarker, Replikationsstartpunkte und Spleisssignale. Zur Herstellung können herkömmliche Klonierungsvektoren verwendet werden, z.B. Plasmide, Bakteriophagen, Phagemide, Cosmide oder virale Vektoren wie Baculovirus, Retroviren, Adenoviren, Adenoassoziierte Viren und Herpes Simplex-Virus, aber auch künstliche Chromosomen/Mini-Chromosomen. Die eukaryontischen Expressionsvektoren enthalten typischerweise auch prokaryontische Sequenzen wie z.B. Replikationsursprung und Antibiotikaresistenzgene, die die Vermehrung und Selektion des Vektors in Bakterien ermöglichen. Eine Vielzahl von eukaryontischen Expressionsvektoren, die multiple Klonierungsstellen zur Einführung einer Polynukleotidsequenz enthalten, sind bekannt und einige sind kommerziell bei verschiedenen Firmen wie Stratagene, La Jolla, CA, USA; Invitrogen, Carlsbad, CA, USA; Promega, Madison, WI, USA oder BD Biosciences Clontech, Palo Alto, CA, USA erhältlich.

Auf eine dem Fachmann geläufige Weise werden der heterologe Promotor, das Gen von Interesse, sowie das modifizierte Neomycin-Phosphotransferase-Gen sowie gegebenenfalls das für ein fluoreszierendes Protein kodierende Gen, zusätzliche regulatorische Elemente wie die interne Ribosomenbindungsstelle (IRES), Enhancer oder ein Polyadenylierungssignal in den Expressionsvektor eingeführt. Ein erfindungsgemäßer Expressionsvektor enthält minimal einen heterologen Promotor, das Gen von Interesse und ein modifiziertes Neomycin-Phosphotransferase-Gen. Vorzugsweise enthält der Expressionsvektor noch ein für ein fluoreszierendes Protein kodierendes Gen. Erfindungsgemäß ist insbesondere die Verwendung eines Ubiquitin/S27a-Promotors als heterologen Promotor. Besonders bevorzugt ist ein Expressionsvektor, bei dem der heterologe Promotor, vorzugsweise ein Ubiquitin/S27a-Promotor, das Gen von Interesse und das Gen, welches für ein fluoreszierendes Protein kodiert, funktionell miteinander verknüpft sind oder in funktioneller Verknüpfung stehen und das Neomycin-Phosphotransferase-Gen in der selben oder aber in einer separaten Transkriptionseinheit lokalisiert ist.

Im Rahmen der vorliegenden Beschreibung bezieht sich der Ausdruck "funktionelle Verknüpfung" bzw. "funktionell verknüpft" auf zwei oder mehr Nukleinsäuresequenzen oder -teilsequenzen, die so positioniert sind, dass sie ihre beabsichtigte Funktion ausüben können. Beispielsweise ist ein Promotor/Enhancer funktionell mit einer kodierenden Gensequenz verknüpft, wenn er in cis-Stellung die Transkription der verknüpften Gensequenz kontrollieren oder modulieren kann. Im Allgemeinen, jedoch nicht notwendigerweise, befinden sich funktionell verknüpfte DNA-Sequenzen in enger Nachbarschaft und, sofern zwei kodierende Gensequenzen verknüpft werden oder im Falle einer Sekretionssignalsequenz, im gleichen Leseraster. Obwohl sich ein funktionell verknüpfter Promotor im Allgemeinen stromaufwärts von der kodierenden Gensequenz befindet, muss er nicht notwendigerweise eng benachbart sein. Enhancer müssen ebenfalls nicht in enger Nachbarschaft vorliegen, solange sie die Transkription der Gensequenz begünstigen. Zu diesem Zweck können sie sowohl stromaufwärts als auch stromabwärts von der Gensequenz vorliegen, gegebenenfalls in einigem Abstand. Eine Polyadenylierungsstelle ist funktionell mit einer Gensequenz verknüpft, wenn sie am 3'-Ende der Gensequenz derart positioniert ist, dass die Transkription über die kodierende Sequenz bis hin zum Polyadenylierungssignal fortschreitet. Die Verknüpfung kann nach üblichen rekombinanten Methoden erfolgen, z.B. mittels der PCR-Technik, durch Ligation an geeigneten Restriktionsschnittstellen oder durch Spleissen. Wenn keine geeigneten Restriktionsschnittstellen vorhanden sind, können in an sich bekannter Weise synthetische Oligonukleotid-Linker oder Adaptoren verwendet werden. Erfindungsgemäß erfolgt die funktionelle Verknüpfung vorzugsweise nicht über Intronsequenzen.

In einer der beschriebenen Ausführungsformen sind der heterologe Promotor, vorzugsweise ein Ubiquitin/S27a-Promotor, das Gen von Interesse und das für ein fluoreszierendes Protein kodierende Gen funktionell miteinander verknüpft. Dies meint z.B., dass sowohl das Gen von Interesse als auch das für ein fluoreszierendes Protein kodierende Gen ausgehend von dem selben heterologen Promotor exprimiert werden. In einer besonders bevorzugten Ausführungsform erfolgt die funktionelle Verknüpfung über ein IRES-Element, so dass von beiden Genen eine bicistronische mRNA synthetisiert wird. Der erfindungsgemäße Expressionsvektor kann zusätzlich Enhancer-Elemente enthalten, die funktionell auf einen oder mehrere Promotoren wirken. Besonders bevorzugt ist ein Expressionsvektor, bei dem der heterologe Promotor, vorzugsweise der Ubiquitin/S27a-Promotor bzw. eine modifizierte Form hiervon, mit einem Enhancer-Element, z.B. einen SV40-Enhancer oder einem CMV-Enhancer-Element, verknüpft ist.

Grundsätzlich kann die Expression der Gene innerhalb eines Expressionsvektors von einer oder mehreren Transkriptionseinheiten aus erfolgen. Als "Transkriptionseinheit" wird dabei eine Region definiert, die ein oder mehr zu transkribierende Gene enthält. Dabei sind die Gene innerhalb einer Transkriptionseinheit funktionell derart miteinander verknüpft, dass alle Gene innerhalb einer solchen Einheit unter der transkriptionellen Kontrolle desselben Promotors oder Promotors/Enhancers stehen. Als Resultat dieser transkriptionellen Verknüpfung von Genen kann mehr als ein Protein oder Produkt von einer Transkriptionseinheit aus transkribiert und somit exprimiert werden. Jede Transkriptionseinheit enthält dabei die regulatorischen Elemente, die für die Transkription und die Translation der in ihr enthaltenen Gensequenzen erforderlich sind. Jede Transkriptionseinheit kann dabei die gleichen oder verschiedene regulatorische Elemente enthalten. Für die funktionelle Verknüpfung der Gene innerhalb einer Transkriptionseinheit können IRES-Elemente oder Introns verwendet werden.

Der Expressionsvektor kann eine einzige Transkriptionseinheit zur Expression des Gens von Interesse, des modifizierten NPT-Gens und gegebenenfalls des Gens, das für das Fluoreszenzprotein kodiert, enthalten. Alternativ können diese Gene auch in zwei oder mehr Transkriptionseinheiten angeordnet sein. Dabei sind verschiedene Kombinationen der Gene innerhalb einer Transkriptionseinheit möglich. In einer weiteren Ausführungsform der vorliegenden Erfindung kann mehr als ein Expressionsvektor, bestehend aus ein, zwei oder mehr Transkriptionseinheiten, in eine Wirtszelle durch Co-Transfektion oder in aufeinanderfolgenden Transfektion in beliebiger Reihenfolge eingeführt werden. Jede Kombination von regulatorischen Elementen und Genen auf jedem Vektor kann gewählt werden, so lange eine ausreichende Expression der Transkriptionseinheiten gewährleistet ist. Falls erforderlich können weitere regulatorische Elemente und Gene, wie z.B. zusätzliche Gene von Interesse oder Selektionsmarker, auf den Expressionsvektoren positioniert werden.

Demnach kann ein erfindungsgemäßer Expressionsvektor, enthaltend ein Gen von Interesse und ein Gen, das für eine modifizierte Neomycin-Phosphotransferase kodiert, beide Gene in einer oder in zwei separaten Transkriptionseinheiten enthalten. Jede Transkriptionseinheit kann ein oder mehrere Genprodukte transkribieren und exprimieren. Wenn beide Gene in einer Transkriptionseinheit enthalten sind, stehen sie unter der Kontrolle desselben Promotors oder Promotors/Enhancers, wobei vorzugsweise ein IRES-Element verwendet wird, um die funktionelle Verknüpfung aller Komponenten zu gewährleisten. Wenn das Gen, das für modifizierte Neomycin-Phosphotransferase kodiert, und das Gen von Interesse in zwei separaten Transkriptionseinheiten enthalten sind, können sie unter der Kontrolle desselben oder verschiedener Promotoren/Enhancer stehen. Vorzugsweise verwendet man jedoch für das modifizierte NPT-Gen einen schwächeren heterologen Promotor, z.B. den SV40 early Promotor, und setzt vorzugsweise auch keinen Enhancer ein. Expressionsvektoren mit zwei separaten Transkriptionseinheiten sind im Rahmen der Erfindung bevorzugt. Hierbei enthält die eine (bicistronische) Transkriptionseinheit das Gen von Interesse und gegebenenfalls ein für ein fluoreszierendes Protein kodierendes Gen, während die andere Transkriptionseinheit das modifizierte NPT-Gen enthält. Vorzugsweise ist jede Transkriptionseinheit am 3'-Ende durch eine Sequenz begrenzt, die für ein polyA-Signal, vorzugsweise BGH-polyA oder SV40 polyA kodiert.

Erfindungsgemäß sind auch solche Expressionsvektoren, die anstelle des Gens von Interesse lediglich eine multiple Klonierungsstelle aufweisen, die die Klonierung des Gens von Interesse über Erkennungssequenzen für Restriktionsendonukleasen ermöglicht. Im Stand der Technik sind zahlreiche Erkennungssequenzen für die verschiedensten Restriktionsendonukleasen, sowie die hierzu gehörigen Restriktionsendonukleasen bekannt. Bevorzugt werden Sequenzen verwendet, die aus zumindest 6 Nukleotiden als Erkennungssequenz bestehen. Eine Auflistung geeigneter Erkennungssequenzen finden sich beispielsweise in Sambrook et al., (1989).

### Wirtszellen

Zur Transfektion mit dem erfindungsgemäßen Expressionsvektor werden eukaryontische Wirtzellen verwendet, vorzugsweise Säugerzellen und insbesondere Nagerzellen wie z.B. Mäuse-, Ratten- und Hamster-Zelllinien. Die erfolgreiche Transfektion der entsprechenden Zellen mit einem erfindungsgemäßen Expressionsvektor resultiert in transformierten, genetisch modifizierten, rekombinanten oder transgenen Zellen, die ebenfalls Gegenstand der vorliegenden Erfindung sind.

Im Rahmen der Erfindung bevorzugte Wirtszellen sind Hamsterzellen wie z.B. BHK21, BHK TK⁻, CHO, CHO-K1, CHO-DUKX, CHO-DUKX B1 und CHO-DG44 Zellen oder Derivate/Abkömmlinge dieser Zelllinien. Besonders bevorzugt sind CHO-DG44, CHO-DUKX, CHO-K1 und BHK21 Zellen, insbesondere CHO-DG44 und CHO-DUKX Zellen. Ebenfalls geeignet sind Myelomzellen der Maus, vorzugsweise NS0 und Sp2/0 Zellen sowie Derivate/Abkömmlinge dieser Zelllinien.

Beispiele für Hamster- und Mäusezellen, die erfindungsgemäß angewandt werden können, sind in der folgenden Tabelle 1 angegeben. Aber auch Derivate und Abkömmlinge dieser Zellen, andere Säugerzellen, einschließlich aber nicht beschränkt auf Zelllinien von Mensch, Maus, Ratte, Affen, Nagetieren, oder eukaryontische Zellen, einschließlich aber nicht beschränkt auf Hefe-, Insekten- und Pflanzenzellen, können ebenfalls als Wirtszellen zur Produktion von biopharmazeutischen Proteinen verwendet werden.

**Tabelle 1: Hamster- and Mäuse-Produktionszelllinien**

| Zelllinie | Hinterlegungsnummer |
|---|---|
| NS0 | ECACC No. 85110503 |
| Sp2/0-Ag14 | ATCC CRL-1581 |
| BHK21 | ATCC CCL-10 |
| BHK TK⁻ | ECACC No. 85011423 |
| HaK | ATCC CCL-15 |
| 2254-62.2 (BHK-21-Derivat) | ATCC CRL-8544 |
| CHO | ECACC No. 8505302 |
| CHO-K1 | ATCC CCL-61 |
| CHO-DUKX (= CHO duk⁻, CHO/dhf⁻) | ATCC CRL-9096 |
| CHO-DUKX B1 | ATCC CRL-9010 |
| CHO-DG44 | Urlaub et al., Cell 33[2], 405-412, 1983 |
| CHO Pro-5 | ATCC CRL-1781 |
| V79 | ATCC CCC-93 |
| B14AF28-G3 | ATCC CCL-14 |
| CHL | ECACC No. 87111906 |

Die Transfektion der eukaryontischen Wirtszellen mit einem Polynukleotid oder einem der erfindungsgemäßen Expressionsvektor erfolgt nach üblichen Methoden (Sambrook et al., 1989; Ausubel et al., 1994). Geeignete Transfektionsmethoden sind z.B. die Liposomen-vermittelte Transfektion, Calciumphosphat-Copräzipitation, Elektroporation, Polykationen (z.B. DEAE-Dextran)-vermittelte Transfektion, Protoplastenfusion, Mikroinjektion und virale Infektionen. Erfindungsgemäß wird vorzugsweise eine stabile Transfektion durchgeführt, wobei die Konstrukte entweder in das Genom der Wirtszelle oder ein artifizielles Chromosom/Minichromosom integriert werden oder in stabiler Weise episomal in der Wirtszelle enthalten sind. Die Transfektionsmethode, die die optimale Transfektionsfrequenz und Expression des heterologen Gens in der jeweiligen Wirtszelle ermöglicht, ist dabei bevorzugt. Per Definition wird jede Sequenz oder jedes Gen, das in eine Wirtszelle eingebracht wird, in Bezug auf die Wirtszelle als "heterologe Sequenz" oder "heterologes Gen" bezeichnet. Selbst dann, wenn die einzubringende Sequenz oder das einzubringende Gen identisch zu einer endogenen Sequenz oder einem endogenen Gen der Wirtszelle ist. Beispielsweise ist ein Hamster Aktingen, das in eine Hamster-Wirtszelle eingebracht wird, per Definition ein heterologes Gen.

Erfindungsgemäß sind insbesondere rekombinante Säugerzellen, vorzugsweise Nagerzellen, besonders bevorzugt Hamsterzellen, wie z.B. CHO- oder BHK-Zellen, die mit einem der hier beschriebenen erfindungsgemäßen Expressionsvektoren transfiziert wurden.

Bei der rekombinanten Herstellung heteromerer Proteine, wie z.B. monoklonaler Antikörper (mAk), kann die Transfektion geeigneter Wirtszellen prinzipiell auf zwei verschiedenen Wegen erfolgen. Derartige mAk sind aus mehreren Untereinheiten, den schweren und leichten Ketten, aufgebaut. Für diese Untereinheiten kodierende Gene können in unabhängigen oder in multicistronischen Transkriptionseinheiten auf einem einzigen Plasmid untergebracht werden, mit dem dann die Wirtszelle transfiziert wird. Dies soll die stöchiometrische Repräsentanz der Gene nach Integration in das Genom der Wirtszelle sichern. Allerdings muss hierbei im Falle unabhängiger Transkriptionseinheiten sichergestellt werden, dass die mRNAs, die für die verschiedenen Proteine kodieren, die gleiche Stabilität, Transkriptions- und Translationseffizienz aufweisen. Im zweiten Fall erfolgt die Expression der Gene innerhalb einer multicistronischen Transkriptionseinheit durch einen einzigen Promotor und es entsteht nur ein Transkript. Durch Verwendung von IRES-Elementen wird eine recht effiziente interne Translationsinitiation der Gene in dem zweiten und den nachfolgenden Cistrons ermöglicht. Dennoch sind die Expressionsraten für diese Cistrons geringer als die des ersten Cistrons, dessen Translationsinitiation über einen sogenannten "cap"-abhängigen Prä-Initiationskomplex wesentlich effizienter ist als die IRES-abhängige Translationsinitiation. Um eine tatsächlich äquimolare Expression der Cistrons zu erreichen, können beispielsweise noch zusätzliche intercistronische Elemente eingeführt werden, die im Zusammenwirken mit den IRES-Elementen für einheitliche Expressionsraten sorgen (WO 94/05785).

Eine andere und erfindungsgemäß bevorzugte Möglichkeit der simultanen Herstellung mehrerer heterologer Proteine ist die Co-Transfektion, bei der die Gene getrennt in verschiedene Expressionsvektoren integriert werden. Dies hat den Vorteil, dass bestimmte Verhältnisse der Gene und Genprodukte zueinander eingestellt werden können, wodurch Unterschiede in der mRNA-Stabilität sowie in der Transkriptions- und Translationseffizienz ausgeglichen werden können. Außerdem sind die Expressionsvektoren wegen ihrer geringeren Größe stabiler und sowohl bei der Klonierung als auch bei der Transfektion einfacher handhabbar.

In einer besonderen Ausführungsform der Erfindung werden daher die Wirtszellen zusätzlich mit einem oder mehreren Vektoren mit Genen, die für ein oder mehrere andere Proteine von Interesse kodieren, transfiziert, bevorzugt co-transfiziert. Der oder die zur Co-Transfektion verwendeten weiteren Vektoren kodieren z.B. für das oder die anderen Proteine von Interesse unter der Kontrolle der gleichen Promotor/Enhancer-Kombination sowie für zumindest einen weiteren Selektionsmarker, z.B. die Dihydrofolat-Reduktase.

Erfindungsgemäß werden die Wirtszellen vorzugsweise unter serumfreien Bedingungen etabliert, adaptiert und kultiviert, gegebenenfalls in Medien, die frei von tierischen Proteinen /Peptiden sind. Beispiele für im Handel erhältliche Medien sind Ham's F12 (Sigma, Deisenhofen, DE), RPMI-1640 (Sigma), Dulbecco's Modified Eagle's Medium (DMEM; Sigma), Minimal Essential Medium (MEM; Sigma), Iscove's Modified Dulbecco's Medium (IMDM; Sigma), CD-CHO (Invitrogen, Carlsbad, CA, USA), CHO-S-SFMII (Invitrogen), serumfreies CHO-Medium (Sigma) und proteinfreies CHO-Medium (Sigma). Jedes dieser Medien kann gegebenenfalls mit verschiedenen Verbindungen ergänzt werden, z.B. Hormonen und/oder anderen Wachstumsfaktoren (z.B. Insulin, Transferrin, epidermalem Wachstumsfaktor, Insulin-ähnlichem Wachstumsfaktor), Salzen (z.B. Natriumchlorid, Calcium, Magnesium, Phosphat), Puffern (z.B. HEPES), Nukleosiden (z.B. Adenosin, Thymidin), Glutamin, Glukose oder anderen äquivalenten Nährstoffen, Antibiotika und/oder Spurenelementen. Obwohl erfindungsgemäß serumfreie Medien bevorzugt sind, können zur Züchtung der Wirtszellen und zur späterern Proteinproduktion auch Medien verwendet werden, die mit einer geeigneten Menge an Serum versetzt wurden. Zur Selektion von genetisch modifizierten Zellen, die ein oder mehrere Selektionsmarkergene exprimieren, wird dem Medium ein oder mehrere geeignete Selektionsmittel zugefügt.

Als "Selektionsmittel" wird eine Substanz bezeichnet, die das Wachstum oder das Überleben von Wirtszellen mit einer Defizienz für das jeweilige Selektionsmarkergen beeinträchtigt. Im Rahmen der vorliegenden Erfindung wird vorzugsweise Geneticin (G418) als Mediumzusatz zur Selektion heterologer Wirtszellen, die ein modifiziertes Neomycin-Phosphotransferase-Gen tragen, verwendet. Vorzugsweise werden G418-Konzentrationen zwischen 100 und 800 µg/ml Medium verwendet, besonders bevorzugt sind 300 bis 400 µg/ml Medium. Sollten die Wirtzellen mit mehreren Expressionsvektoren transfiziert werden, z.B. wenn separat mehrere Gene von Interesse in die Wirtszelle eingebracht werden sollen, so verfügen diese meist über verschiedene Selektionsmarkergene. Ein "Selektionsmarkergen" ist ein Gen, das die spezifische Selektion von Zellen, die dieses Gen erhalten, durch Zugabe eines entsprechenden Selektionsmittels in das Kultivierungsmedium ermöglicht. Zur Verdeutlichung, ein Antibiotika-Resistenzgen kann als positiver Selektionsmarker verwendet werden. Nur Zellen, die mit diesem Gen transformiert wurden, können in der Gegenwart des entsprechenden Antibiotikums wachsen und somit selektioniert werden. Nichttransfomierte Zellen hingegen können unter diesen Selektionsbedingungen nicht wachsen oder überleben. Es gibt positive, negative und bifunktionale Selektionsmarker. Positive Selektionsmarker ermöglichen die Selektion und damit Anreicherung von transformierten Zellen durch Vermittlung einer Resistenz gegenüber dem Selektionsmittel oder durch Kompensierung eines metabolischen oder katabolischen Defekts der Wirtszelle. Im Gegensatz dazu können durch negative Selektionsmarker Zellen, die das Gen für den Selektionsmarker erhalten haben, selektiv eliminiert werden. Ein Beispiel hierfür ist das Thymidinkinasegen des Herpes Simplex Virus, dessen Expression in Zellen bei gleichzeitiger Gabe von Acyclovir oder Gancyclovir zu deren Elimination führt. Die in dieser Erfindung verwendeten Selektionsmarker, einschließlich der amplifzierbaren Selektionsmarker, schließt gentechnologisch veränderte Mutanten und Varianten, Fragmente, funktionelle Äquivalente, Derivate, Homologe und Fusionen mit anderen Proteinen oder Peptiden ein, solange der Selektionsmarker seine selektiven Eigenschaften beibehält. Solche Derivate weisen eine beträchtliche Homologie in der Aminosäuresequenz in den Bereichen oder Domänen auf, denen die selektive Eigenschaft zugeschrieben wird. In der Literatur sind eine Vielzahl von Selektionsmarkergenen, einschließlich bifunktionaler (positiv/negativ) Marker, beschrieben (siehe z.B. WO 92/08796 und WO 94/28143). Beispiele für Selektionsmarker, die für gewöhnlich in eukaryontischen Zellen verwendet werden, beinhalten die Gene für Aminoglykosid-Phosphotransferase (APH), Hygromycin-Phosphotransferase (HYG), Dihydrofolat-Reduktase (DHFR), Thymidinkinase (TK), Glutamin-Synthetase, Asparagin-Synthetase und Gene, die Resistenz gegenüber Neomycin (G418), Puromycin, Histidinol D, Bleomycin, Phleomycin und Zeocin vermitteln.

Eine Selektion von transformierten Zellen ist auch durch fluoreszenzaktivierte Zellsortierung (FACS) möglich. Hierzu werden beispielsweise bakterielle β-Galaktosidase, Zelloberflächenmarker oder fluoreszierende Proteine (z.B. grünes fluoreszierendes Protein (GFP) und dessen Varianten von *Aequorea victoria* und *Renilla reniformis* oder anderer Spezies; rote fluoreszierende Proteine und in anderen Farben fluoreszierende Proteine und deren Varianten von nicht-biolumineszierenden Organismen wie z.B. *Discosoma sp., Anemonia sp., Clavularia sp., Zoanthus sp.)* zur Selektion von transformierten Zellen eingesetzt.

### Genexpression und Selektion hoch produzierender Wirtszellen

Der Ausdruck "Genexpression" bezieht sich auf die Transkription und/oder Translation einer heterologen Gensequenz in einer Wirtszelle. Die Expressionsrate kann hierbei allgemein bestimmt werden, entweder auf Basis der Menge der entsprechenden mRNA, die in der Wirtszelle vorhanden ist, oder auf Basis der produzierten Menge an Genprodukt, das von dem Gen von Interesse kodiert wird. Die Menge der durch Transkription einer ausgewählten Nukleotidsequenz erzeugten mRNA kann beispielsweise durch Northern Blot Hybridisierung, Ribonuklease-RNA-Protektion, *in situ* Hybridisierung von zellulärer RNA oder durch PCR-Methoden bestimmt werden (Sambrook et al., 1989; Ausubel et al., 1994). Proteine, die von einer ausgewählten Nukleotidsequenz kodiert werden, können ebenfalls durch verschiedene Methoden, wie z.B. durch ELISA, Western Blot, Radioimmunassay, Immunpräzipitation, Nachweis der biologischen Aktivität des Proteins oder durch Immunfärbung des Proteins mit nachfolgender FACS-Analyse, bestimmt werden (Sambrook et al., 1989; Ausubel et al., 1994).

Mit "hohem Expressionslevel (-rate)", "hoher Expression", "verstärkter Expression" oder "hoher Produktivität" wird die anhaltende und ausreichend hohe Expression oder Synthese einer in eine Wirtszelle eingebrachten heterologen Sequenz, beispielsweise eines für ein therapeutisches Protein kodierenden Gens, bezeichnet. Eine verstärkte oder hohe Expression bzw. ein(e) hohe(r) Expressionslevel (-rate) oder eine hohe Produktivität liegt vor, wenn eine erfindungsgemäße Zelle nach einem hier beschriebenen erfindungsgemäßen Verfahren ohne Genamplifikation kultiviert wird, und wenn diese Zelle zumindest mehr als ungefähr 0,5 pg des gewünschten Genprodukts pro Tag produziert (0,5 pg/Zelle/Tag). Eine verstärkte oder hohe Expression bzw. ein(e) hohe(r) Expressionslevel (-rate) oder eine hohe Produktivität liegt auch dann vor, wenn die erfindungsgemäße Zelle ohne vorherige Genamplifikation zumindest mehr als ungefähr 1,0 pg des gewünschten Genprodukts pro Tag produziert (1,0 pg/Zelle/Tag). Eine verstärkte oder hohe Expression bzw. ein(e) hohe(r) Expressionslevel (-rate) oder eine hohe Produktivität liegt insbesondere auch vor, wenn die erfindungsgemäße Zelle ohne vorherige Genamplifikation zumindest mehr als ungefähr 1,5 pg des gewünschten Genprodukts pro Tag produziert (1,5 pg/Zelle/Tag). Eine verstärkte oder hohe Expression bzw. ein(e) hohe(r) Expressionslevel (-rate) oder eine hohe Produktivität liegt insbesondere dann vor, wenn die erfindungsgemäße Zelle ohne vorherige Genamplifikation zumindest mehr als ungefähr 2,0 pg des gewünschten Genprodukts pro Tag produziert (2,0 pg/Zelle/Tag). Eine besonders verstärkte oder hohe Expression bzw. ein(e) besonders hohe(r) Expressionslevel (-rate) oder eine besonders hohe Produktivität liegt dann vor, wenn die erfindungsgemäße Zelle ohne vorherige Genamplifikation zumindest mehr als ungefähr 3,0 pg des gewünschten Genprodukts pro Tag produziert (3,0 pg/Zelle/Tag). Durch einen einfachen Genamplifikationsschritt, z.B. mit Hilfe des DHFR/MTX-Amplifikationssystems, wie nachfolgend beschrieben, lassen sich die Produktivitäten um zumindest einen Faktor 2 bis 10 steigern, so dass man in Bezug auf eine Zelle, die einem Genamplifikationsschritt unterzogen wurde, von einer "hohen Expression", "verstärkten Expression" oder "hohen Produktivität" spricht, wenn diese Zelle zumindest mehr als ungefähr 5 pg des gewünschten Genprodukts pro Tag produziert (5 pg/Zelle/Tag), vorzugsweise zumindest mehr als ungefähr 10 pg/Zelle/Tag, besonders bevorzugt zumindest mehr als ungefähr 15 pg/Zelle/Tag, noch weiter bevorzugt zumindest mehr als ungefähr 20 pg/Zelle/Tag, bzw. zumindest mehr als ungefähr 30 pg/Zelle/Tag.

Eine hohe oder verstärkte Expression, eine hohe Produktivität oder ein(e) hohe(r) Expressionslevel (-rate) kann sowohl durch Verwendung einer der erfindungsgemäßen Expressionsvektoren als auch durch Anwendung eines der erfindungsgemäßen Verfahren erreicht werden.

Beispielsweise können durch die Co-Expression des Gens von Interesse und eines modifizierten NPT-Gens Zellen selektioniert und identifiziert werden, die das heterologe Gen in hohem Maße exprimieren. Die modifizierte NPT ermöglicht im Vergleich zur wtNPT eine effizientere Selektion von stabil transfizierten Wirtszellen mit hoher Expression des heterologen Gens von Interesse.

Gegenstand der Erfindung ist somit auch ein Verfahren zur Expression von mindestens einem Gen von Interesse in rekombinanten Säugerzellen, dadurch gekennzeichnet, dass (i) ein Pool von Säugerzellen mit mindestens einem Gen von Interesse und einem Gen für eine modifizierte Neomycin-Phosphotransferase transfiziert wird, die im Vergleich zur Wildtyp-Neomycin-Phosphotransferase lediglich 1 bis 80% der Aktivität aufweist, vorzugsweise lediglich 1 bis 60%, weiter bevorzugt lediglich 1,5 bis 30%, im Besonderen 1,5% bis 26%; (ii) die Zellen unter Bedingungen kultiviert werden, die eine Expression des (der) Gens (Gene) von Interesse und des modifizierten Neomycin-Phosphotransferase-Gens erlauben; (iii) die Säugerzellen in Anwesenheit zumindest eines Selektionsmittels kultiviert werden, vorzugsweise G418, das selektiv auf das Wachstum der Säugerzellen wirkt, und solche Zellen im Wachstum bevorzugt, die das modifizierte Neomycin-Phosphotransferase-Gen exprimieren; und (iv) das (die) Protein(e) von Interesse aus den Säugerzellen oder dem Kulturüberstand gewonnen wird (werden). Vorzugsweise werden hierbei rekombinante Säugerzellen verwendet, die mit einem erfindungsgemäßen Expressionsvektor transfiziert worden sind.

Ferner ist auch ein Verfahren zur Selektion von rekombinanten Säugerzellen Gegenstand der Erfindung, die mindestens ein Gen von Interesse exprimieren, wobei (i) ein Pool von Säugerzellen mit mindestens einem Gen von Interesse und einem Gen für eine modifizierte Neomycin-Phosphotransferase transfiziert wird, die im Vergleich zur Wildtyp-Neomycin-Phosphotransferase lediglich 1 bis 80% der Aktivität aufweist, vorzugsweise lediglich 1 bis 60%, weiter bevorzugt lediglich 1,5 bis 30%, im Besonderen 1,5% bis 26%; (ii) die Säugerzellen unter Bedingungen kultiviert werden, die eine Expression des (der) Gens(Gene) von Interesse und des modifizierten Neomycin-Phosphotransferase-Gens erlauben; und (iii) die Säugerzellen in Anwesenheit zumindest eines Selektionsmittels kultiviert werden, vorzugsweise G418, das selektiv auf das Wachstum der Säugerzellen wirkt, und solche Zellen im Wachstum bevorzugt, die das modifizierte Neomycin-Phosphotransferase-Gen exprimieren.

Besonders bevorzugt sind Verfahren zur Expression von zumindest einem Gen von Interesse und zur Selektion von rekombinanten Zellen, die ein entsprechendes Gen von Interesse exprimieren, wenn ein in dieser Anmeldung näher beschriebenes modifiziertes NPT-Gen verwendet wird, insbesondere wenn ein modifiziertes NPT-Gen verwendet wird, das im Vergleich zum Wildtyp-Gen an Aminosäureposition 182 für Glycin oder Asparaginsäure, an Aminosäureposition 91 für Alanin, an Aminosäureposition 198 für Glycin, an Aminosäureposition 227 für Alanin, Glycin oder Valin, an Aminsäureposition 261 für Glycin oder Asparagin oder an Aminosäureposition 240 für Isoleucin kodiert. Im Besonderen bevorzugt ist die Verwendung der Asp227Val-, Asp227Gly-, Asp261Gly-, Asp261Asn-, Phe240lle- oder Trp91Ala-Mutante. Generell eignen sich für ein entsprechendes Verfahren alle in dieser Patentschrift erwähnten, modifizierten erfindungsgemäßen Neomycin-Phosphotransferase-Gene. Zu den bevorzugten Neomycin-Phosphotransferase-Genen siehe unter dem Abschnitt - modifizierte Neomycin-Phosphotransferase-Gene.

Die Selektion der Zellen, die ein Gen von Interesse und ein modifiziertes NPT-Gen exprimieren, erfolgt z.B. durch Zugabe von G418 als Selektionsmittel. Möglich ist allerdings auch die Verwendung von anderen aminoglykosidischen Antibiotika wie z.B. Neomycin oder Kanamycin. Bevorzugt ist hierbei die Kultivierung und Selektion der erfindungsgemäßen Zellen in 200 bis 800 µg G418 pro mL Kulturmedium. Als besonders bevorzugt hat sich die Zugabe von 300 bis 700 µg G418 pro mL Kulturmedium herausgestellt. Die Zugabe von ungefähr 400 µg G418 pro mL Kulturmedium stellt die am meisten bevorzugte Ausführungsform dar. Mit einem entsprechenden Verfahren konnten rekombinante Zellen mit besonders hoher Expressionsrate selektioniert werden. Im Vergleich zur Verwendung der wtNPT als Selektionsmarker wiesen die Zellen nach Selektion mit 400 µg G418 pro mL Kulturmedium im Fall der Glu182Gly-, Glu182Asp- und Val198Gly-Mutante eine um Faktor 1,4-2,4, im Fall der Asp227Gly-Mutante eine um Faktor 1,6-4,1, im Fall der Asp227Ala- bzw. Trp91Ala-Mutante eine um Faktor 2,2 bzw. 4, im Fall der Phe240lle- bzw. Asp261Asn-Mutante eine um Faktor 5,7 bzw. 7,3 und im Fall der Asp261Gly- bzw. Asp227Val-Mutante eine sogar um Faktor 9,3 bzw. 14,6 erhöhte Produktivität auf. Die spezifischen Produktivitäten für die jeweiligen modifizierten NPT-Gene sind in Abb.6 dargestellt.

Die entsprechenden Verfahren können mit einer durch FACS gestützten Selektion von rekombinanten Wirtszellen, die als weiteren Selektionsmarker beispielsweise ein (oder mehrere) Fluoreszenzprotein(e) (z.B. GFP) oder einen Zelloberflächenmarker enthalten, kombiniert werden. Andere Methoden zur Erzielung einer verstärkten Expression, wobei auch eine Kombination verschiedener Methoden möglich ist, beruhen beispielsweise auf der Verwendung von (artifiziellen) Transkriptionsfaktoren, Behandlung der Zellen mit natürlichen oder synthetischen Agenzien zur Hochregulation endogener oder heterologer Genexpression, Verbesserung der Stabilität (Halbwertszeit) der mRNA oder des Proteins, Verbesserung der mRNA-Translationsinitiation, Erhöhung der Gendosis durch Verwendung von episomalen Plasmiden (basierend auf der Verwendung von viralen Sequenzen als Replikationsursprung, z.B. von SV40, Polyoma, Adenovirus, EBV oder BPV), Einsatz von amplifikationsfördernden Sequenzen (Hemann et al., 1994) oder auf DNA-Konkatemeren basierenden *in vitro* Amplifikationssystemen (Monaco et al., 1996).

Eine gekoppelte Transkription des Gens von Interesse und des Gens, das für das fluoreszierende Protein kodiert, hat sich als besonders effektiv im Zusammenhang mit der Verwendung eines modifizierten NPT-Gens als Selektionsmarker herausgestellt. Von der resultierenden bicistronischen mRNA werden sowohl das Protein/Produkt von Interesse als auch das fluoreszierende Protein exprimiert. Aufgrund dieser Kopplung der Expression des Proteins von Interesse und des fluoreszierenden Proteins ist es erfindungsgemäß leicht möglich, hochproduzierende rekombinante Wirtszellen über das exprimierte fluoreszierende Protein zu identifizieren und zu isolieren, z.B. durch Sortieren mit Hilfe eines Fluoreszenz-aktivierten Zellsortiergeräts (FACS).

Die Selektion von rekombinanten Wirtszellen, die eine hohe Vitalität und eine erhöhte Expressionsrate des gewünschten Genprodukts zeigen, ist ein mehrstufiger Prozess. Die mit dem erfindungsgemäßen Expressionsvektor transfizierten oder gegebenenfalls einem weiteren Vektor z.B. co-transfizierten Wirtszellen werden unter Bedingungen kultiviert, die eine Selektion der Zellen erlauben, die die modifizierte NPT exprimieren, z.B. durch Kultivierung in Gegenwart eines Selektionsmittel wie etwa G418 in Konzentrationen von 100, 200, 400, 600, 800µg oder mehr G418/mL Kulturmedium. Anschließend werden die entsprechenden Zellen zumindest auf die Expression des mit dem Gen von Interesse gekoppelten Gens, das für ein fluoreszierendes Protein kodiert, untersucht, um die Zellen/Zellpopulation zu identifizieren und herauszusortieren, die die höchsten Expressionsraten an fluoreszierendem Protein zeigen. Vorzugsweise werden nur die Zellen aussortiert und weiter kultiviert, die zu den 10 - 20% Zellen mit der höchsten Expressionsrate an fluoreszierendem Protein gehören. In der Praxis bedeutet dies, dass die hellsten 10% der fluoreszierenden Zellen heraussortiert und weiter kultiviert werden. Entsprechend können auch die hellsten 5%, vorzugsweise die hellsten 3%, oder auch nur die hellsten 1% der fluoreszierenden Zellen eines Zellgemisches heraussortiert und vermehrt werden. In einer besonders bevorzugten Ausführungsform werden lediglich die hellsten 0,5% bzw. die hellsten 0,1% der fluoreszierenden Zellen heraussortiert und vermehrt.

Der Selektionsschritt kann an Zellpools oder mit bereits vorsortierten Zellpools/ Zellklonen durchgeführt werden. Es können ein oder mehrere, vorzugsweise zwei oder mehr und insbesondere drei oder mehr Sortierungsschritte durchgeführt werden, wobei zwischen den einzelnen Sortierungsschritten die Zellen über einen bestimmten Zeitraum, z.B. etwa zwei Wochen bei Pools, kultiviert und vermehrt werden. In den Abbildungen 11 und 12 sind spezifische Produktivitäten nach FACSbasiertem Sortieren mit und ohne Genamplifiationsschritt beispielhaft für die Mutante Asp227Gly dargestellt. Gegenstand der Erfindung ist somit auch ein Verfahren zur Gewinnung und Selektion von rekombinanten Säugerzellen, die mindestens ein heterologes Gen von Interesse exprimieren, dadurch gekennzeichnet, dass (i) rekombinante Säugerzellen mit einem erfindungsgemäßen Expressionsvektor transfiziert werden; (ii) die transfizierten Zellen unter Bedingungen kultiviert werden, die eine Expression des (der) Gens (e) von Interesse, des Gens, das für ein fluoreszierendes Protein kodiert, und des modifizierten Neomycin-Phosphotransferase-Gens ermöglicht; (iii) die Säugerzellen in Anwesenheit zumindest eines Selektionsmittels kultiviert werden, das selektiv auf das Wachstum von Säugerzellen wirkt, und solche Zellen im Wachstum bevorzugt, die das modifizierte Neomycin-Phosphotransferase-Gen exprimieren; und (iv) die Säugerzellen mittels flowzytometrischer Analyse aussortiert werden, die eine besonders hohe Expression des fluoreszierenden Gens zeigen. Optional können die Schritte ii) - iv) mit den nach Schritt iv) gewonnenen Zellen einfach oder mehrfach wiederholt werden.

Bevorzugt ist ein entsprechendes Verfahren, das dadurch gekennzeichnet ist, dass die aussortierten Säugerzellen eine durchschnittliche spezifische Produktivität ohne zusätzlichen Genamplifikationsschritt von größer 0,5pg des (der) gewünschten Genprodukts (Genprodukte) pro Tag und Zelle (0,5 pg/Zelle/Tag), vorzugsweise von größer 1 pg/Zelle/Tag, besonders bevorzugt von größer 2 pg/Zelle/Tag, weiter bevorzugt von größer 3 pg/Zelle/Tag, noch weiter bevorzugt von größer 4,pg/Zelle/Tag, beispielsweise von größer 5, 6, 7, 8, 9, 10 usw., von größer 15, 20, 25 pg/Zelle/Tag usw. besitzen. Wie oben erwähnt, lässt sich die Produktivität dieser Zellen durch einen einfachen Genamplifikationsschritt, beispielsweise unter Verwendung des DHFR/MTX-Systems um zumindest einen Faktor 2 bis 10 steigern. Beispielhaft ist dies in Abbildung 12 für die Selektion mit Hilfe der NTP-Mutante Asp227Gly dargestellt. Die spezifischen Produktivitäten lagen zwischen 20 und 25 pg/Zelle/Tag.

Weiterhin erfindungsgemäß ist ein Verfahren, bei dem entsprechend aussortierte Zellen vermehrt werden und zur Herstellung des kodierenden Genprodukt von Interesse verwendet werden. Hierzu werden die selektionierten hoch-produzierenden Zellen vorzugsweise in einem serumfreien Kulturmedium und vorzugsweise in Suspensionskultur unter Bedingungen gezüchtet, die eine Expression des Gens von Interesse erlauben. Das Protein/Produkt von Interesse wird dabei vorzugsweise als sekretiertes Genprodukt aus dem Zellkulturmedium gewonnen. Bei Expression des Proteins ohne Sekretionssignal kann das Genprodukt aber auch aus Zelllysaten isoliert werden. Um ein reines, homogenes Produkt zu erhalten, das im Wesentlichen frei ist von anderen rekombinanten Proteinen und Wirtszellproteinen, werden übliche Reinigungsschritte durchgeführt. Hierzu entfernt man häufig zunächst Zellen und Zelltrümmer aus dem Kulturmedium oder Lysat. Das gewünschte Genprodukt kann dann von kontaminierenden löslichen Proteinen, Polypeptiden und Nukleinsäuren befreit werden, z.B. durch Fraktionierung an Immunaffinitäts- und lonenaustauschsäulen, Ethanolfällung, Umkehrphasen-HPLC oder Chromatographie an Sephadex, Silica oder Kationenaustauscherharzen wie DEAE. Methoden, die zur Aufreinigung eines von rekombinanten Wirtszellen exprimierten heterologen Proteins führen, sind dem Fachmann bekannt und sind in der Literatur beschrieben, z.B. bei Harris et al. (1995) und Scopes (1988).

### Amplifizierbares Selektionsmarkergen

Ferner können die erfindungsgemäßen Zellen optional auch einem oder mehreren Genamplifikationsschritten unterzogen werden, in dem sie in Gegenwart eines Selektionsmittels kultiviert werden, das zu einer Amplifikation eines amplifzierbaren Selektionsmarkergens führt. Dieser Schritt kann sowohl mit Zellen erfolgen, die ein fluoreszentes Protein exprimieren und vorzugsweise mittels FACS einfach oder mehrfach sortiert wurden (vorzugsweise in einer der hier beschriebenen Art und Weise) als auch mit nicht sortierten Zellen.

Voraussetzung ist, dass die Wirtszellen zusätzlich mit einem Gen transfiziert werden, das für einen amplifizierbaren Selektionsmarker kodiert. Denkbar ist, dass das Gen, welches für einen amplifizierbaren Selektionsmarker kodiert, auf einem der erfindungsgemäßen Expressionsvektoren vorhanden ist oder aber mit Hilfe eines weiteren Vektors in die Wirtszelle eingebracht wird. Das amplifizierbare Selektionsmarkergen kodiert gewöhnlich für ein Enzym, das für das Wachstum von eukaryontischen Zellen unter bestimmten Kultivierungsbedingungen erforderlich ist. Beispielsweise kann das amplifizierbare Selektionsmarkergen für Dihydrofolat-Reduktase (DHFR) kodieren. In diesem Fall wird das Gen amplifiziert, wenn man eine damit transfizierte Wirtszelle in Gegenwart des Selektionsmittels Methotrexat (MTX) kultiviert.

In der folgenden Tabelle 2 sind Beispiele für weitere erfindungsgemäß verwendbare amplifizierbare Selektionsmarkergene und die dazugehörigen Selektionsmittel angegeben, die in einem Überblick bei Kaufman, Methods in Enzymology, 185:537-566 (1990) beschrieben sind.

**Tabelle 2: Amplifizierbare Selektionsmarkergene**

| | | |
|---|---|---|
| Amplifizierbares Selektionsmarkergen | Hinterlegungsnummer | Selektionsmittel |
| Dihydrofolat-Reduktase | M19869 (Hamster) E00236 (Maus) | Methotrexat (MTX) |
| Metallothionein | D10551 (Hamster) M13003 (Human) M 11794 (Ratte) | Cadmium |
| CAD (Carbamoylphosphat-Synthetase : Aspartat-Transcarbamylase: Dihydroorotase) | M23652 (Hamster) D78586 (Human) | N-Phosphoacetyl-L-aspartat |
| Adenosin-Deaminase | K02567 (Human) M10319 (Maus) | Xyl-A- oder Adenosin, 2' Deoxycoformycin |
| AMP (Adenylat)-Deaminase | D12775 (Human) J02811 (Ratte) | Adenin, Azaserin, Coformycin |
| UMP-Synthase | J03626 (Human) | 6-Azauridin, Pyrazofuran |
| IMP 5'-Dehydrogenase | J04209 (Hamster) J04208 (Human) M33934 (Maus) | Mycophenolsäure |
| Xanthin-Guanin-Phosphoribosyltransferase | X00221 (E. coli) | Mycophenolsäure mit limitierendem Xanthin |
| Mutanten-HGPRTase oder Mutanten-Thymidin-Kinase | J00060 (Hamster) M13542, K02581 (Human) J00423, M68489(Maus) M63983 (Ratte) M36160 (Herpes virus) | Hypoxanthin, Aminopterin, und Thymidin (HAT) |
| Thymidylat-Synthetase | D00596 (Human) M13019 (Maus) L12138 (Ratte) | 5-Fluordeoxyuridin |
| P-Glycoprotein 170 (MDR1) | AF016535 (Human) J03398 (Maus) | mehrere Arzneistoffe, z.B. Adriamycin, Vincristin, Colchicin |
| Ribonukleotid-Reduktase | M124223, K02927 (Maus) | Aphidicolin |
| Glutamin-Synthetase | AF150961 (Hamster) U09114, M60803 (Maus) M29579 (Ratte) | Methioninsulfoximin (MSX) |
| Asparagin-Synthetase | M27838 (Hamster) M27396 (Human) U38940 (Maus) U07202 (Ratte) | β-Aspartylhydroxamat, Albizziin, 5'Azacytidin |
| Argininosuccinat-Synthetase | X01630 (Human) | Canavanin |
| | M31690 (Maus) | |
| | M26198 (Rind) | |
| Ornithin-Decarboxylase | M34158 (Human) | α-Difluormethylornithin |
| | J03733 (Maus) | |
| | M 16982 (Ratte) | |
| HMG-CoA-Reduktase | L00183,M12705 (Hamster) | Compactin |
| | M11058 (Human) | |
| N-Acetylglucosaminyl-Transferase | M55621 (Human) | Tunicamycin |
| Threonyl-tRNA-Synthetase | M63180 (Human) | Borrelidin |
| Na⁺K⁺-ATPase | J05096 (Human) | Ouabain |
| | M14511 (Ratte) | |

Als amplifizierbares Selektionsmarkergen wird erfindungsgemäß ein Gen bevorzugt, das für ein Polypeptid mit der Funktion von DHFR kodiert, z.B. für DHFR oder ein Fusionsprotein aus dem fluoreszierenden Protein und DHFR. DHFR ist für die Biosynthese von Purinen erforderlich. Zellen, denen das DHFR-Gen fehlt, können in Purin-defizientem Medium nicht wachsen. Das DHFR-Gen ist deshalb ein nützlicher Selektionsmarker zur Selektion und Amplifikation von Genen in Zellen, die in Purin-freiem Medium kultiviert werden. Das Selektionsmittel, das in Verbindung mit dem DHFR-Gen verwendet wird, ist Methotrexat (MTX).

Die vorliegende Erfindung schließt deshalb eine Methode zur Herstellung und Selektion von rekombinanten Säugerzellen ein, die folgende Schritte enthält: (i) Transfektion der Wirtszellen mit Genen, die zumindest für ein Protein/Produkt von Interesse, eine modifizierte Neomycin-Phosphotransferase und DHFR kodieren; (ii) Kultivierung der Zellen unter Bedingungen, die eine Expression der verschiedenen Gene ermöglichen; und (iii) die Amplifikation dieser co-integrierten Gene durch Kultivierung der Zellen in Gegenwart eines Selektionsmittels, das die Amplifikation zumindest des amplifizierbaren Selektionsmarkergens erlaubt, wie z.B. Methotrexat. Bevorzugt werden die transfizierten Zellen hierbei in Hypoxanthin/Thymidin-freiem Medium in der Abwesenheit von Serum und unter Zugabe steigender Konzentrationen an MTX kultiviert. Vorzugsweise beträgt die Konzentration an MTX bei dem ersten Amplifikationsschritt zumindest 5 nM. Die Konzentration an MTX kann aber auch zumindest 20 nM oder 100 nM betragen und stufenweise auf bis zu 1 µM gesteigert werden. Im Einzelfall können auch Konzentration von über 1 µM verwendet werden, z.B. 2 µM.

Sind die entsprechenden Zellen zusätzlich mit einem Gen für ein fluoreszentes Protein transformiert, lassen sich diese Zellen mit Hilfe eines Fluoreszenz-aktivierten Zellsortiergeräts (FACS) identifizieren und aussortieren, und anschließend in einem Genamplifikationsschritt in Gegenwart von mindestens 20, vorzugsweise in Gegenwart von 50 oder 100 nM MTX kultivieren und amplifizieren. Hierdurch kann eine erhebliche Steigerung der Produktivitäten auf über 20pg Genprodukt pro Zelle und Tag, vorzugsweise auf über 21, 22 ,23, 24, 25, usw., 30, 35, 40, usw, erzielt werden. Die Wirtszellen kann man einem oder mehreren Genamplifikationsschritten unterziehen, um die Kopienzahl zumindest des Gens von Interesse und des amplifizierbaren Selektionsmarkergens zu erhöhen. Erfindungsgemäß ist die erzielbare hohe Produktivität an eine effektive Vorselektion über eine Neomycin-Phosphotransferase-vermittelte Resistenz gegenüber aminoglykosidischen Antibiotika wie Neomycin, Kanamycin und G418 gebunden. Es ist daher möglich, die Anzahl der erforderlichen Genamplifikationsschritte zu reduzieren und z.B. nur eine einzige Genamplifikation durchzuführen.

In einer weiteren Ausführung betrifft die vorliegende Erfindung somit auch Verfahren zur Gewinnung und Selektion von rekombinanten Säugerzellen, die mindestens ein heterologes Gen von Interesse exprimieren und dadurch gekennzeichnet sind, dass (i) rekombinante Säugerzellen mit einem erfindungsgemäßen Expressionsvektor sowie dem Gen für ein amplifizierbares Selektionsmarkergen transfiziert werden; (ii) die Säugerzellen unter Bedingungen kultiviert werden, die eine Expression des (der) Gens (e) von Interesse, des modifizierten Neomycin-Phosphotransferase-Gens und des Gens, das für ein fluoreszierendes Protein kodiert, ermöglicht; (iii) die Säugerzellen in Anwesenheit zumindest eines Selektionsmittels kultiviert werden, der selektiv auf das Wachstum von Säugerzellen wirkt, und solche Zellen im Wachstum bevorzugt, die das Neomycin-Phosphotransferase-Gen exprimieren; (iv) die Säugerzellen mittels flowzytometrischer Analyse aussortiert werden, die eine hohe Expression des fluoreszierenden Proteins zeigen; (v) die aussortierten Zellen unter Bedingungen kultiviert werden, unter denen das amplifizierbare Selektionsmarkergen exprimiert wird; und (vi) dem Kulturmedium ein Selektionsmittel zugegeben wird, das die Amplifikation des amplifizierbaren Selektionsmarkergens zur Folge hat.

Besonders bevorzugt ist ein entsprechendes Verfahren, sofern die in dieser Erfindung beschriebenen modifizierten Neomycin-Phosphotransferase-Gene verwendet werden. Weiterhin bevorzugt ist ein Verfahren, bei dem lediglich ein Amplifikationsschritt durchgeführt wird. Weiterhin bevorzugt ist ein entsprechendes Verfahren, dass zu rekombinanten Säugerzellen führt, die eine durchschnittliche spezifische Produktivität von größer 20pg, vorzugsweise von über 21, 22, 23, 24, 25, usw., 30, 35, 40, usw, des (der) gewünschten Genprodukts (Genprodukte) pro Zelle und Tag zeigen.

Säugerzellen, vorzugsweise Mausmyeloma- und Hamsterzellen, sind bevorzugte Wirtszellen für den Einsatz von DHFR als amplifizierbaren Selektionsmarker. Besonders bevorzugt sind die Zelllinien CHO-DUKX (ATCC CRL-9096) und CHO-DG44 (Urlaub et al., 1983), da sie bedingt durch Mutation keine eigene DHFR-Aktivität aufweisen. Um die DHFR-bedingte Amplifikation auch in anderen Zelltypen anwenden zu können, die über eine eigene endogene DHFR-Aktivität verfügen, kann bei der Transfektion ein mutiertes DHFR-Gen verwendet werden, das für ein Protein mit einer reduzierten Sensitivität gegenüber Methotrexat kodiert (Simonson et al., 1983; Wigler et al., 1980; Haber et al., 1982).

Der DHFR-Marker ist bei der Verwendung von DHFR-negativen Grundzellen wie CHO-DG44 oder CHO-DUKX besonders gut zur Selektion und nachfolgenden Amplifikation geeignet, da diese Zellen kein endogenes DHFR exprimieren und somit nicht in Purin-freiem Medium wachsen. Deshalb kann hier das DHFR-Gen als dominanter Selektionsmarker eingesetzt werden und die transformierten Zellen werden in Hypoxanthin/Thymidin-freiem Medium selektioniert.

Im folgenden wird die Erfindung anhand nicht-beschränkender Ausführungsbeispiele näher erläutert.

### BEISPIELE

### Abkürzungen

- Ala (=A):: Alanin
- AP:: alkalische Phosphatase
- Asn (=N):: Asparagin
- Asp (=D):: Asparaginsäure
- bp:: Basenpaar
- BSA:: Rinderserumalbumin
- CHO:: Chinese Hamster Ovary
- dhfr:: Dihydrofolat-Reduktase
- DMSO:: Dimethylsulfoxid
- ELISA:: enzyme-linked immunosorbant assay
- FACS:: fluorescence-activated cell sorter
- FITC:: Fluorescein-lsothiocyanat
- GFP:: grünes fluoreszierendes Protein
- Glu (=E):: Glutaminsäure
- Gly (=G):: Glycin
- HBSS:: Hanks Balanced Salt Solution
- HT:: Hypoxanthin/Thymidin
- Ile (=l):: Isoleucin
- IRES:: internal ribosomal entry site
- kb:: Kilobase
- mAk:: monoklonaler Antikörper
- MCP-1:: monocyte chemoattractant protein-1
- MTX:: Methotrexat
- MW:: Mittelwert
- NPT:: Neomycin-Phosphotransferase
- PCR:: polymerase chain reaction
- PBS:: phosphate buffered saline
- Phe (=F):: Phenylalanin
- Trp (=W):: Tryptophan
- Val (=V):: Valin
- WT:: Wildtyp

### Methoden

### 1. Zellkultur und Transfektion

Die Zellen CHO-DG44/dhfr^{-/-} (Urlaub et al., 1983) wurden permanent als Suspensionszellen in serum-freiem und mit Hypoxanthin und Thymidin supplementiertem CHO-S-SFMII Medium (Invitrogen GmbH, Karlsruhe, DE) in Zellkulturflaschen bei 37°C in feuchter Atmosphäre und 5% CO₂ kultiviert. Die Zellzahlen sowie die Viabilität wurden mit einem CASY1 Cell Counter (Schaerfe System, DE) oder durch Trypanblau-Färbung bestimmt und die Zellen dann in einer Konzentration von 1-3×10⁵/mL eingesät und alle 2 - 3 Tage passagiert.
Zur Transfektion von CHO-DG44 wurde Lipofectamine Plus Reagenz (Invitrogen GmbH) eingesetzt. Pro Transfektionsansatz wurden dabei insgesamt 1 µg Plasmid-DNA, 4 µL Lipofectamine und 6 µL Plus-Reagenz nach den Angaben des Herstellers gemischt und in einem Volumen von 200 µL zu 6 x10⁵ exponentiell wachsenden CHO-DG44 Zellen in 0,8 mL HT-supplementiertem CHO-S-SFMII Medium gegeben. Nach dreistündiger Inkubation bei 37°C in einem Zellinkubator erfolgte eine Zugabe von 2 mL HT-supplementiertem CHO-S-SFMII Medium. Zur NPT-basierten Selektion wurden die Zellen 2 Tage nach Transfektion in HT-supplementiertes CHO-S-SFMII mit G418 (Invitrogen) transferiert, wobei das Medium alle 3 bis 4 Tage gewechselt wurde. In der Regel wurden zur Selektion 400 µg/mL G418 zugesetzt, wobei in einigen experimentellen Serien die Konzentration auch auf 200 µg/mL erniedrigt bzw. auf 500, 600 oder 800 µg/mL erhöht wurde. Bei einer DHFR- und NPT-basierten Selektion im Falle einer Co-Transfektion, in der der eine Expressionsvektor einen DHFR- und der andere Expressionsvektor einen Neomycin-Phosphotransferase-Selektionsmarker enthielt, wurden die Zellen 2 Tage nach Transfektion in CHO-S-SFMII Medium ohne Hypoxanthin- und Thymidinzusatz transferiert und dem Medium außerdem noch G418 (Invitrogen) in einer Konzentration von 400µg/mL zugesetzt. Eine DHFR-basierte Genamplifikation der integrierten heterologen Gene kann durch Zugabe des Selektionsagens MTX (Sigma, Deisenhofen, DE) in einer Konzentration von 5 - 2000 nM zu einem HT-freien CHO-S-SFMII Medium erreicht werden.

### 2. Expressionsvektoren

Zur Expressionsanalyse wurden eukaryontische Expressionsvektoren eingesetzt, die auf dem pAD-CMV Vektor (Werner et al., 1998) basieren und die konstitutive Expression eines heterologen Gens über die Kombination CMV Enhancer/Hamster Ubiquitin/S27a Promotor (WO 97/15664) vermitteln. Während der Basisvektor pBID das dhfr-Minigen enthält, das als amplifzierbarer Selektionsmarker dient (siehe z.B. EP-0-393-438), ist im Vektor pBIN das dhfr-Minigen durch ein Neomycin-Phosphotransferase-Resistenzgen ersetzt worden (Abb. 1). Hierzu wurde der Selektionsmarker Neomycin-Phosphotransferase, inklusive SV40 early Promotor und TK-Polyadenylierungssignal, aus dem kommerziellen Plasmid pBK-CMV (Stratagene, La Jolla, CA, USA) als 1640 bp Bsu361-Fragment isoliert. Nach einer Auffüllreaktion der Fragmentenden durch Klenow-DNA-Polymerase wurde das Fragment mit dem 3750 bp Bsu361/Stul-Fragment des Vektors pBID, das ebenfalls mit Klenow-DNA-Polymerase behandelt wurde, ligiert.
Im bicistronischen Basisvektor pBIDG (Abb. 1) wurde die IRES-GFP-Genregion aus dem Vektor pIRES2-EGFP (Clontech, Palo Alto, CA, USA) isoliert und in einer Weise unter die Kontrolle des CMV Enhancer/Promotors im Vektor pBID gebracht, dass die multiple Klonierungsstelle zwischen Promotorregion und IRES-Element erhalten blieb. Dabei wurde wie folgt vorgegangen. In einer PCR-Mutagenese, wobei das Plasmid pIRES2-EGFP als Template diente, wurde zum einen die Hindlll-Schnittstelle AAGCTT innerhalb der IRES-Sequenz durch den Einsatz mutagener Primer in die Sequenzabfolge ATGCTT umgewandelt und somit entfernt. Zum anderen wurde mittels eines Primers mit Komplementarität zum 5'Ende der IRES-Sequenz eine Xbal-Schnittstelle bzw. mit Komplementarität zum 3'Ende der GFP-Sequenz eine Spel-Schnittstelle eingeführt. Das resultierende PCR-Fragment, das die komplette IRES- und GFP-Sequenz umfasste, wurde mit Xbal und Spel verdaut und in die singuläre Xbal-Schnittstelle am 3'Ende der multiplen Klonierungsstelle des Vektors pBID kloniert. Auf gleiche Weise wurde die IRES-GFP-Genregion aus dem Vektor pIRES2-EGFP unter die Kontrolle des CMV Enhancer/Hamster Ubiquitin/S27a Promotors im Vektor pBIN gebracht. Daraus resultierte der bicistronische Basisvektor pBING (Abb. 1).

Die humane MCP-1 cDNA (Yoshimura et al., 1989) wurde als 0,3 kb Hindlll/EcoRI-Fragment in die entsprechenden Schnittstellen des Vektors pBIN kloniert, wobei der Vektor pBIN-MCP1 resultierte (Abb. 2A).

Zur Expression eines monoklonalen humanisierten lgG2 Antikörpers wurde die schwere Kette als 1,5 kb BamHl/Hindlll-Fragment in den mit BamHl und Hindlllverdauten Vektor pBID bzw. pBIDG kloniert, wobei der Vektor pBID-HC bzw. pBIDG-HC resultierte (Abb. 2B). Die leichte Kette hingegen wurde als 0,7 kb BamHl/Hindlll-Fragment in die entsprechenden Schnittstellen des Vektors pBIN bzw. pBING kloniert, wodurch der Vektor pBIN-LC bzw. pBING-LC (Abb. 2B) entstand.

### 3. FACS

Die flowcytometrischen Analysen und Sorting wurden mit einem Coulter Epics Altra-Gerät durchgeführt. Das FACS ist mit einem Helium-Argon-Laser mit einer Anregungswellenlänge von 488 nm ausgestattet. Die Fluoreszenzintensität wird bei einer dem Fluoreszenzprotein adäquaten Wellenlänge aufgenommen und mittels der angeschlossenen Software Coulter Expo32 prozessiert. Das Sorting wird standardmäßig mit einer Rate von 8000 - 10000 Events/Sekunde durchgeführt. Die suspendierten Zellen lassen sich abzentrifugieren (5 min bei 180xg) und in HBSS auf eine Zellkonzentration von 1-1,5 ×10⁷/mL eingestellen. Anschließend kann ein Sorting der Zellen entsprechend ihres Fluoreszenzprotein-Signals erfolgen. Die Zellen werden in Röhrchen mit vorgelegtem Kultivierungsmedium aufgenommen, abzentrifugiert und abhängig von der aussortierten Zellzahl in entsprechende Kultivierungsgefäße eingesät oder direkt in Mikrotiterplatten abgelegt.

### 4. ELISA

Die MCP-1-Titer in Überständen von stabil transfizierten CHO-DG44 Zellen wurden mittels ELISA mit dem Kit OptEIA Human MCP-1 Set nach dem Protokoll des Herstellers quantifiziert (BD Biosciences Pharmingen, Heidelberg, DE).
Die Quantifizierung des lgG2 mAks in den Überständen von stabil transfizierten CHO-DG44 Zellen erfolgte mittels ELISA nach Standardprotokollen (Current Protocols in Molecular Biology, Ausubel et al., 1994, updated), wobei zum einen ein Ziege anti Human lgG Fc-Fragment (Dianova, Hamburg, DE) und zum anderen ein AP-konjugierter Ziege anti Human Kappa light chain Antikörper (Sigma) eingesetzt wurde. Als Standard diente gereinigter lgG2 Antikörper.
Produktivitäten (pg/Zelle/Tag) wurden dabei mit der Formel pg/((Ct-Co) t / In (Ct-Co)) berechnet, wobei Co und Ct die Zellzahl bei Aussaat bzw. Ernte und t die Kultivierungsdauer angibt.

### 5. Dot Assay zur Bestimmung der NPT-Enzymaktivität

Zur Herstellung eines Zellextrakts nach einem Protokoll von Duch et al., 1990 wurden jeweils 6x10⁶ Zellen zweimal mit PBS gewaschen und nachfolgend in 600 µL Extraktionspuffer (0,135 M Tris-HCI pH 6,8, 20% Glycerin, 4 mM Dithiothreitol) resuspendiert. Nach viermaligem Einfrieren und Auftauen in einem Trockeneis- bzw. Wasserbad wurden die Zelltrümmer durch Zentrifugation entfernt und der Überstand für den nachfolgenden Enzym-Assay eingesetzt. Die Proteinkonzentration in den Zellextrakten wurde mittels eines Bradford-Assays unter Verwendung des BIO-RAD Protein Assays (Bio-Rad Laboratories GmbH, München, DE) bestimmt, wobei BSA als Standardprotein diente (Current Protocols in Molecular Biology, Ausubel et al., 1994, updated). Zur Bestimmung der NPT-Enzymaktivität wurde ein Dot Assay, basierend auf dem Protokoll von Platt et al. 1987, durchgeführt. Dazu wurden je 5 µg, 2,5 µg und 1,25 µg Protein mit Extraktionspuffer auf ein Endvolumen von 20 µL gebracht, wobei mit Zellextrakt aus nicht-transfizierten CHO-DG44 Zellen auf einen Gesamtproteingehalt von jeweils 5 µg aufgefüllt wurde. Nach Zugabe von jeweils 200 µL Assaypuffer (67 mM Tris-HCl pH 7,1, 42 mM MgCl2, 400 mM NH₄Cl) plus/minus 40 µg/mL G418 und plus/minus 5 µCi [γ-³³P]-ATP/mL (NEN) wurden die Extrakte bei 27°C für 135 Minuten inkubiert. Anschließend wurden die Extrakte in einem 96 Well Vakuum Manifold (Schleicher & Schüll, Dassel, DE) durch einen Sandwich aus je einer Lage Whatman 3MM-Papier, P81 Phosphocellulose-Membran (Whatman Laboratory Division, Maidstone, Großbritannien) und Nitrocellulose-Membran (Schleicher & Schüll) filtriert. Durch Proteinkinasen phosphorylierte Proteine sowie nicht-phosphorylierte Proteine binden dabei an die Nitrocellulose, während phosphoryliertes G418 die Nitrocellulose passiert und an die Phosphocellulose bindet. Nach dreimaligem Nachwaschen mit deionisiertem H₂O wurden die Membranen aus der Apparatur entnommen, nochmals mit H₂O gewaschen und dann luftgetrocknet. Die Quantifizierung der radioaktiven Signale erfolgte mittels eines Phospho Imagers (Molecular Dynamics, Krefeld, DE).

### 6. Northern Blot Analyse

Die Isolierung von Gesamt-RNA aus den Zellen erfolgte mit dem TRIZOL-Reagenz nach den Angaben des Herstellers (Invitrogen GmbH, Karlsruhe, DE) und die gelektrophoretische Auftrennung von je 30 µg RNA sowie der Transfer auf eine Hybond N+ Nylonmembran (Amersham Biosciences, Freiburg, DE) nach dem Standardprotokoll für Glyoxal/DMSO-denaturierte RNA (Current Protocols in Molecular Biology, Ausubel et al., 1994, updated). Als Sonde für die nachfolgende nicht-radioaktive Hybridisierung mit dem Genelmages CDP-Star Detection Kit (Amersham Biosciences) wurde ein mit dem Genelmages random prime labelling Kit (Amersham Biosciences, Freiburg, DE) FITC-dUTP-markiertes PCR-Produkt, das die Kodierregion des NPT-Gens umfasste, nach den Angaben des Herstellers verwendet.

### 7. Dot Blot Analyse

Die Isolierung von genomischer DNA aus den Zellen erfolgte unter Verwendung eines DNA-Isolierungskits nach den Angaben des Herstellers (DNA Isolation Kit for Cells and Tissue; Roche Diagnostics GmbH, Mannheim, DE). Verschiedene DNA-Mengen (10 µg, 5 µg, 2,5 µg, 1,25 µg, 0,63 µg und 0,32 µg) wurden nach Standardprotokoll (Ausubel et al., 1994) in einem alkalischen Puffer unter Verwendung eines 96 Well Vakuum Maniolds (Schleicher & Schüll, Dassel, DE) auf eine Hybond N+ Nylonmembran (Amersham Biosciences, Freiburg, DE) filtriert. Nicht-transfizierte CHO-DG44 Zellen dienten als Negativkontrolle. Als Standard wurde das Plasmid pBIN-LC eingesetzt (320 pg, 160 pg, 80 pg, 40 pg, 20 pg, 10 pg, 5 pg, 2,5 pg). Als Sonde für die nachfolgende nicht-radioaktive Hybridisierung mit dem Genelmages CDP-Star Detection Kit (Amersham Biosciences) wurde ein mit dem Genelmages random prime labelling Kit (Amersham Biosciences, Freiburg, DE) FITC-dUTP-markiertes PCR-Produkt, das die Kodierregion des NPT-Gens umfasste, nach den Angaben des Herstellers verwendet. Die Chemilumineszenzsignale wurden mittels eines ImageMaster VDS-CL (Amersham Biosciences) quantifiziert. Anschließend wurde die Kopienzahl der npt-Gene in den jeweiligen Zellen mit Hilfe der Standardreihe, die aus den ermittelten Signalintensitäten der titrierten Plasmid-DNA erstellt wurde, ermittelt. Die Anzahl der Plasmidmoleküle wurde dabei mit der Avogadroschen Konstante berechnet und der DNA-Gehalt einer CHO-Zelle als ca. 5 pg angenommen.

### Beispiel 1: Mutagenese der Neomycin-Phosphotransferase

Die zur Herstellung der NPT-Mutanten Glu182Gly (SEQ ID NO:3), Trp91Ala (SEQ ID NO:5), Val198Gly (SEQ ID NO:7), Asp227Ala (SEQ ID NO:9), Asp227Val (SEQ ID NO:11), Asp261Gly (SEQ ID NO:13), Asp261Asn (SEQ ID NO:15), Phe240lle (SEQ ID NO:17), Glu182Asp (SEQ ID NO:19), Asp227Gly (SEQ ID NO:21), Asp190Gly (SEQ ID NO:23) und Asp208Gly (SEQ ID NO:25) erforderlichen Basensubstitutionen im Wildtyp NPT-Gen erfolgten mittels PCR unter Verwendung von mutagenen Primern (Abb. 3). Der Vektor pBIN (Abb. 1) bzw. pBK-CMV (Stratagene, La Jolla, USA) diente dabei als Template für die PCR-Mutagenese. Zunächst wurden in separaten PCR-Ansätzen die 5' bzw. 3'Anteile der jeweiligen Mutanten hergestellt.
Für die Herstellung der Mutanten Glu182Gly, Glu182Asp, Trp91A1a, Asp190Gly, Vall98Gly, Asp208Gly und Asp227Gly wurden für die Amplifikation dazu Primerkombinationen eingesetzt, die aus Neofor5 (SEQ ID NO:27) und dem jeweiligen mutagenen reverse (rev) Primer bzw. aus Neorev5 (SEQ ID NO:28) und dem jeweiligen mutagenen forward (for) Primer bestanden:
- Im Fall von NPT-Mutante Glu182Gly (SEQ ID NO:3) aus Neofor5 (SEQ ID NO:27) und E182Grev (SEQ ID NO:32) bzw. aus Neorev5 (SEQ ID NO:28) und E182Gfor (SEQ ID NO:31);
- Im Fall von NPT-Mutante Glu182Asp (SEQ ID NO:19) aus Neofor5 (SEQ ID NO:27) und E182Drev (SEQ ID NO:48) bzw. aus Neorev5 (SEQ ID NO:28) und E182Dfor (SEQ ID NO:47);
- im Fall von NPT-Mutante Trp91Ala (SEQ ID NO:5) aus Neofor5 (SEQ ID NO:27) und W91Arev (SEQ ID NO:34) bzw. aus Neorev5 (SEQ ID NO:28) und W91Afor (SEQ ID NO:33);
- im Fall von NPT-Mutante Val198Gly (SEQ ID NO:7) aus Neofor5 (SEQ ID NO:27) und V198Grev (SEQ ID NO:36) bzw. aus Neorev5 (SEQ ID NO:28) und V198Gfor (SEQ ID NO:35)
- Im Fall von NPT-Mutante Asp190Gly (SEQ ID NO:23) aus Neofor5 (SEQ ID NO:27) und D190Grev (SEQ ID NO:50) bzw. aus Neorev5 (SEQ ID NO:28) und D190Gfor (SEQ ID NO:49);
- Im Fall von NPT-Mutante Asp208Gly (SEQ lD NO:25) aus Neofor5 (SEQ lD NO:27) und D208Grev (SEQ ID NO:52) bzw. aus Neorev5 (SEQ ID NO:28) und D208Gfor (SEQ lD N0:51);
- Im Fall von NPT-Mutante Asp227Gly (SEQ ID NO:21) aus Neofor5 (SEQ ID NO:27) und D227Grev (SEQ lD NO:54) bzw. aus Neorev5 (SEQ lD NO:28) und D227Gfor (SEQ lD NO:52).

Für die Herstellung der Mutanten Asp227Ala, Asp227Val, Asp261Gly, Asp261Asn und Phe240lle wurden für die Amplifikation Primerkombinationen eingesetzt, die aus Neofor2 (SEQ lD NO:29) und dem jeweiligen mutagenen reverse (rev) Primer bzw. aus lC49 (SEQ ID NO:30) und dem jeweiligen mutagenen forward (for) Primer bestanden:
- Im Fall von NPT-Mutante Asp227Ala (SEQ ID NO:9) aus Neofor2 (SEQ ID NO:29) und D227Arev (SEQ ID NO:38) bzw. aus IC49 (SEQ ID NO:30) und D227Afor (SEQ ID NO:37);
- im Fall von NPT-Mutante Asp227Val (SEQ ID NO:11) aus Neofor2 (SEQ ID NO:29) und D227Vrev (SEQ ID NO:40) bzw. aus IC49 (SEQ ID NO:30) und D227Vfor (SEQ ID NO:39);
- im Fall von NPT-Mutante Asp261Gly (SEQ ID NO:13) aus Neofor2 (SEQ ID NO:29) und D261Grev (SEQ ID NO:42) bzw. aus IC49 (SEQ ID NO:30) und D261Gfor (SEQ ID NO:41);
- im Fall von NPT-Mutante Asp261Asn (SEQ ID NO:15) aus Neofor2 (SEQ ID NO:29) und D261Nrev (SEQ ID NO:44) bzw. aus IC49 (SEQ ID NO:30) und D261 Nfor (SEQ ID NO:43);
- im Fall von NPT-Mutante Phe240lle (SEQ ID NO:17) aus Neofor2 (SEQ ID NO:29) und F240Irev (SEQ ID NO:46) bzw. aus IC49 (SEQ ID NO:30) und F240Ifor (SEQ ID NO:45).

Nachfolgend wurden der Kodierstrang des 5'Anteils und der Komplementärstrang des 3'Anteils der jeweiligen Mutanten durch Hybridisierung im Überlappungsbereich, der von den mutagenen Primersequenzen gebildet wird, zusammengeführt, die Einzelstrangbereiche aufgefüllt und das gesamte Produkt nochmals in einer PCR mit den Primern Neofor5 (SEQ ID NO:27) und Neorev5 (SEQ ID NO:28) bzw. Neofor2 (SEQ ID NO:29) und IC49 (SEQ ID NO:30) amplifiziert. Diese PCR-Produkte wurden mit Stul/Rsrll (Neofor5/Neorev5 PCR-Produkte der Mutanten Glu182Gly, Trp91Ala und Val198Gly), Stul/BstBI (Neofor5/Neorev5 PCR-Produkte der Mutanten Glu182Asp, Asp190Gly, Asp208Gly und Asp227Gly) bzw. Dralll/Rsrll (Neofor2/IC49 PCR-Produkte der Mutanten Asp227Ala, Asp227Val, Asp261Gly, Asp261Asn und Phe240IIe) verdaut. Anschließend wurde im Vektor pBIN-LC (Abb. 2B) bzw. pBK-CMV (Stratagene, La Jolla, USA) ein Teil der Wildtyp NPT-Sequenz durch Stul/Rsrll Verdau, Dralll/Rsrll Verdau bzw. Stul/BstBI Verdau entfernt und durch die korrespondierenden Fragmente der PCR-Produkte ersetzt. Durch Sequenzanalyse sowohl des Komplementär- als auch des Kodierstrangs wurden die gewünschten Basensubstitutionen in den jeweiligen Mutanten verifiziert und sichergestellt, dass die restliche DNA-Sequenz der Wildtyp NPT-Sequenz entsprach. Auf diese Weise wurden die Expressionsvektoren pBIN1-LC, pBlN2-LC, pBlN3-LC, pBlN4-LC, pBlN5-LC, pBlN6-LC, pBlN7-LC und pBlN8-LC generiert, die die NPT-Mutanten Glu182Gly, Trp91Ala, Val198Gly, Asp227Ala, Asp227Val, Asp261Gly, Asp261Asn bzw. Phe240lle enthalten (Abb. 2B).
Die restlichen NPT-Mutanten wurden als 1640 bp Bsu361-Fragment aus dem modifizierten pBK-CMV isoliert, die Fragmentenden mit Klenow-DNA-Polymerase aufgefüllt und mit dem 3750 bp Bsu361/Stul-Fragment des Vektors pBID, das ebenfalls mit Klenow-DNA-Polymerase behandelt wurde, ligiert. Auf diese Weise wurden die Expressionsvektoren pKS-N5, pKS-N6, pKS-N7 und pKS-N8 generiert, die die NPT-Mutanten Glu182Asp, Asp190Gly, Asp208Gly bzw. Asp227Gly enthalten. In diese Expressionsvektoren wurde dann jeweils die humane MCP-1 cDNA als 0,3 kb HindIII/EcoRI Fragment (Abb. 2A) bzw. die leichte Kette des humanisierten lgG2 Antikörpers als 0,7 kb HindIII/BamHI-Fragment kloniert (Abb. 2B).

Bei den eingeführten Mutationen in der Neomycin-Phosphotransferase handelt es sich zum einen um Substitutionen von mehr (Val198Gly, Phe240lle) oder weniger (Trp91Ala, Glu182Gly, Glu182Asp, Asp227Ala, Asp227Val, Asp227Gly) konservierten Aminosäuren, die konservierte Domänen, wie z.B. die Motive 1, 2 und 3 (Shaw et al., 1993), flankieren (Abb. 4). Zum anderen liegen die Mutationen innerhalb des konservierten Motivs 1 (Asp190Gly), 2 (Asp208Gly) oder 3 (Asp261Gly, Asp261Asn) und betreffen eine konservierte Aminosäure.

### Beispiel 2: Einfluss der NPT-Mutationen auf die Selektion von stabil transfizierten MCP-1 exprimierenden Zellen

MCP-1 wurde als Beispiel für die Expression eines einzelkettigen Proteins in CHO-Zellen gewählt. Dazu wurden CHO-DG44 mit pKS-N5-MCP1, pKS-N6-MCP1, pKS-N7-MCP1, pKS-N8-MCP1 bzw. pBIN-MCP1 transfiziert (Abb. 2A). Es wurden dabei jeweils Doppelansätze durchgeführt. Zwei Tage nach Transfektion wurden die Zellen in einer 96 Well-Platte ausgesät (2000 Zellen/Well) und mit 400 µg/mL G418 in HT-supplementiertem CHO-S-SFMII Medium selektioniert. Im Fall der mit pBIN-MCP1 transfizierten Zellen wurde parallel auch noch eine Selektion mit 800 µg/mL G418 durchgeführt. Die erhaltenen Zellpopulationen wurden sukzessive über 24 Well- in 6 Well-Platten überführt. Schon während der Selektionsphase konnten Unterschiede in den verschiedenen Transfektionsansätzen festgestellt werden. Im Gegensatz zu den Zellpopulationen, in denen mit einem NPT Wildtyp-Gen (SEQ ID NO:1) selektioniert wurde, überlebten in den Zellpopulationen, die mit einer mutierten NPT transfiziert worden waren, weniger Zellen die anfängliche Selektion mit G418. Diese Zellpopulationen konnten deshalb auch erst ca. 4 Tage später in die 24 Well-Platten transferiert werden. Und in den mit pKS-N6-MCP1 und pKS-N7-MCP1 transfizierten Ansätzen konnten bei einer Konzentration von 400 µg/mL G418 überhaupt keine stabil transfizierten Zellen selektioniert werden. Vermutlich ist in den NPT-Mutanten mit den Mutationen Asp190Gly bzw. Asp208Gly die Enzymfunktion so stark beeinträchtigt, dass nicht mehr genug G418-Moleküle inaktiviert werden können, um ein Wachstum der stabil transfizierten Zellen zu ermöglichen. Bei einer Reduktion der G418-Konzentration auf 200 µg/mL überlebten zwar einige wenige Zellen die erste Selektionsphase, aber sie zeigten alle starke Beeinträchtigungen in Wachstum und Vitalität und eine Expansion war, bis auf wenige Ausnahmen bei der Mutante Asp208Gly, nicht möglich.
Von den mit den Mutanten Glu182Asp und Asp227Gly bzw. mit dem NPT-Wildtyp transfizierten Zellen wurden jeweils 18 Pools (je 9 Pools aus Ansatz 1 und 2) über vier Passagen hinweg in 6 Well-Platten kultiviert und die Konzentration des produzierten MCP-1 im Zellkulturüberstand durch ELISA ermittelt. Zellpools, bei denen als Selektionsmarker die NPT-Mutanten eingesetzt wurden, zeigten durchschnittlich um 50% - 57% (Glu182Asp-Mutante) bzw. 57% - 65% (Asp227Gly-Mutante) höhere Produktivitäten als Zellpools, in denen die Selektion mit dem NPT-Wildtyp bei 400 oder sogar 800 µg/mL G418 durchgeführt worden war (Abb. 5). Somit konnte durch die Verwendung von NPT-Mutanten als Selektionsmarker der Anteil von Hochproduzenten in den transfizierten Zellpopulationen tatsächlich erhöht werden.

### Beispiel 3: Einfluss der NPT-Mutationen auf die Selektion von stabil transfizierten mAk exprimierenden Zellen

In einer Co-Transfektion wurden CHO-DG44 Zellen zunächst mit den Plasmidkombination pBIDG-HC/pBIN-LC (NPT-Wildtyp), pBIDG-HC/pKS-N5-LC (G1u182Asp NPT-Mutante) bzw. pBIDG-HC/pKS-N8-LC (Asp227Gly NPT-Mutante) transfiziert (Abb. 2B). In den eingesetzten Vektorkonfigurationen werden die beiden Proteinketten eines humanisierten lgG2 Antikörpers jeweils von einem eigenen Vektor, der zusätzlich noch für einen DHFR- bzw. Neomycin-Selektionsmarker in einer separaten Transkriptionseinheit kodiert, exprimiert. Die Expression der Produktgene wird dabei von einer CMV-Enhancer/Hamster Ubiquitin/S27a Promotor-Kombination vermittelt. Vergleichbare Daten können aber auch beispielsweise mit einem CMV Enhancer/Promotor, einem SV40-Enhancer/Hamster Ubiquitin/S27a Promotor oder anderen Promotorkombinationen erzielt werden.
Insgesamt wurden vier Transfektionsserien mit jeweils 6 Pools pro Plasmidkombination durchgeführt. Im Gegensatz zu den Zellpopulationen, in denen mit einem NPT-Wildtypgen selektioniert wurde, überlebten in den Zellpopulationen, die mit einem mutierten NPT transfiziert worden waren, weniger Zellen die anfängliche Selektion mit G418. Nach einer zwei- bis dreiwöchigen Selektion der transfizierten Zellpools in HT-freiem CHO-S-SFMII Medium mit Zusatz von 400 µg/mL G418 wurde über mehrere Passagen (6 - 8) hinweg eine Bestimmung des Antikörper-Titers in den Zellkulturüberständen mittels ELISA vorgenommen. Im Vergleich zur Verwendung eines NPT-Wildtypgens als Selektionsmarker wiesen die Zellen, die mit der GIu182Asp-Mutante selektioniert worden waren, durchschnittlich eine Erhöhung der Produktivität und des Titers um 86% bzw. 77% und die mit der Asp227Gly-Mutante selektionierten Zellen sogar eine Erhöhung der Produktivität und des Titers um 126% bzw. 107% auf. Durch die Verwendung einer NPT-Mutante mit verminderter Enzymaktivität konnten somit selektiv Zellen mit bis zu doppelt so hoher Grundproduktivität angereichert werden.
In einer weiteren Transfektionsserie wurde der Einfluss von verschiedenen G418-Konzentrationen auf die Selektion getestet. Zur Selektion der transfizierten Zellpools, jeweils 3 Pools, wurden hierbei 400, 500 bzw. 600 µg/mL G418 eingesetzt. Bei den höheren Konzentration überlebten in den Zellpopulationen, in denen mit einem NPT Wildtypgen selektioniert wurde, deutlich weniger Zellen die anfängliche Selektion, wobei der Effekt bei der Asp227Gly-Mutante am größten war. Die erhaltenen stabil transfizierten Zellpopulationen zeigten jedoch keine Beeinträchtigung bezüglich Wachstum und Vitalität. Zwischen den erzielten Produktivitäten und Titern konnte jedoch innerhalb einer zur Transfektion eingesetzten Plasmidkombination kein signifikanter Unterschied festgestellt werden. Aber auch hier wiesen die mit den NPT-Mutanten selektionierten Zellen wiederum im Durchschnitt die höchsten Produktivitäten auf, angeführt von der Asp227Gly-Mutante mit einer im Vergleich zum NPT-Wildtyp vierfach höheren Produktivität, gefolgt von der GIu182Asp-Mutante mit einer um Faktor 2,4 höheren Produktivität (Abb. 6A).

Nachfolgend wurden in einer Co-Transfektion CHO-DG44 Zellen mit den Plasmidkombinationen pBIDG-HC/pBIN-LC (NPT-Wildtyp), pBIDG-HC/pBIN1-LC (Glu182Gly NPT-Mutante), pBIDG-HC/pBIN2-LC (Trp91Ala NPT-Mutante), pBIDG-HC/pBlN3-LC (Val198Gly NPT-Mutante), pBIDG-HC/pBIN4-LC (Asp227Ala), pBIDG-HC/pBlN5-LC (Asp227Val NPT-Mutante), pBIDG-HC/pBIN6-LC (Asp261Gly NPT-Mutante), pBIDG-HC/pBIN7-LC (Asp261Asn NPT-Mutante) bzw. pBlDG-HC/pBlN8-LC (Phe240lle NPT-Mutante), transfiziert (Abb. 2B). Auch in diesen eingesetzten Vektorkonfigurationen werden die beiden Proteinketten eines monoklonalen humanisierten lgG2 Antikörpers jeweils von einem eigenen Vektor, der zusätzlich noch für einen DHFR- bzw. Neomycin-Selektionsmarker in einer separaten Transkriptionseinheit kodiert, exprimiert.
Pro Plasmidkombination wurden jeweils 5 Pools transfiziert. Im Gegensatz zu den Zellpopulationen, in denen mit einem NPT-Wildtypgen selektioniert wurde, überlebten in den Zellpopulationen, die mit einem mutierten NPT transfiziert worden waren, weniger Zellen die anfängliche Selektion mit G418. Nach einer zwei- bis dreiwöchigen Selektion der transfizierten Zellpools in HT-freiem CHO-S-SFMII Medium mit Zusatz von 400 µg/mL G418 wurde über sechs Passagen hinweg eine Bestimmung des Antikörper-Titers in den Zellkulturüberständen mittels ELISA vorgenommen. In Abb. 6B sind die Mittelwerte der ermittelten Titer und Produktivitäten aus den jeweiligen Pools im Test zusammengefasst. Im Vergleich zur Verwendung eines NPT-Wildtypgens als Selektionsmarker wiesen alle Zellpools, die mit einer NPT-Mutante selektioniert worden waren, durchschnittlich eine Erhöhung der Produktivität und des Titers um den Faktor 1, 4 - 14,6 bzw. 1,4 - 10,8 auf (Abb. 6B). Die beste selektive Anreicherung von Zellen mit höherer Grundproduktivität konnte dabei mit den NPT-Mutanten Asp227Val bzw. Asp261Gly erzielt werden, mit Steigerungen der durchschnittlichen Produktivität um den Faktor 14,6 bzw. 9,3.

Im Vektor pBIDG-HC ist ein weiterer Selektionsmarker, das GFP, enthalten. Das GFP ist dabei transkriptionell mit der schweren Kette über ein IRES-Elements verknüpft. Die dadurch bedingte Korrelation zwischen der Expression des Zielproteins und des Selektionsmarkers GFP ermöglicht deshalb auch eine schnelle Bewertung der Höhe und der Verteilung der Expressionslevel in den transfizierten Zellpopulationen auf der Basis der in FACS-Analysen bestimmten GFP-Fluoreszenz.
Nach einer zwei- bis dreiwöchigen Selektion der transfizierten Zellpools in HT-freiem CHO-S-SFMII Medium mit Zusatz von G418 wurde in einer FACS-Analyse die GFP-Fluoreszenz gemessen (Abb. 7). Die erhaltenen GFP-Fluoreszenzsignale korrelierten in der Tat mit den ermittelten Titerdaten für den monoklonalen IgG2 Antikörper. Mit den NPT-Mutanten Asp227Val, Asp261Gly, Asp161Asn und Phe240Ile selektionierte Pools wiesen auch den größeren Anteil an Zellen mit hoher GFP-Fluoreszenz auf, gefolgt von den mit den NPT-Mutanten Trp91Ala, Asp227Gly, Gly182Asp, Asp227Ala, Glu182Gly und Val198Gly selektionierten Zellen.

Durch die Zugabe des Selektionsagens Methotrexat (MTX) zum Kulturmedium konnte die Produktivität der Zellen durch die Induktion einer dhfr-vermittelten Genamplifikation noch weiter erhöht werden. So konnte zum Beispiel nach einem einfachen Genamplifikationsschritt mit 100 nM MTX die spezifische Produktivität in den Zellpools, die aus einer Co-Transfektion mit den Plasmidkombinationen pBIDG-HC/pBIN5-LC (NPT-Mutante Asp227Val), pBIDG-HC/pBIN6-LC (NPT-Mutante Asp261Gly), pBIDG-HC/pBIN7-LC (NPT-Mutante Asp261Asn) und pBIDG-HC/pBIN8-LC (NPT-Mutante Phe240Ile) resultierten, um den Faktor 2 bis 4 gesteigert und je nach Pool Produktivitäten zwischen 4 und 14 pg/Zelle/Tag erzielt werden. In Abbildung 8 sind exemplarisch an einem Zellpool, der aus der Co-Transfektion pBIDG-HC/pBIN5-LC (NPT-Mutante Asp227Val) erhalten wurde, die durch MTX-Zugabe (100 nM MTX gefolgt von 500 nM MTX) erzielten Steigerungen in der Produktivität auf 27 pg/Zelle/Tag dargestellt.

### Beispiel 4: Bestimmung und Vergleich der NPT-Enzymaktivität

Um die Enzymaktivität der NPT-Mutanten mit der des NPT-Wildtyps zu vergleichen wurde ein Dot Assay zur Bestimmung der NPT-Aktivität in Zellextrakten, basierend auf dem Protokoll von Platt et al. 1987 und beispielhaft in Abb. 9A für die NPT-Mutanten Glul82Asp und Asp227Gly gezeigt, durchgeführt. Dazu wurden Zellextrakte von jeweils zwei verschiedenen mAk exprimierenden Zellpools hergestellt, die entweder mit dem NPT-Wildtypgen (SEQ ID NO:1) oder mit den NPT-Mutanten GIu182Gly (SEQ ID NO:3), Glu182Asp (SEQ ID NO:19), Trp91Ala (SEQ ID NO:5), Asp190Gly (SEQ ID NO:23), Val198Gly (SEQ ID NO:7), Asp208Gly (SEQ ID NO:25), Asp227Ala (SEQ ID NO:9), Asp227Val (SEQ ID NO:11), Asp227Gly (SEQ ID NO:21), Asp261Gly (SEQ ID NO:13), Asp261Asn (SEQ ID NO:15), bzw. Phe240lle (SEQ ID NO:17) transfiziert und selektioniert worden waren. Als Negativkontrolle dienten Zellextrakte aus nicht-transfizierten CHO-DG44 Zellen.
Die Enzymaktivitäten der NPT-Mutanten waren im Vergleich zum NPT-Wildtyp signifikant reduziert. Durchschnittlich wiesen die NPT-Mutanten lediglich noch zwischen 1,5% und 62% der Wildtyp-Enzymaktivität auf, wobei die NPT-Mutanten Asp261Gly und Asp261Asn mit 3,1% bzw. 1,5% die geringste Restaktivität und die NPT-Mutanten Val198Gly sowie Trp91Ala mit 61,9% bzw. 53,2% die höchste Restaktivität aufwiesen (Abb. 9B). Die auf der Phosphocellulose erhaltenen Signale waren dabei spezifisch für die durch die NPT-Enzymaktivität hervorgerufene Phosphorylierung von G418. Ohne Zusatz des NPT-Substrats G418 zum Assaypuffer konnte keine Aktivität auf der Phosphocellulose nachgewiesen werden. Die auf der Nitrocellulose-Membran erhaltenen Signale, die aus den durch Proteinkinasen phosphorylierten Proteinen innerhalb des Zellextrakts resultierten, wurden als interne Kontrolle für gleiche Probenauftragsmengen herangezogen.
Die reduzierte Enzymaktivität der NPT-Mutanten im Vergleich zum NPT-Wildtyp war dabei nicht auf eine verringerte Genexpression zurückzuführen. Im Gegenteil, Northern Blot Analysen an Gesamt-RNA ergaben, dass Zellpools, die mit dem NPT-Wildtyp transfiziert worden waren und eine hohe NPT-Enzymaktivität aufwiesen, weniger RNA exprimierten als mit NPT-Mutanten transfizierte Zellpools (Abb. 10). Die einzige Ausnahme bildeten Zellen, die mit der NPT-Mutante Glu182Gly transfiziert worden waren und vergleichbare Mengen an NPT-mRNA exprimierten. In Dot Blot Analysen, die an genomischer DNA aus diesen transfizierten Zellpopulationen durchgeführt wurden, konnte gezeigt werden, dass die höhere Expression der NPT-Mutanten durch Gendosiseffekte und/oder durch Integration der exogenen DNA in transkriptionsaktivere genomische Regionen erzielt wurden (Abb. 10). Zum Beispiel, in den mit der NPT-Mutante Trp91A1a selektionierten Zellen dominiert in Pool 1 der Gendosiseffekt, in Pool 2 hingegen der Integrationseffekt. Auf diese Weise können transfizierte Zellen, bei denen zur Selektion Marker mit reduzierter Enzymaktivität verwendet wurden, eine ausreichende hohe Menge an Markerprotein synthetisieren, um bei identischem Selektionsdruck die verminderte Resistenz gegenüber dem selektiven Agens auszugleichen.

### Beispiel 5: Isolierung von Zellen mit hoher Expression eines mAks durch GFPbasiertes FACS-Sorting

In einer Co-Transfektion wurden CHO-DG44 Zellen mit der Plasmidkombination pBID-HC und pBING-LC, kodierend für einen monoklonalen humanisierten lgG2 Antikörper, transfiziert (Abb.2B). In den eingesetzten Vektorkonfigurationen werden die beiden Proteinketten des Antikörpers jeweils von einem eigenen Vektor, der zusätzlich noch für einen DHFR- bzw. modifizierten Neomycin-Phosphotransferase-Selektionsmarker (Asp227Gly-Mutante; SEQ ID NO:21) in einer separaten Transkriptionseinheit kodiert, exprimiert. Zusätzlich ist im Vektor pBING-LC ein weiterer Selektionsmarker, das GFP, enthalten. Durch die transkriptionelle Verknüpfung der Expression des GFPs und der leichten Kette mittels eines IRES-Elements konnten bei der Co-Transfektion von CHO-DG44 mit den Vektoren pBID-HC/pBING-LC in kurzer Zeit Zellen mit einer hohen Expression des monoklonalen Antikörpers allein dadurch isoliert werden, dass mittels sequentiellem FACS-Sorting die Zellen mit einem hohen GFP-Gehalt selektioniert wurden. Insgesamt wurden 8 separate Zellpools transfiziert, aus denen nach einer ersten zwei- bis dreiwöchigen Selektion in HT-freiem CHO-S-SFMII Medium mit Zusatz von 400 µg/mL G418 stabil transfizierte Zellpopulationen erhalten wurden. Mittels ELISA wurden die Titer und Produktivitäten aller 8 Pools über mehrere Passagen (7- 8) hinweg bestimmt. Im Durchschnitt lagen die Titer bei ca. 1,4 mg/L und die Produktivitäten bei ca. 1,3 pg/Zelle/Tag. Für das sich anschließende sequentielle FACS-basierte Sortierung wurden die Pools 5 und 8 ausgewählt, wobei der Pool 5 die höchste Produktivität und der Pool 8 eine dem Durchschnitt aller Pools entsprechende Produktivität aufwies. Bei jedem Schritt wurden jeweils die 5% Zellen mit der höchsten GFP-Fluoreszenz durch FACS heraussortiert wurden und im Pool weiter kultiviert. Diese Sortierung wurde insgesamt bis zu sechsmal durchgeführt, wobei zwischen jeder Sortierung eine Kultivierungsperiode von ca. zwei Wochen lag. Erstaunlicherweise konnte dabei eine gute Korrelation zwischen mAk-Produktivität und GFP-Fluoreszenz gezeigt werden (Abb. 13), obwohl beide Proteinketten jeweils von einem eigenen Vektor aus exprimiert wurden und bei der GFP-basierten FACS-Sortierung zudem auch nur auf die Expression der leichten Kette, bedingt durch deren transkriptionelle Kopplung mit GFP, selektioniert werden konnte. Die Produktivitäten konnten dabei allein durch die FACS-basierte Sortierung auf bis zu 9,5 pg/Zelle/Tag gesteigert werden (Abb. 11). Vergleichbare Daten konnten auch bei einer funktionellen Verknüpfung des Hamster-Promotors mit dem SV40-Enhancer anstelle des CMV-Enhancers erzielt werden. Durch einen einzigen nachfolgenden MTX-Amplifikationsschritt, ausgehend von Pools 5 und 8 aus dem ersten Sortierungsschritt, durch Zugabe von 100 nM MTX zum Selektionsmedium konnte die Produktivität der Pools noch auf durchschnittlich über 20 pg/Zelle/Tag erhöht werden (Abb. 12). Die hohen Expressionslevel des fluoreszenten Proteins hatten dabei keinerlei negativen Einfluss auf Zellwachstum und -vitalität. Bei der Genamplifikation werden die Wachstumseigenschaften der Zellen auch maßgeblich durch die Zugabe von MTX, vor allem bei Zusatz in höheren Konzentrationen, negativ beeinflusst. Die vorsortierten Zellpools wiesen jedoch ein erheblich robusteres Verhalten gegenüber der recht hohen initialen Dosis von 100 nM MTX auf. Sie überstanden die Selektionsphase viel besser, d.h. es wurden schon nach 2 Wochen wieder Zellpopulationen mit hoher Vitalität und guter Wachstumsrate erhalten.
Zudem konnte die Entwicklungszeit zur Selektion von hochproduzierenden Zellen im Vergleich zu einer konventionellen stufenweisen Genamplifikationsstrategie, die in der Regel vier Amplifikationsstufen mit steigenden Zugaben von MTX umfasst, auf ca. 6 Wochen reduziert werden. Dies wurde durch die kombinierte Anwendung einer Anreicherung von transfizierten Zellen mit erhöhter Expression der Gene von Interesse, erzielt durch die Verwendung eines modifizierten NPT-Selektionsmarkers mit reduzierter Enzymaktivität, gefolgt von einer GFP-basierten FACS-Sortierung mit einem nachfolgenden Genamplifikationsschritt erreicht.

### LITERATUR

Adam, M.A. et al., J Virol 1991, 65, 4985 - 4990
Altschul, S.F. et al., Nucleic Acids Res. 1997, 25, 3389 - 3402
Altschul, S.F. et al., J Mol Biol 1990, 215, 403 - 410
Ausubel, F.M. et al., Current Protocols in molecular biology. New York : Greene Publishing Associates and Wiley-Interscience. 1994 (updated)
Blázques, J. et al., Molecular Microbiology 1991, 5(6), 1511 - 1518
Bennett, R.P. et al., BioTechniques 1998, 24, 478 - 482
Burk, D.L. et al., Biochemistry 2001, 40 (30), 8756 - 8764
Chalfie, M. et al., Science 1994, 263, 802 - 805
Chamov, S.M. et al., Antibody Fusion Proteins, Wiley-Liss Inc., 1999
Davies, M.V. et al., J Virol 1992, 66, 1924 - 1932
Faisst, S. et al., Nucleic Acids Research 1992, 20, 3 - 26
Gish, W. & States, D.J., Nature Genet. 1993, 3, 266 - 272
Gossen, M. et al., Curr Opi Biotech 1994, 5, 516 - 520
Duch, M. et al., Gene 1990, 95, 285 - 288
Hanson, K.D. et al., Mol Cell Biol 1995, 15(1), 45 - 51
Haber, D.A. et al., Somatic Cell Genetics 1982, 8, 499 - 508
Harris et al., Protein Purification : A Practical Approach, Pickwood and Hames, eds., IRL Press, 1995
Hemann, C. et al., DNA Cell Biol 1994, 13 (4), 437 - 445
Hon, W. et al., Cell 1997, 89, 887 - 895
Hu, S. et al., Cancer Res. 1996, 56 (13), 3055 - 3061
Huston, C. et al., Proc Natl Acad Sci USA 1988, 85 (16), 5879 - 5883
Jang, S.K. et al., J Virol 1989, 63, 1651 - 1660
Kaufman, R.J., Methods in Enzymology 1990, 185, 537 - 566
Kocabiyik, S. et al., Biochem Biophys Res Commun 1992, 185(3), 925 - 931
Kortt, A.A. et al., Protein Engineering 1997, 10 (4), 423 - 433
Lottspeich F. and Zorbas H. eds., Bioanalytic, Spektrum Akad. Verl., 1998
Lovejoy, B. et al., Science 1993, 259, 1288 - 1293
Madden, T.L. et al., Meth. Enzymol. 1996, 266, 131 - 141;
Monaco, L. et al., Gene 1996, 180, 145 - 15
Morgan, R.A. et al., Nucleic Acids Research 1992, 20, 1293 - 1299
Mosser, D.D. et al., BioTechniques 1997, 22, 150 - 161
Ohshima, Y. et al., J Mol Biol 1987, 195, 247 - 259
Pack, P. et al., Biotechnology 1993, 11, 1271 - 1277
Pack, P. et al., J Mol Biol 1995, 246 (11), 28 - 34
Pelletier, J. et al., Nature 1988, 334, 320 - 325
Perisic, O. et al., Structure 1994, 2, 1217 - 1226
Platt, S.G. et al., Analyt Biochem 1987, 162, 529 - 535
Ramesh, N. et al., Nucleic Acids Research 1996, 24, 2697 - 2700
Sambrook, J., Fritsch, E.F. & Maniatis, T.,Molecular Cloning: A Laboratory Manual Cold Spring Harbor Laboratory, Cold Spring Harbor, New York, 1989
Scopes, R., Protein Purification, Springer Verlag, 1988
Shaw, K.J. et al., Microbiological Reviews 1993, 57(1), 138 - 163
Simonson, C.C. et al., Proc Natl Acad Sci USA 1983, 80, 2495 - 2499
Sugimoto et al., Biotechnology 1994, 12, 694 - 698
Yenofsky, R.L. et al., Proc Natl Acad Sci USA 1990, 87, 3435 - 3439
Yoshimura, T. et al., FEBS LETTERS 1989, 244(2), 487 - 493
Urlaub, G. et al., Cell 1983, 33, 405 - 412
Werner, R.G. et al., Arzneim.-Forsch./Drug.Res. 1998, 48, 870 - 880
Wigler, M. et al., Proc Natl Acad Sci USA 1980, 77, 3567 - 3570
Zhang, J. & Madden, T.L. Genome Res. 1997, 7, 649 - 656;

## Patentansprüche

1. Modifiziertes Neomycin-Phosphotransferase-Gen ***dadurch gekennzeichnet,* dass** das modifizierte Neomycin-Phosphotransferase-Gen an Aminosäureposition 91, 198 und/oder 240 bezogen auf das Wildtyp-Gen für eine andere Aminosäure als das Wildtyp-Neomycin-Phosphotransferase-Gen kodiert.

2. Modifiziertes Neomycin-Phosphotransferase-Gen nach Anspruch 1 ***dadurch gekennzeichnet,* dass** die modifizierte Neomycin-Phosphotransferase, die durch das Neomycin-Phosphotransferase-Gen kodiert wird, eine geringere Enzymaktivität als die Wildtyp-Neomycin-Phosphotransferase aufweist.

3. Modifiziertes Neomycin-Phosphotransferase-Gen nach Anspruch 1 oder 2 ***dadurch gekennzeichnet,* dass** das modifizierte Neomycin-Phosphotransferase-Gen im Vergleich zum Wildtyp-Gen an Aminosäureposition 91 bezogen auf das Wildtyp-Gen für Alanin, an Aminosäureposition 198 für Glycin, und/oder an Aminsäureposition 240 für Isoleucin kodiert.

4. Modifiziertes Neomycin-Phosphotransferase-Gen nach einem der Ansprüche 1 bis 3 ***dadurch gekennzeichnet,* dass** das modifizierte Neomycin-Phosphotransferase-Gen für ein Polypeptid mit einer Aminosäuresequenz nach SEQ ID NO:6, SEQ SEQ ID NO:8 oder SEQ ID NO:18 kodiert.

5. Modifiziertes Neomycin-Phosphotransferase-Gen nach einem der Ansprüche 1 bis 4 enthaltend oder bestehend aus eine(r) Sequenz nach SEQ ID NO:5, SEQ ID NO:7 oder SEQ ID NO:17.

6. Modifiziertes Neomycin-Phosphotransferase-Gen ***dadurch gekennzeichnet,* dass** das modifizierte Neomycin-Phosphotransferase-Gen im Vergleich zum Wildtyp-Gen an Aminosäureposition 182 bezogen auf das Wildtyp-Gen für Glycin, an Aminossäureposition 227 für Alanin oder Valin und/oder an Aminosäureposition 261 für Glycin kodiert.

7. Modifiziertes Neomycin-Phosphotransferase-Gen nach Anspruch 6 ***dadurch gekennzeichnet,* dass** das modifizierte Neomycin-Phosphotransferase-Gen für ein Polypeptid mit einer Aminosäuresequenz nach SEQ ID NO:4, SEQ ID NO:10, SEQ ID NO:12 oder SEQ ID NO:14 kodiert.

8. Modifiziertes Neomycin-Phosphotransferase-Gen nach einem der Ansprüche 6 oder 7 enthaltend oder bestehend aus eine(r) Sequenz nach SEQ ID NO:3, SEQ ID NO:9, SEQ ID NO:11 oder SEQ ID NO:13.

9. Modifizierte Neomycin-Phosphotransferase kodiert durch ein modifiziertes Neomycin-Phosphotransferase-Gen nach einem der Ansprüche 1 bis 8.

10. Eukaryontischer Expressionsvektor enthaltend ein modifiziertes Neomycin-Phosphotransferase-Gen nach einem der Ansprüche 1 bis 8.

11. Eukaryontischer Expressionsvektor enthaltend ein heterologes Gen von Interesse in funktioneller Verknüpfung mit einem heterologen Promotor und ein modifiziertes Neomycin-Phosphotransferase-Gen, welches für eine Neomycin-Phosphotransferase kodiert, die im Vergleich zur Wildtyp-Neomycin-Phosphotransferase eine geringere Enzymaktivität aufweist.

12. Expressionsvektor nach Anspruch 11 ***dadurch gekennzeichnet,***
a) **dass** es sich bei dem modifizierten Neomycin-Phosphotransferase-Gen um ein Gen nach einem der Ansprüche 1 bis 5 handelt, oder
b) das modifizierte Neomycin-Phosphotransferase-Gen an Aminosäureposition 182 und/oder 227 für eine andere Aminosäure als das Wildtyp-Gen an der ensprechenden Stelle kodiert, oder
c) das modifizierte Neomycin-Phosphotransferase-Gen an Aminosäureposition 261 für ein Glycin kodiert.

13. Expressionsvektor nach Anspruch 12 ***dadurch gekennzeichnet,* dass** das modifizierte Neomycin-Phosphotransferase-Gen an Aminosäureposition 182 bezogen auf das Wildtyp-Gen für Glycin oder Asparaginsäure kodiert und/oder an Aminosäureposition 227 bezogen auf das Wildtyp-Gen für ein Alanin, Glycin oder Valin kodiert.

14. Expressionsvektor nach Anspruch 12 ***dadurch gekennzeichnet,* dass** das modifizierte Neomycin-Phosphotransferase-Gen für ein Protein mit einer Aminosäuresequenz nach SEQ ID NO:4, SEQ ID NO:6, SEQ ID NO:8, SEQ ID NO:10, SEQ ID NO:12, SEQ ID NO:14, SEQ ID NO:18, SEQ ID:20 oder SEQ ID NO:22 kodiert.

15. Expressionsvektor nach einem der Ansprüche 11 bis 14 ***dadurch gekennzeichnet,* dass** er einen oder mehrere Enhancer in funktioneller Verknüpfung mit dem oder den Promotoren enthält.

16. Expressionsvektor nach Anspuch 15 ***dadurch gekennzeichnet,* dass** der Enhancer ein CMV- oder SV40-Enhancer ist.

17. Expressionsvektor nach einem der Ansprüche 11 bis 16 ***dadurch gekennzeichnet,* dass** er einen Hamster Ubiquitin/S27a-Promotor enthält.

18. Expressionsvektor nach Anspruch 17 ***dadurch gekennzeichnet,* dass** das heterologe Gen von Interesse unter der Kontrolle des Ubiquitin/S27a-Promotors steht.

19. Expressionsvektor nach einem der Ansprüche 11 bis 18 ***dadurch gekennzeichnet,* dass** er zusätzlich ein Gen für ein fluoreszierendes Protein enthält, welches optional funktionell mit dem Gen von Interesse und dem heterologen Promotor verknüpft ist/wird.

20. Expressionsvektor nach Anspruch 19 ***dadurch gekennzeichnet,* dass** er zusätzlich eine interne Ribosomenbindungsstelle (IRES) enthält, welche eine bicistronische Expression des Gens, das für ein fluoreszierendes Protein kodiert, und des Gens, das für ein Protein/Produkt von Interesse kodiert, unter der Kontrolle eines heterologen Promotors ermöglicht.

21. Expressionsvektor nach einem der Ansprüche 19 oder 20 ***dadurch gekennzeichnet,* dass** sich das Gen, das für ein fluoreszierendes Protein kodiert, und das modifizierte Neomycin-Phosphotransferase-Gen in einer oder in zwei separaten Transkriptionseinheiten befinden.

22. Säugerzelle enthaltend ein modifiziertes Neomycin-Phosphotransferase-Gen nach einem der Ansprüche 1 bis 8.

23. Säugerzelle die mit einem Expressionsvektor nach einem der Ansprüche 10 bis 18 transfiziert wurde.

24. Säugerzelle die mit einem Expressionsvektor nach einem der Ansprüche 19 bis 21 transfiziert wurde.

25. Säugerzelle nach einem der Ansprüche 22 bis 24 ***dadurch gekennzeichnet,* dass** sie zusätzlich mit einem Gen für einen amplizifierbaren Selektionsmarker transfiziert ist.

26. Säugerzelle nach Anspruch 25 ***dadurch gekennzeichnet,* dass** es sich bei dem amplifizierbaren Selektionsmarkergen um die Dihydrofolat-Reduktase (DHFR) handelt.

27. Säugerzelle nach einem der Ansprüche 22 bis 26 ***dadurch gekennzeichnet,* dass** es sich bei der Säugerzelle um eine Nagerzelle handelt.

28. Säugerzelle nach Anspruch 27 ***dadurch gekennzeichnet,* dass** es sich bei der Nagerzelle um eine CHO- oder BHK-Zelle handelt.

29. Verfahren zur Anreicherung von Säugerzellen ***dadurch gekennzeichnet,* dass**
(i) ein Pool von Säugerzellen mit einem Gen für eine modifizierte Neomycin-Phosphotransferase nach einem der Ansprüche 1 bis 8, oder mit einer E182D-, D227G- oder D261 N-Neomycin-Phosphotransferase-Mutante transfiziert wird;
(ii) die Säugerzellen unter Bedingungen kultiviert werden, die eine Expression des modifizierten Neomycin-Phosphotransferase-Gens erlauben; und
(iii) die Säugerzellen in Anwesenheit zumindest eines Selektionsmittels kultiviert werden, das selektiv auf das Wachstum von Säugerzellen wirkt, und solche Zellen im Wachstum bevorzugt, die das modifizierte Neomycin-Phosphotransferase-Gen exprimieren.

30. Verfahren zur Gewinnung und Selektion von Säugerzellen, die mindestens ein heterologes Gen von Interesse exprimieren, ***dadurch gekennzeichnet,* dass**
(i) ein Pool von Säugerzellen mit mindestens einem Gen von Interesse und einem Gen für eine modifizierte Neomycin-Phosphotransferase nach einem der Ansprüche 1 bis 8 oder, mit einer E182D-, D227G- oder D261N-Neomycin-Phosphotransferase-Mutante transfiziert wird;
(ii) die Säugerzellen unter Bedingungen kultiviert werden, die eine Expression des (der) Gens(Gene) von Interesse und des modifizierten Neomycin-Phosphotransferase-Gens erlauben; und
(iii) die Säugerzellen in Anwesenheit zumindest eines Selektionsmittels kultiviert werden, das selektiv auf das Wachstum von Säugerzellen wirkt, und solche Zellen im Wachstum bevorzugt, die das modifizierte Neomycin-Phosphotransferase-Gen exprimieren.

31. Verfahren nach Anspruch 30 ***dadurch gekennzeichnet,* dass** die Säugerzellen zusätzlich mit einem Gen für einen amplifizierbaren Selektionsmarker transfiziert werden und die selektionierten Säugerzellen vorzugsweise zumindest einem Genamplifikationsschritt unterzogen werden, wobei es sich bei dem amplifizierbaren Selektionsmarkergen vorzugsweise um DHFR handelt und die Genamplifikation vorzugsweise durch die Zugabe von Methotrexat erfolgt.

32. Verfahren zur Gewinnung und Selektion von Säugerzellen, die mindestens ein heterologes Gen von Interesse exprimieren, ***dadurch gekennzeichnet,* dass**
(i) rekombinante Säugerzellen mit einem Expressionsvektor nach einem der Ansprüche 19 bis 21 transformiert werden;
(ii) unter Bedingungen kultiviert werden, die eine Expression des (der) Gens (e) von Interesse, des Gens, das für ein fluoreszierendes Protein kodiert, und des modifizierten Neomycin-Phosphotransferase-Gens ermöglicht;
(iii) die Säugerzellen in Anwesenheit zumindest eines Selektionsmittels kultiviert werden, das selektiv auf das Wachstum von Säugerzellen wirkt, und solche Zellen im Wachstum bevorzugt, die das modifizierte Neomycin-Phosphotransferase-Gen exprimieren; und
(iv) die Säugerzellen mittels flowzytometrischer Analyse sortiert werden.

33. Verfahren nach Anspruch 32 ***dadurch gekennzeichnet,* dass** die Säugerzellen zusätzlich mit einem Gen für einen amplifizierbaren Selektionsmarker transfiziert werden und die mittels flowzytometrischer Analyse sortierten Zellen zumindest einem Genamplifikationsschritt unterzogen werden, wobei es sich bei dem amplifizierbaren Selektionsmarkergen vorzugsweise um DHFR handelt und die Genamplifikation vorzugsweise durch die Zugabe von Methotrexat erfolgt.

34. Verfahren zur Herstellung von mindestens einem Protein von Interesse in rekombinanten Säugerzellen ***dadurch gekennzeichnet,* dass**
(i) ein Pool von Säugerzellen mit mindestens einem Gen von Interesse und einem Gen für eine modifizierte Neomycin-Phosphotransferase nach einem der Ansprüche 1 bis 8, oder mit einer E182D-, D227G- oder D261N-Neomycin-Phosphotransferase-Mutante transfiziert wird;
(ii) die Zellen unter Bedingungen kultiviert werden, die eine Expression des (der) Gens (Gene) von Interesse und des modifizierten Neomycin-Phosphotransferase-Gens erlauben;
(iii) die Säugerzellen in Anwesenheit zumindest eines Selektionsmittels kultiviert werden, das selektiv auf das Wachstum von Säugerzellen wirkt, und solche Zellen im Wachstum bevorzugt, die das modifizierte Neomycin-Phosphotransferase-Gen exprimieren; und
(iv)das (die) Protein(e) von Interesse aus den Säugerzellen oder dem Kulturüberstand gewonnen wird (werden).

35. Verfahren zur Herstellung von mindestens einem Protein von Interesse in rekombinanten Säugerzellen ***dadurch gekennzeichnet,* dass**
(i) rekombinante Säugerzellen mit einem Expressionsvektor nach einem der Ansprüche 19 bis 21 transfiziert werden;
(ii) unter Bedingungen kultiviert werden, die eine Expression des (der) Gens (e) von Interesse, des Gens, das für ein fluoreszierendes Protein kodiert, und des modifizierten Neomycin-Phosphotransferase-Gens ermöglicht;
(iii) die Säugerzellen in Anwesenheit zumindest eines Selektionsmittels kultiviert werden, das selektiv auf das Wachstum von Säugerzellen wirkt, und solche Zellen im Wachstum bevorzugt, die das modifizierte Neomycin-Phosphotransferase-Gen exprimieren; und
(iv) die Säugerzellen mittels flowzytometrischer Analyse sortiert werden
(v) das (die) Protein(e) von Interesse aus den Säugerzellen oder dem Kulturüberstand gewonnen wird (werden).

36. Verfahren zur Herstellung von mindestens einem Protein von Interesse ***dadurch gekennzeichnet,* dass**
(i) Säugerzellen nach einem der Ansprüche 25 oder 26 unter Bedingungen kultiviert werden, die eine Expression des Gens von Interesse, des modifizierten Neomycin-Phosphotransferase-Gens und des amplifizierbaren Selektionsmarkergens erlauben;
(ii) die Säugerzellen in Anwesenheit zumindest eines Selektionsmittels kultiviert und selektioniert werden, das selektiv auf das Wachstum von Säugerzellen wirkt, und solche Zellen im Wachstum bevorzugt, die das modifizierte Neomycin-Phosphotransferase-Gen exprimieren;
(iii) die selektionierten Säugerzellen zumindest einem Genamplifikationsschritt in unterzogen werden; und
(iv) das (die) Protein(e) von Interesse anschließend aus den Säugerzellen oder dem Kulturüberstand gewonnen wird (werden).

37. Verfahren nach einem der Ansprüche 34 bis 36 ***dadurch gekennzeichnet,* dass** die Säugerzellen mit mindestens zwei Genen von Interesse transfiziert sind, die für ein heteromeres Protein/Produkt kodieren, und
(i) unter Bedingungen kultiviert werden, die eine Expression der Untereinheiten des heteromeren Proteins/Produkts ermöglichen; und
(ii) das heteromere Protein/Produkt aus der Kultur oder dem Kulturmedium isoliert wird.

38. Verfahren nach einem der Ansprüche 34 bis 37 ***dadurch gekennzeichnet,* dass** die aussortierten Säugerzellen eine durchschnittliche spezifische Produktivität von größer 5pg des (der) gewünschten Genprodukts (Genprodukte) pro Tag und Zelle zeigen.

39. Verfahren nach Anspruch 36 oder 37 ***dadurch gekennzeichnet,* dass** die aussortierten Wirtzellen eine durchschnittliche spezifische Produktivität von größer 20pg des (der) gewünschten Genprodukts (Genprodukte) pro Tag und Zelle zeigen.

40. Verfahren nach einem der Ansprüche 29 bis 39 ***dadurch gekennzeichnet,* dass** es sich bei der Säugerzelle um eine Nagerzelle handelt.

41. Verfahren nach Anspruch 40 ***dadurch gekennzeichnet,* dass** es sich bei der Nagerzelle um eine CHO- oder BHK-Zelle handelt.

42. Verfahren nach einem der Ansprüche 29 bis 41 ***dadurch gekennzeichnet,* dass** die Säugerzellen in Suspensionskultur kultiviert werden.

43. Verfahren nach einem der Ansprüche 29 bis 42 ***dadurch gekennzeichnet,* dass** die Säugerzellen serumfrei kultiviert werden.
